# EUROPEAN PATENT SPECIFICATION

(11) **EP 2 846 803 B1**
(45) Date of publication and mention of the grant of the patent: **21.06.2017**
(21) Application number: 13722661.9
(22) Date of filing: 08.05.2013
(51) Int. Cl.: C07D 401/14, C07D 213/82, C07D 213/81, C07D 401/06, C07D 237/24, C07D 261/18, C07D 403/06

(54) **ALPHA 7 NICOTINIC ACETYLCHOLINE RECEPTOR ALLOSTERIC MODULATORS, THEIR DERIVATIVES AND USES THEREOF**
ALLOSTERISCHE MODULATOREN DES NIKOTIN-ALPHA-7-ACETYLCHOLINREZEPTORS, DEREN DERIVATE UND VERWENDUNGEN DAVON
MODULATEURS ALLOSTÉRIQUES DU RÉCEPTEUR NICOTINIQUE À L'ACÉTYLCHOLINE ALPHA 7, LEURS DÉRIVÉS ET LEURS UTILISATIONS

(30) Priority: 08.05.2012 US 201261644318 P; 08.05.2012 US 201261644411 P; 11.05.2012 US 201261645935 P; 15.03.2013 US 201361801544 P
(43) Date of publication of application: 18.03.2015
(73) Proprietor: Alpharmagen, LLC, South San Francisco, CA 94080 (US)
(72) Inventor: PUTMAN, David, Ballston Spa, NY 12020 (US); DASSE, Olivier, Foothill Ranch, CA 92610 (US)
(74) Representative: Kelly, Donal Morgan
(86) International application number: PCT/US2013/040117
(87) International publication number: WO 2013/169889

(56) References cited:
- WO-A1-2006/071184
- WO-A2-2006/076644
- US-A1- 2007 037 794
- US-A1- 2011 118 248

## Description

### Related Application

The present application claims the benefit of priority to U.S. Serial No. 61/644,318, and filed on May 8, 2012; U.S. Serial No. 61/644,411, and filed on May 8, 2012; U.S. Serial No. 61/645,935 and filed on May 11, 2012; and U.S. Serial No. 61/801,544 filed on March 15, 2013.

### Background

The disclosure of the present application is in the field of medicinal chemistry. In particular, this application discloses a class of novel compounds that allosterically modulate the α7 nicotinic acetylcholine receptor (α7 nAChR) and may be used to treat disorders amenable to modulation of the α7 nAChR.

α7 nAChRs belong to the ligand-gated ion channel superfamily of Cys-loop receptors. The Cys-loop superfamily includes muscle and neuronal nAChRs, 5-hydroxytryptamine type 3 (5HT₃), γ-aminobutyric acid_{A} (GABA_{A}), GABA_{C} and glycine receptors. α7 nAChRs are ion channels that recognize acetylcholine and choline as endogenous orthosteric ligands and also bind nicotine at the orthosteric site. α7 nAChRs contain 5 orthosteric receptor sites per receptor. Agonist binding to the orthosteric site effects functional states of the receptor depending on the concentration and kinetics of agonist application. Four functional states have been described for α7 nAChRs: one open and three closed states (resting, fast-onset desensitized, slow-onset desensitized). Unlike agonists, allosteric modulators of α7 nAChRs do not bind to the orthosteric site, and cannot affect the functional state of the ion channel by themselves. An allosteric modulator of α7 nAChRs requires the presence of an agonist to activate the channel, and in-turn potentiates the action of the agonist. In the brain, activation of neuronal α7 nAChRs mediates fast synaptic transmission and controls synaptic transmission by the major inhibitory and excitatory neurotransmitters, GABA and glutamate.

α7 nAChRs mediate the predominant nicotinic current in hippocampal neurons. The α7 nAChR was initially identified from a chick brain library as an α-bungarotoxin binding protein that exhibits ∼40% sequence homology to other nAChRs. α7 nAChRs share similar features of other neuronal and muscle nAChRs such as a pentameric Cys-loop receptor structure and M2 segment of each subunit lining of the channel pore, however the α7 nAChRs exhibits a homopentameric structure when reconstituted in *Xenopus* oocytes, a characteristic shared only with the α8 and α9 nAChRs. Heterologously expressed homomeric α7 nAChRs in *Xenopus* oocytes are inactivated by α-bungarotoxin with high affinity, whereas other nAChRs are not. α7 nAChRs have also been pharmacologically identified by distinct types of whole cell currents elicited by nicotinic agonists in hippocampal neurons. When exposed to various nicotinic agonists, whole cell recordings from cultured hippocampal neurons show, in general, type IA currents that have a very brief open time, high conductance, very high Ca⁺⁺ permeability, decay rapidly, and are sensitive to blockade by methyllycaconitine (MLA) and α-bungarotoxin. The properties of these nicotinic currents in hippocampal neurons correspond to the currents mediated by α7 nAChRs expressed in oocytes.

### Summary of the Invention

Briefly, this invention is generally directed to allosteric modulators of the a7 nAChR, as well as to methods for their preparation and use, and to pharmaceutical compositions containing the same. More specifically, the allosteric α7 nAChR modulators of this invention are compounds represented by the general structure: 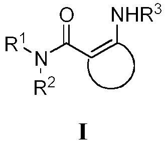 including pharmaceutically acceptable salts and solvatesthereof, wherein R¹, R², R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R²⁹, R³⁰, R³¹, R³², R³³, R³⁴, R³⁵, R³⁶, R³⁷ and X¹, X², X³, X⁴, X⁵, X⁶, X⁷, X⁸, X⁹, X¹⁰, X¹¹, X¹², X¹³, X¹⁴, X¹⁵, X¹⁶, X¹⁷, X¹⁸, X¹⁹, X²⁰,
X²¹, X²², X²³, X²⁴, X²⁵, X²⁶, X²⁷, X²⁸, X²⁹, X³⁰, X³¹, X³², X³³, X³⁴, X³⁵, X³⁶, X³⁷, X³⁸ are as defined below.

Further, the present invention is directed to ²H, ³H, ¹¹C, ¹⁸F, ³⁵S, ³⁶Cl, ¹⁴C and ¹²⁵I labeled compounds of Formulae **I-VII** and their use as stablely isotopically labeled analogs or as radioligands for their binding site on the α7 nAChR complex.

This invention also is directed to compounds for treating disorders responsive to enhancement of acetylcholine action on α7 nAChRs in a mammal by administering an effective amount of a compound of Formulae **I-VII** as described herein. Compounds of the present invention may be used in the treatment and/or prevention of a variety of disorders, including those of the central nervous system (CNS) and the peripheral nervous system (PNS). Disorders of the CNS and the PNS include neurodegenerative diseases, senile dementias, schizophrenia, Alzheimer's disease, learning, cognition and attention deficits, memory loss, Lewy Body dementia, attention-deficit disorder, attention deficit hyperactivity disorder, anxiety, mania, manic depression, Parkinson's disease, Huntington's disease, depression, amyotrophic lateral sclerosis, brain inflammation, cognitive deficit due to traumatic brain injury and Tourette's syndrome. Compounds of the invention are also useful in the treatment (therapeutic or prophylactic), prevention or delay of progression of dyskinesia associated with dopamine agonist therapy in Parkinson's disease. In addition, compounds of the present invention may be used to treat pain, inflammation, septic shock, ulcerative colitis, irritable bowel syndrome and Crohn's disease. In addition, compounds of the invention are useful in tobacco cessation treatment (Brunzell et al. Neuropsychopharm. 2011, 1-10), in the treatment of diabetes (Marrero et al. JPET, 2009, 332, 173) and in treating jetlag. Compounds are also useful in treating immune system disorders, Fragile X, autism spectrum disorder, Angelman's syndrome, Rett syndrome, Prader Willi syndrome and Down's syndrome.

The present invention also is directed to pharmaceutical formulations which include a compound of the present invention. Such formulations contain a therapeutically effective amount of a compound of Formulae **I-VII,** pharmaceutically acceptable salts and solvates thereof and one or more pharmaceutically acceptable carriers or diluents.

Additional embodiments and advantages of the invention will be set forth in part in the description that follows, and in part will be apparent from the description, or may be learned by practice of the invention. The embodiments and advantages of the invention will be realized and attained by means of the elements and combinations particularly pointed out in the appended claims.

It is to be understood that both the foregoing general description and the following detailed description are exemplary and explanatory only and are not restrictive of the invention, as claimed.

### Detailed Description

In one aspect, the present invention is directed to a compound of Formula **I**: 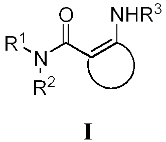 and pharmaceutically acceptable salts and solvates thereof, wherein:
R¹ and R² taken together with the nitrogen atom to which they are attached form a bicyclic heteroaryl or partially unsaturated bicyclic heteroaryl group, wherein said bicyclic heteroaryl group or partially unsaturated bicyclic heteroaryl group is selected from: 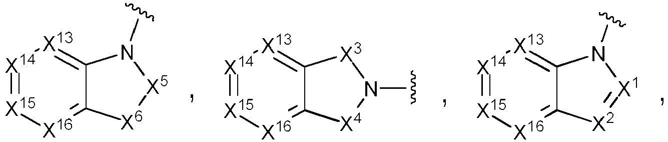 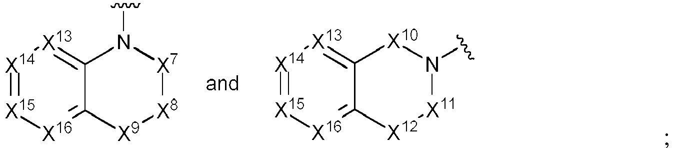
and 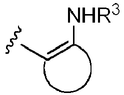 is a heteroaryl group selected from: 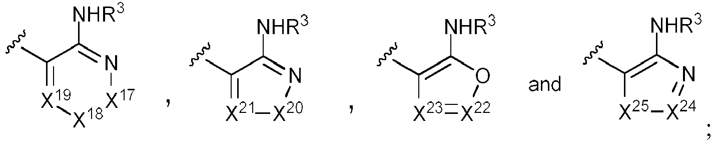
X¹ is N or CR⁴; X² is N or CR⁵ except that X¹ and X² are not both N;
each of X³, X⁴, X⁵, X⁶, X⁷, X⁸, X⁹, X¹⁰, X¹¹ and X¹² is independently O, C=O, S(=O)ₘ, NR⁶ or CR⁷R⁸;
X¹³ is N or CR⁹;
X¹⁴ is N or CR¹⁰;
X¹⁵ is N or CR¹¹;
X¹⁶ is N or CR¹²;
X¹⁷ is N or CR¹³;
X¹⁸ is N or CR¹⁴;
X¹⁹ is N or CR¹⁵;
X²⁰ is NR⁶, S(O)ₘ or O;
X²¹ is N or CR¹⁶
X²² is N or CR¹⁷
X²³ is N or CR¹⁸;
X²⁴ is N or CR¹⁹;
X²⁵ is NR⁶, S(O)ₘ or O;
m is 0, 1 or 2;
R³ is selected from the group consisting of C₂₋₈ alkyl, C₂₋₈ alkenyl, C₂₋₈ alkynyl, C₁₋₈ haloalkyl, arylalkyl, heteroarylalkyl, C₃₋₈ cycloalkyl, cycloalkenyl, carbon-attached heterocycloalkyl, and carbon-attached heterocycloalkenyl, each optionally substituted; and
R⁶ is selected from the group consisting of hydrogen, C₁₋₈ alkyl, C₃₋₈ alkenyl, C₃₋₈ alkynyl, C₁₋₈ haloalkyl, aryl, heteroaryl and C₃₋₈ cycloalkyl; and
R⁴, R⁵, R⁷, R⁸, R⁹, R¹⁰, R¹¹, R¹², R¹⁴, R¹⁵, R¹⁶, R¹⁷, R¹⁸, and R¹⁹ are each independently selected from the group consisting of hydrogen, halogen, nitro, cyano, hydroxyl, amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkamino, C₁₋₈ haloalkamino, dialkylamino, alkenylamino, alkynylamino, arylamino, heteroarylamino, C₁₋₈ cycloalkamino, cycloalkenylamino, heterocycloalkylamino, heterocycloalkenylamino, C₁₋₈ alkthio, C₁₋₈ haloalkthio, alkenylthio, alkynylthio, arylthio, heteroarylthio, C₃₋₈ cycloalkthio, cycloalkenylthio, heterocycloalkylthio, heterocycloalkenylthio, -C(=O)R²⁰, -N(R²¹)C(=O)R²², -OC(=O)R²²,-N(R²¹)S(=O)₂R²², -S(=O)₂R²⁰, and -S(=O)R²⁰, each optionally substituted; and
R¹³ is selected from the group consisting of hydrogen, halogen, nitro, cyano, hydroxyl, C₁₋₈ alkyl, C₂₋₈ alkenyl, C₂₋₈ alkynyl, C₁₋₈ haloalkyl, aryl, heteroaryl, C₁₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ haloalkamino, alkenylamino, alkynylamino, arylamino, heteroarylamino, C₃₋₈ cycloalkamino, cycloalkenylamino, heterocycloalkylamino, heterocycloalkenylamino, C₁₋₈ alkthio, C₁₋₈ haloalkthio, alkenylthio, alkynylthio, arylthio, heteroarylthio, C₃₋₈ cycloalkthio, cycloalkenylthio, heterocycloalkylthio, heterocycloalkenylthio, -C(=O)R²⁰,-N(R²¹)C(=O)R²², -OC(=O)R²², -N(R²¹)S(=O)₂R²², -S(=O)₂R²⁰, and -S(=O)R²⁰, each optionally substituted; and
R⁴ and R⁵, or R⁶ and R¹⁶, or R⁶ and R¹⁹, or R⁹ and R¹⁰, or R¹⁰ and R¹¹, or R¹¹ and R¹², or R¹³ and R¹⁴, or R¹⁴ and R¹⁵, or R¹⁷ and R¹⁸ taken together with the atoms to which they are attached form an unsubstituted or substituted fused 5 or 6-membered unsaturated or partially unsaturated ring optionally interrupted by one -O-, -NR⁶-, -S-, -SO- or -SO₂-; and
each R²⁰ is independently selected from the group consisting of hydroxyl, amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkylamino, C₁₋₈ haloalkylamino, dialkylamino, alkenylamino, alkynylamino, arylamino, heteroarylamino, C₃₋₈ cycloalkylamino, cycloalkenylamino, heterocycloalkylamino, and heterocycloalkenylamino, each optionally substituted; and
each R²¹ is independently selected from the group consisting of hydrogen, hydroxyl, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, and heterocycloalkenyloxy, each optionally substituted; and
each R²² is independently selected from the group consisting of amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkylamino, C₁₋₈ haloalkylamino, dialkylamino, alkenylamino, alkynylamino, arylamino, heteroarylamino, C₃₋₈ cycloalkylamino, cycloalkenylamino, heterocycloalkylamino, and heterocycloalkenylamino, each optionally substituted.

### Definition

Unless specifically noted otherwise herein, the definitions of the terms used are standard definitions used in the art of organic synthesis and pharmaceutical sciences.

In one aspect, groups for R¹R²N include:

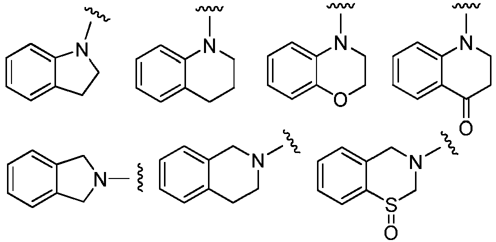

The term "halogen" as used herein refers to a halogen radical selected from fluoro, chloro, bromo and iodo.

The term "cyano" refers to -C≡N.

The term "nitro" refers to -NO₂.

The term "hydroxyl" refers to -OH.

The term "alkyl" refers to a saturated aliphatic hydrocarbon radical. "Alkyl" refers to both branched and unbranched alkyl groups. One or more of the carbons may be oxidized to C(=O). Examples of "alkyl" include alkyl groups that are straight chain alkyl groups containing from one to ten carbon atoms and branched alkyl groups containing from three to ten carbon atoms. "Alkyl" includes but is not limited to straight chain alkyl groups containing from one to six carbon atoms and branched alkyl groups containing from three to six carbon atoms. This term is exemplified by groups such as methyl, ethyl, n-propyl, 1-methylethyl (isopropyl), 1,1-dimethylethyl (*tert-*butyl)*,* and the like. It may be abbreviated "Alk". It should be understood that any combination term using an "alk" or "alkyl" prefix refers to analogs according to the above definition of "alkyl" including the number of carbon atoms. For example, terms such as "alkoxy", "alkylthio", "alkylamino" refer to alkyl groups linked to a second group via an oxygen, sulfur, or nitrogen atom, respectively.

The term "haloalkyl" refers to an alkyl group in which one or more hydrogen atoms are replaced with halogen atoms. One or more of the carbons may be oxidized to C(=O). This term includes but is not limited to groups such as trifluoromethyl. In one embodiment the haloalkyl groups are alkyl groups substituted with one or more fluoro or chloro. The term "haloalkoxy" refers to haloalkyl groups linked to a second group via an oxygen atom.

The term "alkenyl" refers to a mono or polyunsaturated aliphatic hydrocarbon radical. The mono or polyunsaturated aliphatic hydrocarbon radical contains at least one carbon-carbon double bond. "Alkenyl" refers to both branched and unbranched alkenyl groups, each optionally partially or fully halogenated. One or more of the carbons may be oxidized to C(=O). Examples of "alkenyl" include alkenyl groups that are straight chain alkenyl groups containing from two to ten carbon atoms and branched alkenyl groups containing from three to ten carbon atoms. Other examples include alkenyl groups which are straight chain alkenyl groups containing from two to six carbon atoms and branched alkenyl groups containing from three to six carbon atoms. Alkenyl groups include but are not limited to ethenyl, propenyl, n-butenyl, isobutenyl, 3-methylbut-2-enyl, n-pentenyl, heptenyl, octenyl, decenyl, and the like. It should be understood that any combination term using an "alkenyl" prefix refers to analogs according to the above definition of "alkenyl" including the number of carbon atoms. For example, terms such as "alkenyloxy", "alkenylthio", "alkenylamino" refer to alkenyl groups linked to a second group via an oxygen, sulfur, or nitrogen atom, respectively.

The term "alkynyl" refers to a mono or polyunsaturated aliphatic hydrocarbon radical. The mono or polyunsaturated aliphatic hydrocarbon radical contains at least one carbon-carbon triple bond. "Alkynyl" refers to both branched and unbranched alkynyl groups, each optionally partially or fully halogenated. One or more of the carbons may be oxidized to C(=O). Examples of "alkynyl" include alkynyl groups that are straight chain alkynyl groups containing from two to ten carbon atoms and branched alkynyl groups containing from four to ten carbon atoms. Other examples include alkynyl groups that are straight chain alkynyl groups containing from two to six carbon atoms and branched alkynyl groups containing from four to six carbon atoms. This term is exemplified by groups such as ethynyl, propynyl, octynyl, and the like. It should be understood that any combination term using an "alkynyl" prefix refers to analogs according to the above definition of "alkynyl" including the number of carbon atoms. For example, terms such as "alkynyloxy", "alkynylthio", "alkynylamino" refer to alkynyl groups linked to a second group via an oxygen, sulfur, or nitrogen atom, respectively.

The term "cycloalkyl" refers to the mono- or polycyclic analogs of an alkyl group, as defined above. One or more of the carbons may be oxidized to C(=O). Unless otherwise specified, the cycloalkyl ring may be attached at any carbon atom that results in a stable structure and, if substituted, may be substituted at any suitable carbon atom which results in a stable structure. Examples of cycloalkyl groups are saturated cycloalkyl groups containing from three to ten carbon atoms. Other examples include cycloalkyl groups containing three to eight carbon atoms or three to six carbon atoms. Exemplary cycloalkyl groups include but are not limited to cyclopropyl, cyclobutyl, cyclopentyl, cyclohexyl, cyclooctyl, cyclononyl, cyclodecyl, norbornyl, adamantyl, and the like. It should be understood that any combination term using "cycloalkyl" refers to analogs according to the above definition of "cycloalkyl" including the number of carbon atoms. Terms such as "cycloalkyloxy", "cycloalkylthio", "cycloalkylamino" refer to a cycloalkyl groups linked to a second group via an oxygen, sulfur, or nitrogen atom, respectively.

The term "cycloalkenyl" refers to the mono- or polycyclic analogs of an alkenyl group, as defined above. One or more of the carbons may be oxidized to C(=O). Unless otherwise specified, the cycloalkenyl ring may be attached at any carbon atom that results in a stable structure and, if substituted, may be substituted at any suitable carbon atom which results in a stable structure. Examples of cycloalkenyl groups are cycloalkenyl groups containing from four to ten carbon atoms. Other examples include cycloalkenyl groups containing four to eight carbon atoms or four to six carbon atoms. Exemplary cycloalkenyl groups include but are not limited to cyclobutenyl, cyclopentenyl, cyclohexenyl, norbornene, and the like. It should be understood that any combination term using "cycloalkenyl" refers to analogs according to the above definition of "cycloalkenyl" including the number of carbon atoms. Terms such as "cycloalkenyloxy", "cycloalkenylthio", "cycloalkenylamino" refer to a cycloalkenyl groups linked to a second group via an oxygen, sulfur, or nitrogen atom, respectively.

The term "heterocycloalkyl" refers to the mono- or polycyclic structures of "cycloalkyl" where one or more of the carbon atoms are replaced by one or more atoms independently selected from nitrogen, oxygen, or sulfur atoms. Any nitrogen atom maybe optionally oxidized or quaternized, and any sulfur atom maybe optionally oxidized. Generally, the heteroatoms may be selected from the group consisting of N, S, S=O, S(=O)₂, and O. One or more of the carbons may be oxidized to C(=O). Unless otherwise specified, the heterocycloalkyl ring may be attached at any carbon atom or heteroatom that results in a stable structure and, if substituted, may be substituted at any suitable carbon atom or heteroatom which results in a stable structure. Examples of heterocycloalkyl groups are saturated heterocycloalkyl groups containing from two to nine carbon atoms and one to four heteroatoms. Generally, 5-7 membered heterocycloalkyl groups contain 3-6 carbon atoms and 1-2 heteroatoms independently selected from the group consisting of N, S, S=O, S(=O)₂, and O. Examples of heterocycloalkyl groups include but are not limited to morpholino, pyrazino, tetrahydrofurano, and the like. "Carbon-attached heterocycloalkyl" refers to a heterocycloalkyl group which is bound via a constituent carbon atom. A heterocycloalkyl that is fused with a phenyl can include, but is not limited to the following: 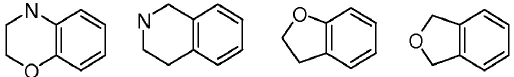 A heterocycloalkyl that is fused with a 5-6 membered heteroaryl can include, but is not limited to the following: 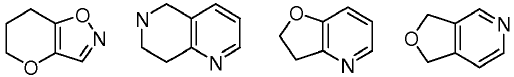 Terms such as "heterocycloalkyloxy", "heterocycloalkylthio", "heterocycloalkylamino" refer to heterocycloalkyl groups linked to a second group via an oxygen, sulfur, or nitrogen atom, respectively.

The term "heterocycloalkenyl" refers to the mono- or polycyclic structures of "cycloalkenyl" where one or more of the carbon atoms are replaced by one or more atoms independently chosen from nitrogen, oxygen, or sulfur atoms. Any nitrogen atom maybe optionally oxidized or quaternized, and any sulfur atom maybe optionally oxidized. One or more of the carbons may be oxidized to C(=O). Unless otherwise specified, the heterocycloalkenyl ring may be attached at any carbon atom or heteroatom that results in a stable structure and, if substituted, may be substituted at any suitable carbon atom or heteroatom which results in a stable structure. Examples of heterocycloalkenyl groups are saturated heterocycloalkenyl groups containing from two to nine carbon atoms and one to four heteroatoms. Generally, 5-7 membered heterocycloalkenyl groups contain 3-6 carbon atoms and 1-2 heteroatoms independently selected from the group consisting of N, S, S=O, S(=O)₂, and O. Examples of heterocycloalkenyl groups include but are not limited to dihydropyran, dihydrofuran, and the like. "Carbon-attached heterocycloalkenyl" refers to a heterocycloalkenyl group which is bound via a constituent carbon atom. Terms such as "heterocycloalkenyloxy", "heterocycloalkenylthio", "heterocycloalkenylamino" refer to heterocycloalkenyl groups linked to a second group via an oxygen, sulfur, or nitrogen atom, respectively.

The term "acyl" refers to a monovalent radical of the formula -C(=O)-alkyl and -C(=O)-cycloalkyl, i.e., an alkyl or cycloalkyl group linked to a second group via carbonyl group C(=O), wherein said alkyl maybe further substituted with cycloalkyl, aryl, or heteroaryl. Examples of acyl groups include -C(=O)Me (acetyl), -C(=O)CH₂-cyclopropyl (cyclopropylacetyl), - C(=O)CH₂Ph (phenylacetyl), and the like.

The term "aryl" refers to 6-10 membered mono- or polycyclic aromatic carbocycles, for example, phenyl and naphthyl. Unless otherwise specified, the aryl ring may be attached at any carbon atom that results in a stable structure and, if substituted, may be substituted at any suitable carbon atom which results in a stable structure. The term "aryl" refers to non-substituted aryls and aryls optionally substituted with one or more substituents. Aryl may be abbreviated "Ar". It should be understood that any combination term using an "ar" or "aryl" prefix refers to analogs according to the above definition of "aryl" including the number of carbon atoms. For example, terms such as "aryloxy", "arylthio", and "arylamino" refer to aryl groups linked to a second group via an oxygen, sulfur, or nitrogen atom, respectively.

The term "arylalkyl" refers to alkyl groups substituted with an aryl group and refers to aryl groups linked to another group via an sp³ carbon atom. Examples include benzyl, α-methylbenzyl and phenethyl groups.

The term "heteroaryl" refers to a stable 5-8 membered monocyclic or 8-11 membered bicyclic aromatic heterocycle radical. In one embodiment the monocyclic groups are 5 or 6 membered. Each heteroaryl contains 1-10 carbon atoms and from 1 to 5 heteroatoms independently chosen from nitrogen, oxygen and sulfur, wherein any sulfur heteroatom may optionally be oxidized and any nitrogen heteroatom may optionally be oxidized or quaternized. Unless otherwise specified, the heteroaryl ring may be attached at any suitable heteroatom or carbon atom that results in a stable structure and, if substituted, may be substituted at any suitable heteroatom or carbon atom which results in a stable structure. The term "heteroaryl" includes heteroaryl groups that are non-substituted or those optionally substituted. Generally, heteroaryl groups containing 2-9 carbon atoms and 1-4 heteroatoms independently selected from the group N, S, S=O, S(=O)₂, and O. Examples of "heteroaryl" include but are not limited to radicals such as furanyl, thienyl, pyrrolyl, oxazolyl, thiazolyl, imidazolyl, pyrazolyl, isoxazolyl, isothiazolyl, oxadiazolyl, triazolyl, tetrazolyl, thiadiazolyl, pyridinyl, pyridazinyl, pyrimidinyl, pyrazinyl, indolizinyl, indolyl, isoindolyl, benzofuranyl, benzothienyl, indazolyl, benzimidazolyl, benzothiazolyl, benzoxazolyl, benzisoxazolyl, benzisothiazolyl, purinyl, quinolizinyl, quinolinyl, isoquinolinyl, cinnolinyl, phthalazinyl, quinazolinyl, quinoxalinyl, naphthyridinyl, pteridinyl, carbazolyl, acridinyl, phenazinyl, phenothiazinyl and phenoxazinyl. It should be understood that any combination term using "heteroaryl" refers to analogs according to the above definition of "heteroaryl" including the number of carbon and heteroatoms. Terms such as "heteroaryloxy", "heteroarylthio", "heteroarylamino" refer to heteroaryl groups linked to a second group via an oxygen, sulfur, or nitrogen atom, respectively.

The term "heteroarylalkyl" refers to alkyl groups substituted with a heteroaryl group and refers to a heteroaryl group that is linked to a second group via an sp³ carbon atom. Examples include 2- 3- and 4-pyridylmethyl and 2-(2-pyridyl)ethyl groups.

The term "amino" group is -NH₂. Alkylamino and dialkylamino groups, for example, include the groups -NHR⁶ and -NR⁶R²⁰ wherein each R⁶ and R²⁰ are independently substituted or unsubstituted C₁₋₁₀ alkyl groups. Examples of such groups include -NHMe, -NHEt, -NHcyclohexyl, -NHCH₂phenyl, -N(Me)₂ and the like. Useful dialkylamino groups include any of the above-mentioned C₁₋₁₀ alkyl groups, each substituted or unsubstituted. Also, a substituted amino group may include for example-NHMe, -NHEt, -NHcyclohexyl, -NHCH₂phenyl, -N(Me)₂ and the like, and -NHCOMe, -NHCOEt, -NHCONHMe and the like. Useful alkylamino and dialkylamino are -NHR⁶, and -NR⁶R²⁰, wherein R⁶ and R²⁰ are C₁₋₁₀ alkyl groups, each unsubstituted or substituted by any of the previously mentioned dialkyl amino groups. In one aspect, R⁶ and R²⁰ are C₁₋₆ alkyl groups. A dialkylamino group, such as -NR⁶R²⁰ includes the group wherein R⁶ and R²⁰ are combined with the nitrogen to which they attach to form a ring, such as a 3-membered, 4-membered, 5-membered or 6-membered ring and their fused, bicyclic analogs, each of which may be further substituted as defined herein. Non-exclusive examples of such rings may include aziridines, pyrrolidines, piperidines, piperazines, morpholines and the like. In certain variations of the nitrogen containing ring, the ring may comprise one or more double bonds and may be fully or partially unsaturated.

All of the groups defined above may be optionally substituted as defined below.

The terms "optional" or "optionally" mean that the subsequently described event or circumstances may or may not occur, and that the description includes instances where the event or circumstance occurs and instances in which it does not. For example, "optionally substituted aryl" means that the aryl radical may or may not be substituted and that the description includes both substituted aryl radicals and aryl radicals having no substitution. In one aspect, optional substitution is 0-5 substitutions of the groups described below. Optional substituents include one or more of the following groups: halogen, C₁-C₁₀ alkyl, C₁-C₃ haloalkyl, C₁-C₃ haloalkoxy, C₃-C₆ cycloalkyl, C₂-C₁₀ alkenyl, C₄-C₆ cycloalkenyl, C₂-C₆ alkynyl, nitro, cyano, hydroxyl, C₁-C₆ alkoxy, C₃-C₆ cycloalkoxy, amido, amino, C₁-C₆ alkylamino (for example, -NHMe- or -N(Me)₂), C₁-C₆ carbamoyl, C₁-C₆ carboxy, C₁-C₆ carbonyl, C₁-C₆ acyl, thiol, C₁-C₆ alkylthio, and C₁-C₆ carboxylic acid. Such substituents can further be substituted with optionally selected groups to form a stable structure.

As used herein "solvate" refers to a complex of variable stoichiometry formed by a solute (*e.g.* a compound of Formula **I** or a salt or ester thereof) and a solvent. Such solvents for the purpose of the invention may not interfere with the biological activity of the solute. Examples of suitable solvents include water, methanol, ethanol and acetic acid. Generally the solvent used is a pharmaceutically acceptable solvent. Examples of suitable pharmaceutically acceptable solvents include water, ethanol and acetic acid. Generally the solvent used is water.

"Isomers" mean any compound with an identical molecular formula but having a difference in the nature or sequence of bonding or arrangement of the atoms in space. Examples of such isomers include, for example, *E*- and *Z*-isomers of double bonds, enantiomers, and diastereomers. Compounds of the present invention depicting a bond with a straight line or "squiggly line" representation that is attached to a double bond, unless specifically noted otherwise, is intended to encompass a single isomer and/or both isomers of the double bond as shown below mean any compound with an identical molecular formula but having a difference in the nature or sequence of bonding or arrangement of the atoms in space.

As used herein "allosteric modulator" of α7 nAChRs refers to a compound that binds allosterically to the α7 nAChR, thereby increasing (positive allosteric modulator) or decreasing (negative allosteric modulator) the agonist-evoked response in cells.

As used herein "disorders amenable to modulation of α7 nAChRs" refers to neurodegenerative diseases, senile dementias, schizophrenia, Alzheimer's disease, learning, cognition and attention deficits, memory loss, Lewy Body dementia, attention-deficit disorder, attention deficit hyperactivity disorder, anxiety, mania, manic depression, Parkinson's disease, Huntington's disease, depression, amyotrophic lateral sclerosis, brain inflammation, cognitive deficit due to traumatic brain injury ("TBI") and Tourette's syndrome. In addition, such disorders include immune system disorders such as, but not limited to, type I diabetes, multiple schlerosis, and rheumatoid arthritis. "Disorders amenable to modulation of α7 nAChRs" also include pain, inflammation, septic shock, ulcerative colitis, Crohn's disease, irritable bowel syndrome, and jet lag. Also included are autism spectrum disorders, inflammation, and mild cognitive impairment.

As used herein "a cognitive disorder related to learning or memory" refers to mild cognitive impairment, age related cognitive decline, senile dementia and Alzheimer's disease.

### Formulations

Compounds of the invention are administered orally in a total daily dose of about 0.01 mg/kg/dose to about 100 mg/kg/dose, alternately from about 0.1 mg/kg/dose to about 10 mg/kg/dose. The use of time-release preparations to control the rate of release of the active ingredient may be employed. The dose may be administered in as many divided doses as is convenient. When other methods are used (e.g. intravenous administration), compounds are administered to the affected tissue at a rate from 0.05 to 10 mg/kg/hour, alternately from 0.1 to 1 mg/kg/hour. Such rates are easily maintained when these compounds are intravenously administered as discussed below.

For the purposes of this invention, the compounds may be administered by a variety of means including orally, parenterally, by inhalation spray, topically, or rectally in formulations containing pharmaceutically acceptable carriers, adjuvants and vehicles. The term parenteral as used here includes subcutaneous, intravenous, intramuscular, and intraarterial injections with a variety of infusion techniques. Intraarterial and intravenous injection as used herein includes administration through catheters. Oral administration is generally employed.

Pharmaceutical compositions containing the active ingredient may be in any form suitable for the intended method of administration. When used for oral use for example, tablets, troches, lozenges, aqueous or oil suspensions, dispersible powders or granules, emulsions, hard or soft capsules, syrups or elixirs may be prepared. Compositions intended for oral use may be prepared according to any method known to the art for the manufacture of pharmaceutical compositions and such compositions may contain one or more agents including sweetening agents, flavoring agents, coloring agents and preserving agents, in order to provide a palatable preparation. Tablets containing the active ingredient in admixture with non-toxic pharmaceutically acceptable excipient which are suitable for manufacture of tablets are acceptable. These excipients may be, for example, inert diluents, such as calcium or sodium carbonate, lactose, calcium or sodium phosphate; granulating and disintegrating agents, such as maize starch, or alginic acid; binding agents, such as starch, gelatin or acacia; and lubricating agents, such as magnesium stearate, stearic acid or talc. Tablets may be uncoated or may be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax may be employed.

Formulations for oral use may be also presented as hard gelatin capsules where the active ingredient is mixed with an inert solid diluent, for example calcium phosphate or kaolin, or as soft gelatin capsules wherein the active ingredient is mixed with water or an oil medium, such as peanut oil, liquid paraffin or olive oil.

Aqueous suspensions of the invention contain the active materials in admixture with excipients suitable for the manufacture of aqueous suspensions. Such excipients include a suspending agent, such as sodium carboxymethylcellulose, methylcellulose, hydroxypropyl methylcellulose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethyleneoxycetanol), a condensation product of ethylene oxide with a partial ester derived from a fatty acid and a hexitol anhydride (e.g., polyoxyethylene sorbitan monooleate). The aqueous suspension may also contain one or more preservatives such as ethyl or n-propyl p-hydroxy-benzoate, one or more coloring agents, one or more flavoring agents and one or more sweetening agents, such as sucrose or saccharin.

Oil suspensions may be formulated by suspending the active ingredient in a vegetable oil, such as arachis oil, olive oil, sesame oil or coconut oil, or in a mineral oil such as liquid paraffin. The oral suspensions may contain a thickening agent, such as beeswax, hard paraffin or cetyl alcohol. Sweetening agents, such as those set forth above, and flavoring agents may be added to provide a palatable oral preparation. These compositions may be preserved by the addition of an antioxidant such as ascorbic acid.

Dispersible powders and granules of the invention suitable for preparation of an aqueous suspension by the addition of water provide the active ingredient in admixture with a dispersing or wetting agent, a suspending agent, and one or more preservatives. Suitable dispersing or wetting agents and suspending agents are exemplified by those disclosed above. Additional excipients, for example sweetening, flavoring and coloring agents, may also be present.

The pharmaceutical compositions of the invention may also be in the form of oil-in-water emulsions. The oily phase may be a vegetable oil, such as olive oil or arachis oil, a mineral oil, such as liquid paraffin, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan monooleate, and condensation products of these partial esters with ethylene oxide, such as polyoxyethylene sorbitan monooleate. The emulsion may also contain sweetening and flavoring agents.

Syrups and elixirs may be formulated with sweetening agents, such as glycerol, sorbitol or sucrose. Such formulations may also contain a demulcent, a preservative, a flavoring or a coloring agent.

The pharmaceutical compositions of the invention may be in the form of a sterile injectable preparation, such as a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, such as a solution in 1,3-butanediol or prepared as a lyophilized powder. Among the acceptable vehicles and solvents that may be employed are water, Ringer's solution and isotonic sodium chloride solution. In addition, sterile fixed oils may conventionally be employed as a solvent or suspending medium. For this purpose any bland fixed oil may be employed including synthetic mono- or diglycerides. In addition, fatty acids such as oleic acid may likewise be used in the preparation of injectables.

The amount of active ingredient that may be combined with the carrier material to produce a single dosage form will vary depending upon the host treated and the particular mode of administration. For example, a time-release formulation intended for oral administration to humans may contain approximately 1 to 1000 mg of active material compounded with an appropriate and convenient amount of carrier material which may vary from about 5 to about 95% of the total compositions. The pharmaceutical composition can be prepared to provide easily measurable amounts for administration. For example, an aqueous solution intended for intravenous infusion should contain from about 3 to 330 µg of the active ingredient per milliliter of solution in order that infusion of a suitable volume at a rate of about 30 mL/hr can occur.

As noted above, formulations of the present invention suitable for oral administration may be presented as discrete units such as capsules, cachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be administered as a bolus, electuary or paste.

A tablet may be made by compression or molding, optionally with one or more accessory ingredients. Compressed tablets may be prepared by compressing in a suitable machine the active ingredient in a free flowing form such as a powder or granules, optionally mixed with a binder (e.g., povidone, gelatin, hydroxypropylmethyl cellulose), lubricant, inert diluent, preservative, disintegrant (e.g., sodium starch glycolate, cross-linked povidone, cross-linked sodium carboxymethyl cellulose) surface active or dispersing agent. Molded tablets may be made by molding in a suitable machine a mixture of the powdered compound moistened with an inert liquid diluent. The tablets may optionally be coated or scored and may be formulated so as to provide slow or controlled release of the active ingredient therein using, for example, hydroxypropyl methylcellulose in varying proportions to provide the desired release profile. Tablets may optionally be provided with an enteric coating, to provide release in parts of the gut other than the stomach. This is particularly advantageous with the compounds of Formula **I** when such compounds are susceptible to acid hydrolysis.

Formulations suitable for topical administration in the mouth include lozenges comprising the active ingredient in a flavored base, usually sucrose and acacia or tragacanth; pastilles comprising the active ingredient in an inert base such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous isotonic sterile injection solutions which may contain antioxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose sealed containers, for example, ampoules and vials, and may be stored in a freeze-dried (lyophilized) condition requiring only the addition of the sterile liquid carrier, for example water for injections, immediately prior to use. Injection solutions and suspensions may be prepared from sterile powders, granules and tablets of the kind previously described.

Suitable unit dosage formulations are those containing a daily dose or unit, daily sub-dose, or an appropriate fraction thereof, of a compound of Formulae **I-VII**.

It will be understood, however, that the specific dose level for any particular patient will depend on a variety of factors including the activity of the specific compound employed; the age, body weight, general health, sex and diet of the individual being treated; the time and route of administration; the rate of excretion; other drugs which have previously been administered; and the severity of the particular disease undergoing therapy, as is well understood by those skilled in the art.

In one embodiment of this invention X¹³ is CR⁹, X¹⁴ is CR¹⁰, X¹⁵ is CR¹¹, X¹⁶ is CR¹², X¹⁷ is C-R¹³, X¹⁸ is C-R¹⁴, X¹⁹ is C-R¹⁵ with the remaining groups as defined for Formula I such that representative allosteric α7 nAChR modulators of this invention include compounds having the structure of Formula **II:** 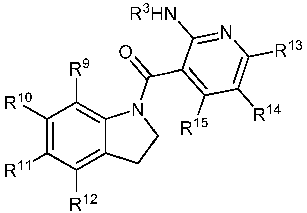 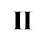 and pharmaceutically acceptable salts thereof.

In one embodiment of this invention X¹³ is CR⁹, X¹⁴ is CR¹⁰, X¹⁵ is CR¹¹, X¹⁶ is CR¹² and R³ is a group R²⁶CH₂- with the remaining groups as defined for Formula I such that representative allosteric α7 nAChR modulators of this invention include compounds having the structure of Formula **III:** 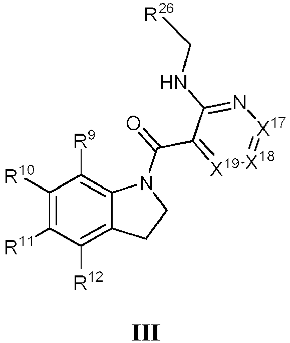
X¹⁷ is N or CR¹³,
X¹⁸ is Nor CR¹⁴;
X¹⁹ is N or CR¹⁵;
wherein R²⁶ is an aryl or heteroaryl group selected from: 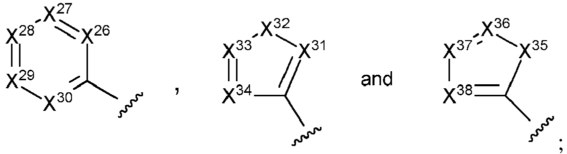
X²⁶ is N or C-R²⁷;
X²⁷ is N or C-R²⁸;
X²⁸ is N or C-R²⁹;
X²⁹ is N or C-R³⁰;
X³⁰ is N or C-R³¹;
X³¹ is N or C-R³²;
X³² is NR⁶, O or S(O)ₘ;
X³³ is N or C-R³³;
X³⁴ is N or C-R³⁴;
X³⁵ is NR⁶ or O;
X³⁶ is N or C-R³⁵;
X³⁷ is N or C-R³⁶;
X³⁸ is N or C-R³⁷;
   R²⁷, R²⁸, R³⁰, R³¹, R³², R³³, R³⁴, R³⁵, R³⁶ and R³⁷ are independently selected 2 from the group consisting of hydrogen, halogen, nitro, cyano, hydroxyl, amino, C₁₋₈ alkyl, C₁₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkamino, C₁₋₈ haloalkamino, dialkylamino, alkenylamino, alkynylamino, arylamino, heteroarylamino, C₃₋₈ cycloalkamino, cycloalkenylamino, heterocycloalkylamino, heterocycloalkenylamino, C₁₋₈ alkthio, C₁₋₈ haloalkthio, alkenylthio, alkynylthio, arylthio, heteroarylthio, C₃₋₈ cycloalkthio, cycloalkenylthio, heterocycloalkylthio, heterocycloalkenylthio,-C(=O)R²O, -N(R²¹)C(=O)R²² -OC(=O)R²² -N(R²¹)S(=O)₂R²², -S(=O)₂R²⁰, and-S(=O)R²⁰, each optionally substituted; and
   R²⁹ is selected from the group consisting of hydrogen, halogen, nitro, cyano, hydroxyl, amino, C₁₋₈ alkyl, C₂₋₈ alkenyl, C₂₋₈ alkynyl, C₁₋₈ haloalkyl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkamino, C₁₋₈ haloalkamino, dialkylamino, alkenylamino, alkynylamino, arylamino, heteroarylamino, C₃₋₈ cycloalkamino, cycloalkenylamino, heterocycloalkylamino, heterocycloalkenylamino, C₁₋₈ alkthio, C₁₋₈ haloalkthio, alkenylthio, alkynylthio, arylthio, heteroarylthio, C₃₋₈ cycloalkthio, cycloalkenylthio, heterocycloalkylthio, heterocycloalkenylthio, -C(=O)R²⁰, -N(R²¹)C(=O)R²²,-OC(=O)R²², -N(R²¹)S(=O)₂R²², -S(=O)₂R²⁰, and -S(=O)R²⁰, each optionally substituted; and
   R²⁷ and R²⁸, or R²⁸ and R²⁹, or R²⁹ and R³⁰, or R³⁰ and R³¹, or R³² and R⁶, or R⁶ and R³³, or R³³ and R³⁴, or R⁶ and R³⁵, or R³⁵ and R³⁶, or R³⁶ and R³⁷ taken together with the atoms to which they are attached form an unsubstituted or substituted fused 5 or 6-membered unsaturated or partially unsaturated ring optionally interrupted by one -O-, -NR⁶-, -S-, -SO- or -SO₂-; and pharmaceutically acceptable salts thereof.

In one embodiment of the invention, compounds of Formula **III** wherein
R⁹, R¹⁰, R¹¹ and R¹² are each independently selected from the group consisting of hydrogen, halogen, C₁₋₈ alkyl, C₁₋₈ haloalkyl, C₃₋₈ cycloalkyl, C₁₋₈ alkoxy, C₁₋₈ haloalkoxy, and C₃₋₈ cycloalkoxy, each optionally substituted; and
R¹³, R¹⁴ and R¹⁵ are each independently selected from the group consisting of hydrogen, halogen, C₁₋₈ alkyl, C₁₋₈ haloalkyl, C₃₋₈ cycloalkyl, and C₁₋₈ haloalkoxy, each optionally substituted; and
R²⁶ is an aryl group selected from: 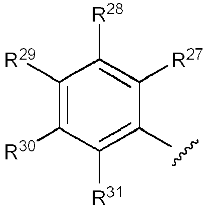
X¹⁷ is N or CR¹³;
X¹⁸ is Nor CR¹⁴;
X¹⁹ is N or CR¹⁵;
R²⁷, R²⁸, R²⁹, R³⁰ and R³¹ are independently selected from the group consisting of hydrogen, halogen, nitro, cyano, C₁₋₈ alkyl, C₁₋₈ haloalkyl, C₃₋₈ cycloalkyl, C₁₋₈ alkoxy, C₁₋₈ haloalkoxy, alkenyloxy, alkynyloxy, and C₃₋₈ cycloalkoxy, and pharmaceutically acceptable salts thereof.

In another embodiment, such compounds are selected from those wherein R⁹ and R¹² are hydrogen; and pharmaceutically acceptable salts thereof.

In another embodiment, such compounds are selected from those wherein
X¹⁷ is CR¹³;
X¹⁸ is CR¹⁴;
X¹⁹ is CR¹⁵;
and pharmaceutically acceptable salts thereof.

In another embodiment, such compounds are selected from those wherein:
X¹⁷ is CR¹³
X¹⁸ is CR¹⁴;

X¹⁹ is N;
and pharmaceutically acceptable salts thereof.

In another embodiment, such compounds are selected from those wherein:
X¹⁷ is N;
X¹⁸ is CR¹⁴;
X¹⁹ is CR¹⁵;
and pharmaceutically acceptable salts thereof.

In another embodiment, such compounds are selected from those wherein:
X¹⁷ is CR¹³
X¹⁸ is N;
X¹⁹ is CR¹⁵;
and pharmaceutically acceptable salts thereof.

In one variation of this invention X¹³ is CR⁹, X¹⁴ is CR¹⁰, X¹⁵ is CR¹¹, X¹⁶ is CR¹², X¹⁷ is N, X¹⁸ is C-R¹⁴, X¹⁹ is C-R¹⁵ with the remaining substituents as defined in Formula I such that representative allosteric α7 nAChR modulators of this invention include compounds having the structure of Formula IV: 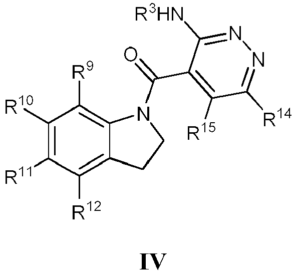 and pharmaceutically acceptable salts thereof.

In another embodiment of this invention X¹³ is CR⁹, X¹⁴ is CR¹⁰, X¹⁵ is CR¹¹, X¹⁶ is CR¹², X¹⁷ is CR¹³, X¹⁸ is C-R¹⁴, X¹⁹ is N with the remaining substituents as defined for Formula I such that representative allosteric α7 nAChR modulators of this invention include compounds having the structure of Formula **V:** 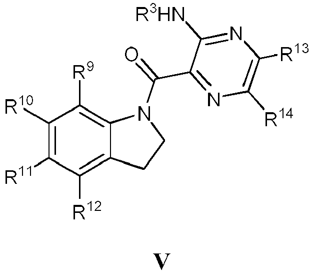 and pharmaceutically acceptable salts thereof.

In yet another embodiment of this invention, X¹³ is CR⁹, X¹⁴ is CR¹⁰, X¹⁵ is CR¹¹, X¹⁶ is CR¹² X¹⁷ is CR¹³, X¹⁸ is N, X¹⁹ is CR¹⁵ with the remaining substituents as defined for Formula I such that representative allosteric α7 nAChR modulators of this invention include compounds having the structure of Formula **VI:** 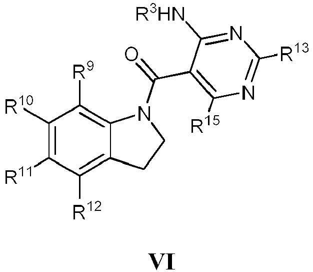 and pharmaceutically acceptable salts thereof.

Another embodiment of this invention involves the use of a compound of Formula **VII** as an allosteric modulator of α7 nAChRs: 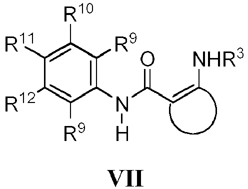 wherein 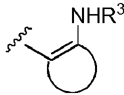 is taken from the following: 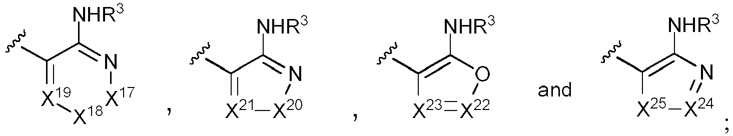 and R³, R⁹, R¹⁰, R¹¹, R¹² and X¹⁷, X¹⁸, X¹⁹, X²⁰, X²¹, X²³, X²⁴ and X²⁵ are as defined for Formula **I,** and pharmaceutically acceptable salts thereof.

In one aspect, novel compounds of Formula **I** include:
[2-(benzylamino)pyridin-3-yl](5-chloro-2,3-dihydro-1H-indol-1-yl)methanone;
(5-chloro-2,3-dihydro-1*H-*indol-1-yl)[2-(phenylamino)pyridin-3-yl]methanone;
(5-chloro-2,3-dihydro-1*H-*indol-1-yl)[2-[(pyridin-2-ylmethyl)amino]pyridin-3-yl]methanone;
(5-chloro-2,3-dihydro-1*H*-indol-1-yl)[2-[(2-phenylethyl)amino]pyridin-3-yl]-methanone;
(5-chloro-2,3-dihydro-1*H-*indol-1-yl)[2-[(pyridin-3-ylmethyl)amino]pyridin-3-yl]methanone;
(5-chloro-2,3-dihydro-1*H-*indol-1-yl)[2-[[2-(pyridin-2-yl)ethyl]amino]-pyridin-3-yl]methanone;
(5-chloro-2,3-dihydro-1*H-*indol-1-yl)[2-[(4-fluorobenzyl)amino]pyridin-3-yl]-methanone;
(5-chloro-2,3-dihydro-1*H-*indol-1-yl)[2-[(1-(4-fluorophenyl)ethyl)amino]-pyridin-3-yl]methanone;
[2-(benzylamino)pyridin-3-yl](2,3-dihydro-1*H-*indol-1-yl)methanone;
[3-(benzylamino)pyridazin-4-yl](5-chloro-2,3-dihydro-1H-indol-1-yl)methanone;
(5-chloro-2,3-dihydro-1H-indol-1-yl)[2-[(4-fluorobenzyl)amino]pyrazin-3-yl]-methanone;
(5-chloro-2,3-dihydro-1H-indol-1-yl)[2-(2,5-difluorobenzylamino)pyridin-3-yl]-methanone;
(5-chloro-2,3-dihydro-1H-indol-1-yl)[2-(3,4-difluorobenzylamino)pyridin-3-yl]-methanone;
(5-chloro-2,3-dihydro-1H-indol-1-yl)[2-(2,4-difluorobenzylamino)pyridin-3-yl]-methanone;
(5-chloro-2,3-dihydro-1H-indol-1-yl)[2-(cyclopropylmethylamino)pyridin-3-yl]-methanone;
(5-chloro-2,3-dihydro-1H-indol-1-yl)[2-(2-propyn-1-ylamino)pyridin-3-yl]-methanone;
(5-fluoro-2,3-dihydro-1H-indol-1-yl)[2-(4-fluorobenzylamino)pyridin-3-yl]-methanone; and
(5-chloro-2,3-dihydro-1H-indol-1-yl)[2-[(pyridin-4-ylamino)pyridin-3-yl]-methanone, [0089] and pharmaceutically acceptable salts thereof.

In one aspect, the following compounds are useful in treating disorders amenable to modulation of α7 nAChRs including neurodegenerative diseases, senile dementias, schizophrenia, Alzheimer's disease, learning, cognition and attention deficits, memory loss, Lewy Body dementia, attention-deficit disorder, attention deficit hyperactivity disorder, anxiety, mania, manic depression, Parkinson's disease, Huntington's disease, depression, amyotrophic lateral sclerosis, brain inflammation, cognitive deficit due to traumatic brain injury ("TBI") and Tourette's syndrome; in addition, the following compounds of the present invention may be used to treat immune system disorders, such as, but not limited to, type I diabetes, multiple sclerosis, rheumatoid arthritis; the following compounds may be used in other indications including pain, inflammation, septic shock, ulcerative colitis, Crohn's disease, irritable bowel syndrome and jet lag; in addition, the following compounds may be used to treat a cognitive disorder related to learning or memory including mild cognitive impairment, age related cognitive decline, senile dementia and Alzheimer's disease:
[2-(benzylamino)pyridin-3-yl](5-chloro-2,3-dihydro-1H-indol-1-yl)methanone (compound 1);
(5-chloro-2,3-dihydro-1*H*-indol-1-yl)[2-(phenylamino)pyridin-3-yl]methanone (compound 2);
(5-chloro-2,3-dihydro-1*H*-indol-1-yl)[2-[(pyridin-2-ylmethyl)amino]pyridin-3-yl]methanone (compound 3);
(5-chloro-2,3-dihydro-1*H*-indol-1-yl)[2-[(2-phenylethyl)amino]pyridin-3-yl]methanone (compound 4);
(5-chloro-2,3-dihydro-1*H*-indol-1-yl)[2-[(pyridin-3-ylmethyl)amino]pyridin-3-yl]methanone (compound 5);
(5-chloro-2,3-dihydro-1*H*-indol-1-yl)[2-[[2-(pyridin-2-yl)ethyl]amino]-pyridin-3-yl]methanone (compound 6);
(5-chloro-2,3-dihydro-1*H*-indol-1-yl)[2-[(4-fluorobenzyl)amino]pyridin-3-yl]methanone (compound 7);
(5-chloro-2,3-dihydro-1*H*-indol-1-yl)[2-[(1-(4-fluorophenyl)ethyl)-amino]pyridin-3-yl]methanone (compound 8);
[2-(benzylamino)pyridin-3-yl](2,3-dihydro-1*H*-indol-1-yl)methanone (compound 9);
[3-(benzylamino)pyridazin-4-yl](5-chloro-2,3-dihydro-1H-indol-1-yl)methanone (compound 10);
(5-chloro-2,3-dihydro-1H-indol-1-yl)[2-[(4-fluorobenzyl)amino]pyrazin-3-yl]methanone (compound 11);
N-(4-ethoxyphenyl)-2-[(2-phenylethyl)amino]pyridine-3-carboxamide (compound 12);
N-phenyl-2-[(2-phenylethyl)amino]pyridine-3-carboxamide (compound 13);
N-(4-chlorophenyl)-2-[(2-phenylethyl)amino]pyridine-3-carboxamide (compound 14);
2-(benzylamino)-*N*-(4-ethoxyphenyl)pyridine-3-carboxamide (compound 15);
N-(4-ethoxyphenyl)-2-(propylamino)pyridine-3-carboxamide (compound 16);
*N*-(4-hydroxyphenyl)-2-[(2-phenylethyl)amino]pyridine-3-carboxamide (compound 17);
*N*-(4-ethoxyphenyl)-2-(phenylamino)pyridine-3-carboxamide (compound 18);
2-[(cyclohexylmethyl)amino]-*N*-(4-ethoxyphenyl)pyridine-3-carboxamide (compound 19);
3-(benzylamino)-*N*-(4-ethoxyphenyl)pyridine-4-carboxamide (compound 20);
4-(benzylamino)-*N*-(4-ethoxyphenyl)pyridine-3-carboxamide (compound 21);
3-(benzylamino)-6-chloro-*N*-(4-ethoxyphenyl)pyridazine-4-carboxamide (compound 22);
2-(benzylamino)-*N*-(4-chlorophenyl)pyridine-3-carboxamide (compound 23);
2-(benzylamino)-*N*-[4-(trifluoromethyl)phenyl]pyridine-3-carboxamide (compound 24);
2-(benzylamino)-*N*-(4-fluorophenyl)pyridine-3-carboxamide (compound 25);
*N*-(4-chlorophenyl)-5-[2-[(4-chlorophenyl)ethyl]amino]-3-methyl-4-isoxazolecarbox-amide (compound 26);
(5-chloro-2,3-dihydro-1H-indol-1-yl) [2-(2,5-difluorobenzylamino)pyridin-3-yl]-methanone (compound 27);
(5-chloro-2,3-dihydro-1H-indol-1-yl)[2-(3,4-difluorobenzylamino)pyridin-3-yl]-methanone (compound 28);
(5-chloro-2,3-dihydro-1H-indol-1-yl)[2-(2,4-difluorobenzylamino)pyridin-3-yl]-methanone (compound 29);
(5-chloro-2,3-dihydro-1H-indol-1-yl)[2-(cyclopropylmethylamino)pyridin-3-yl]-methanone (compound 30);
(5-chloro-2,3-dihydro-1H-indol-1-yl)[2-(2-propyn-1-ylamino)pyridin-3-yl]methanone (compound 31);
(5-fluoro-2,3-dihydro-1H-indol-1-yl)[2-(4-fluorobenzylamino)pyridin-3-yl]methanone (compound 32); and
(5-chloro-2,3-dihydro-1H-indol-1-yl)[2-[(pyridin-4-ylamino)pyridin-3-yl]methanone (compound 33), and pharmaceutically acceptable salts thereof.

In another aspect, there is provided pharmaceutical compositions comprising a compound of Formulae **I-VII,** and pharmaceutically acceptable salts thereof.

In yet another aspect there is provided a compound of Formulae **I-VII** or a pharmaceutically acceptable salt thereof for use in the treatment of disorders amenable to modulation of α7 nAChR. In one embodiment, the disorder is a neurodegenerative disorder. In another embodiment, the disorder is a senile dementia. In another embodiment, the disorder is schizophrenia. In another embodiment, the disorder is a cognition deficit disorder. In another embodiment, the disorder is Alzheimer's disease. In another embodiment, the disorder includes cognition and attention deficits, memory loss, Lewy Body dementia, attention-deficit disorder, attention deficit hyperactivity disorder, anxiety, mania, manic depression, Parkinson's disease, Huntington's disease, depression, amyotrophic lateral sclerosis, brain inflammation, cognitive deficit due to traumatic brain injury, and Tourette's syndrome. In another embodiment, the disorder is, pain, inflammation, septic shock, ulcerative colitis, Crohn's disease, and irritable bowel syndrome. In another embodiment the disorder is inflammation. In another embodiment, the disorder is depression and the treatment comprising the administration of a compound of Formulae **I-VII** or a pharmaceutically acceptable salt thereof and the administration of an SSRI drug, a drug that augments5-HT release or blocks 5-HT reuptake. In yet another embodiment, the disorder is an immune system disorder.

In another aspect, there is provided a compound of Formulae **I-VII** or a pharmaceutically acceptable salt thereof for use in the treatment of disorders related to learning and memory such as mild cognitive impairment, age related cognitive decline, senile dementia, and Alzheimer's disease. In one embodiment the treatment of such disorders is achieved via modulation of mono and divalent cation conductance through the site mediating the action of a compound of Formulae **I-VII** or a pharmaceutically acceptable salt thereof.

In another aspect, there is a provided a compound of Formulae **I-VII,** a pharmaceutically acceptable salt or solvate thereof for use in the treatment of Fragile X, autism spectrum disorder, Angelman's syndrome, Rett syndrome, Prader Willi
syndrome and Down's syndrome.

For use in medicine, the salts of the compounds of Formulae **I-VII** will be pharmaceutically acceptable salts. Other salts may, however, be useful in the preparation of the compounds according to the invention or of their pharmaceutically acceptable salts. Suitable pharmaceutically acceptable salts of the compounds of this invention include acid addition salts which may, for example, be formed by mixing a solution of the compound according to the invention with a solution of a pharmaceutically acceptable acid such as hydrochloric acid, sulfuric acid, methanesulfonic acid, fumaric acid, maleic acid, succinic acid, acetic acid, benzoic acid, oxalic acid, citric acid, tartaric acid, or phosphoric acid. Furthermore, where the compound comprises an acidic moiety, suitable pharmaceutically acceptable salts thereof may include alkali metal salts, e.g. sodium or potassium salts; alkaline earth metal salts, e.g. calcium or magnesium salts; and salts formed with suitable organic ligands, e.g. quaternary ammonium salts. Standard methods for the preparation of pharmaceutically acceptable salts and their formulations are well known in the art, and are disclosed in various references, including for example, "Remington: The Science and Practice of Pharmacy", A. Gennaro, ed., 20th edition, Lippincott, Williams & Wilkins, Philadelphia, PA.

Disclosed herein are prodrugs of the compounds of Formulae **I-VII** above. In general, such prodrugs will be functional derivatives of the compounds of Formulae **I-VII** that are readily convertible *in vivo* into the required compound of Formulae **I-VII.** Conventional procedures for the selection and preparation of suitable prodrug derivatives are described, for example, in Design of Prodrugs, ed. H. Bundgaard, Elsevier, 1985. Such prodrugs include but are not limited to ester prodrugs from alcohols and acids, phosphate prodrugs of alcohols, and N-oxide derivatives of heteroaryl moieties. The prodrug can be formulation to achieve a goal of improved chemical stability, improved patient acceptance and compliance, improved bioavailability, prolonged duration of action, improved organ selectivity, improved formulation (e.g., increased hydrosolubility), and/or decreased side effects (e.g., toxicity).

Where the compounds of the present invention have at least one asymmetric center, they may accordingly exist as enantiomers. Where the compounds possess two or more asymmetric centers, they may additionally exist as diastereoisomers. It is
to be understood that all such stereoisomers and mixtures thereof in any proportion are encompassed within the scope of the present invention. Where the compounds possess geometrical isomers, all such isomers and mixtures thereof in any proportion are encompassed within the scope of the present invention.

Tautomers of the compounds of the invention are encompassed by the present application. Thus, for example, a carbonyl includes its hydroxyl tautomer.

### Examples

Standard procedures and chemical transformation and related methods are well known to one skilled in the art, and such methods and procedures have been described, for example, in standard references such as Fiesers' Reagents for Organic Synthesis, John Wiley and Sons, New York, NY, 2002; Organic Reactions, vols. 1-83, John Wiley and Sons, New York, NY, 2006; March J. and Smith M.: Advanced Organic Chemistry, 6th ed., John Wiley and Sons, New York, NY; and Larock R.C.: Comprehensive Organic Transformations, Wiley-VCR Publishers, New York, 1999.

Reactions using compounds having functional groups may be perfomled on compounds with functional groups that may be protected. A "protected" compound or derivatives means derivatives of a compound where one or more reactive site or sites or functional groups are blocked with protecting groups. Protected derivatives are useful in the preparation of the compounds of the present invention. An example of a comprehensive text listing suitable protecting groups may be
found in T. W. Greene, Protecting Groups in Organic Synthesis, 3rd edition, John Wiley & Sons, Inc. 1999.

Compounds of Formula **II** can be prepared as shown in Scheme 1, starting with indoles of Formula **A.** Reduction with sodium cyanoborohydride in acetic acid followed by basic workup affords the corresponding indolines **B.** Addition of a 2-chloronicotinoyl chloride in the presence of base affords the amide C. Further reaction with an appropriate amine (R³NH₂) leads to molecules of Formula **II**. 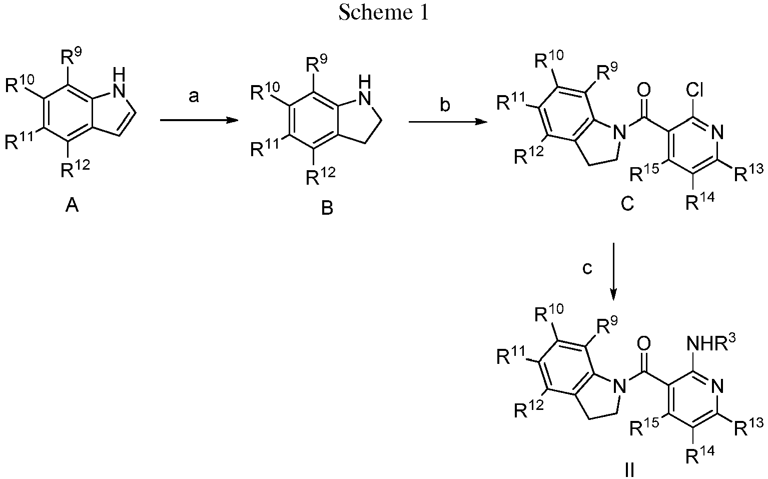 Reagents/Solvents: a. NaBH₃CN/HOAc, then aq. NaOH b. 2-chloronicotinoyl chloride/CH₂Cl₂/pyridine c. R³NH₂, DMSO, 130 °C.

Compounds of Formula **IV** can be prepared as shown in Scheme 2, starting with a 3,6-dichloropyridazine-4-carboxylic acid **D.** Reaction with an amine H₂NR³ and hydrogenolysis gave the acid **F.** Coupling with the indoline **B** then gave compounds of Formula **IV.** 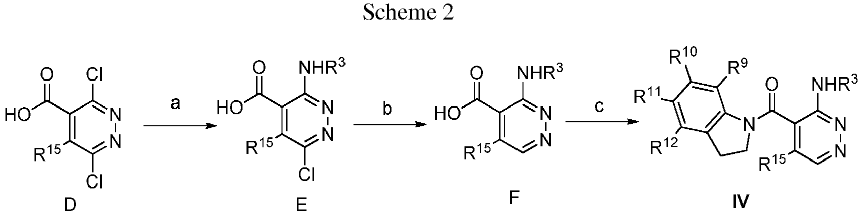 Reagents/Solvents: a. H₂NR³/DMSO, 100°C b. Pd/C, ammonium formate/MeOH 50 °C c. Indoline **B**/ DMF/Et₃N/O-benzotriazol-1-yl-N,N,N',N'-tetramethyluronium tetrafluoroborate.

Starting with a methyl 3-aminopyrazine-2-carboxylate (Scheme 3, **G**), reductive amination with an aldehyde using sodium triacetoxyborohydride affords the akylated product **H.** Hydrolysis to the acid **I** following by coupling with an indoline **B** affords compounds of Formula **V.** 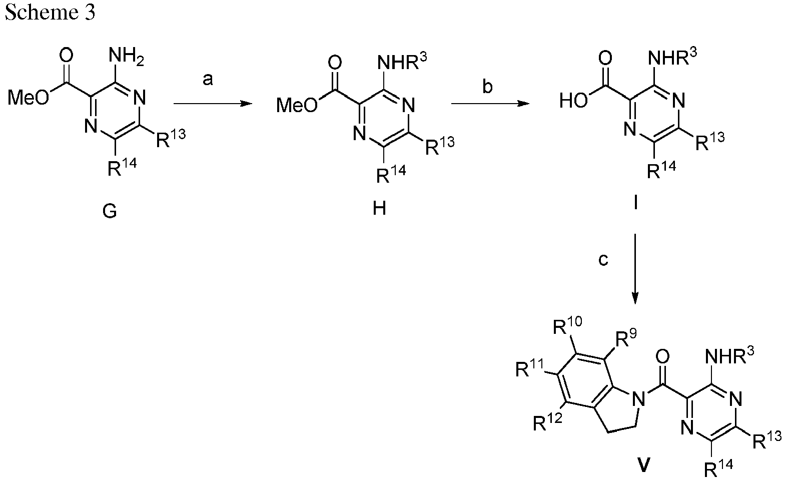 Reagents/Solvents: a. Aldehyde/1,2-dichloroethane/NaHB(OAc)₃/HOAc b. NaOH/water/MeOH c. Indoline B, DMF/Et₃N/O-benzotriazol-1-yl-N,N,N',N'-tetramethyluronium tetrafluoroborate.

Compounds of Formula **VII** can be prepared as shown in Schemes 4 and 5. Acylation of a 3-substituted-5-aminoisoxazole followed by reduction with LiAlH₄ gave the alkylated isoxazole which was reacted with an isocyanate to give the isoxazole-4-carboxamide **VII** (Scheme 4). 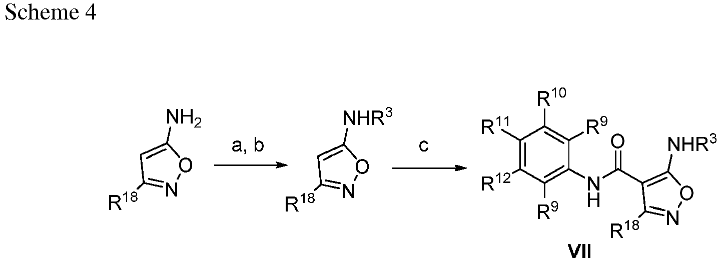 Reagents/Solvents: a. Acid chloride/ether/sat. aq. NaHCO₃ solution b. LiAlH₄/THF c. Arylisocyanate/ toluene/heat.

Compounds of Formula **VII** can also be prepared as shown in Scheme 5. Reaction of a 2-chloronicotinoyl chloride with an aniline gave the expected amide. Reaction with an amine (H₂NR³) in DMSO then afforded the compound of Formula **VII**. 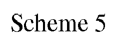 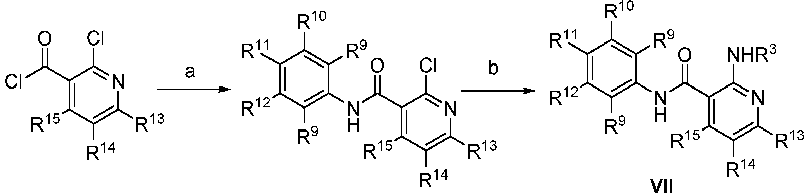 Reagents/Solvents: a. Substitiuted aniline/pyridine/CH₂Cl₂ b. H₂NR³/DMSO, heat.

**OOCYTE ELECTROPHYSIOLOGY:** Individual compounds were tested for modulation of submaximal nicotine-evoked currents at α7 nAChRs using oocytes expressing human receptors. For each oocyte, the maximal nicotine-evoked currents were determined in response to 3 µM nicotine. All other currents were scaled to this value. The concentration of nicotine was adjusted to evoke a fractional current of approximately 0.05 (5% of max, or "EC₅"), and this concentration of nicotine was used to generate EC₅ control currents. Increasing concentrations of test compounds were applied to oocytes alone (pretreatment) and then in combination with the EC₅ concentration of nicotine (co-application). This protocol allowed measurement of both direct effects of test compounds on α7 nAChRs, and modulatory effects of compounds on nicotine-evoked responses. mRNA was prepared and stored using conventional techniques from cDNA clones encoding the human nicotinic receptor subunits. Preparation, micro-injection and maintenance of oocytes were performed as reported in detail previously (Whittemore et al., Mol. Pharmacol. 50: 1364-1375, 1996). Individual oocytes were injected with 5 - 50 ng of each subunit mRNA. For multiple subunit combinations, the mRNA ratios are: (1) α4β2 and α3β4 nAChRs (a 1:1 mixture); Following injections, oocytes were maintained at 16-17 °C in Barth's medium. Two-electrode voltage clamp recordings were made 3-14 days following mRNA injections at a holding voltage of -70 mV unless specified. The nicotinic recordings were done in Ca⁺⁺-free Ringer solution (mM: NaCl, 115; KCl, 2; BaCl₂, 1.8; HEPES, 5; pH 7.4) to limit Ca⁺⁺-activated chloride and muscarinic currents. Drug and wash solutions were applied using a microcapillary "linear array" (Hawkinson et al., Mol. Pharmacol. 49: 897-906, 1996) in order to allow rapid application of agonists. Currents were recorded on a chart recorder and/or PC-based computer for subsequent analysis. Test compounds were made up in DMSO over a concentration range of 0.001 - 10 mM and diluted 1000-3000-fold into the appropriate saline just prior to testing (final [DMSO] ≤ 0.1%). The concentration-dependence of modulation was analyzed using GraphPad "Prism" curve-fitting software.

Positive allosteric modulators can also be assayed by imaging of calcium flux through α7 nAChR transiently expressed in a cell line, including HEK-293 and cell cultered neurons. (see for example international published application WO 2006/071184)

### Example 1

### [2-(Benzylaminio)pyridin-3yl](5-chloro-2,3-dihydro-1H-indol-1-yl)methanone

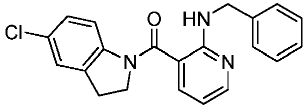

**5-Chloroindoline.** To a solution of 5-chloroindole (2.18 g, 14.4 mmol) in glacial acetic acid (38 mL) under argon at 15-17°C was added in one portion sodium cyanoborohydride (2.8 g, 44.6 mmol). After the addition, the mixture was stirred for 2 hours at 15-17°C. Water (200 mL) was then added and the mixture was cooled in an ice-bath. Sodium hydroxide pellets were added slowly until a strongly basic pH was obtained. The mixture was extracted with diethyl ether. The organic layer was washed with water, brine and dried over magnesium sulfate to yield 5-chloroindoline (2.04 g, 13.3 mmol, 92%). MS: 154 (MH⁺).

**(5-Chloro-2,3-dihydro-1*H*-indol-1-yl)(2-chloropyridin-3-yl)methanone.** To a stirred solution of 2-chloronicotinoyl chloride (2.57 g, 14.6 mmol) in methylene chloride (30 mL) at 0°C under argon was added pyridine (1.29 mL, 16 mmol) followed by the dropwise addition of 5-choloroindoline (2.04 g, 13.3 mmol) in methylene chloride (10 mL). The reaction mixture was then stirred at room temperature overnight. It was quenched with saturated aqueous sodium bicarbonate and extracted with methylene chloride. The organic layer was washed with brine, dried over magnesium sulfate and evaporated. The residue was purified by flash chromatography (2% MeOH/HCCl₃) to yield (5-chloro-2,3-dihydro-1*H*-indol-1-yl)(2-chloropyridin-3-yl)methanone (3.77 g, 97%). MS: 293 (MH⁺).

### [2-(Benzylamino)pyridin-3-yl](5-chloro-2,3-dihydro-1H-indol-1-yl)methanone.

To a stirred solution of (5-chloro-2,3-dihydro-1*H*-indol-1-yl)(2-chloropyridin-3-yl)methanone (1.0 mmol, 293 mg) in DMSO (4 mL) was added benzylamine (4 mmol, 0.44 mL). The mixture was heated to 130°C and was stirred overnight. After cooling, it was diluted with acetonitrile and purified by reverse-phase HPLC to yield [2-(benzylamino)-pyridin-3-yl](5-chloro-2,3-dihydro-1*H*-indol-1-yl)methanone (200 mg). MS: 364 (MH⁺).

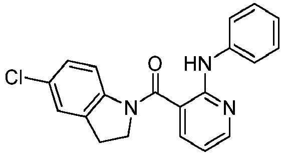

### (5-Chloro-2,3-dihydro-1H-indol-1-yl)[2-(phenylamino)pyridin-3-yl]methanone.

(5-Chloro-2,3-dihydro-1*H*-indol-1-yl)[2-(phenylamino)pyridin-3-yl]methanone was prepared using the procedure described for [2-(benzylamino)pyridin-3-yl](5-chloro-2,3-dihydro-1*H*-indol-1-yl)methanone except benzylamine was replaced with aniline. MS: 350 (MH⁺).

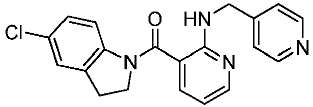

**(5-Chloro-2,3-dihydro-1*H*-indol-1-yl)[2-[(pyridin-4-ylmethyl)amino]pyridin-3-yl]methanone.** (5-Chloro-2,3-dihydro-1*H*-indol-1-yl)[2-[(pyridin-4-ylmethyl)amino]-pyridin-3-yl]methanone was prepared using the procedure described for [2-(benzylamino)pyridin-3-yl](5-chloro-2,3-dihydro-1*H*-indol-1-yl)methanone except benzylamine was replaced with 4-(aminomethyl)pyridine. MS: 365 (MH⁺).

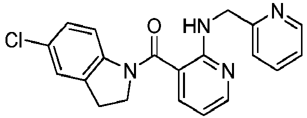

**(5-Chloro-2,3-dihydro-1*H*-indol-1-yl)[2-[(pyridin-2-ylmethyl)amino]pyridin-3-yl]-methanone.** (5-Chloro-2,3-dihydro-1*H*-indol-1-yl)[2-[(pyridin-2-ylmethyl)amino]-pyridin-3-yl]methanone was prepared using the procedure described for [2-(benzyl-amino)pyridin-3-yl](5-chloro-2,3-dihydro-1*H*-indol-1-yl)methanone except benzylamine was replaced with 2-(aminomethyl)pyridine. MS: 365 (MH⁺).

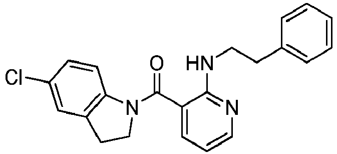

**(5-Chloro-2,3-dihydro-1*H*-indol-1-yl)[2-[(2-phenylethyl)amino]pyridin-3-yl]-methanone.** (5-Chloro-2,3-dihydro-1*H*-indol-1-yl)[2-[(2-phenylethyl)amino]pyridin-3-yl]methanone was prepared using the procedure described for [2-(benzylamino)-pyridin-3-yl](5-chloro-2,3-dihydro-1*H*-indol-1-yl)methanone except benzylamine was replaced with phenethylamine. MS: 378 (MH⁺).

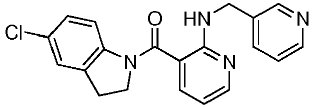

**(5-Chloro-2,3-dihydro-1*H*-indol-1-yl)[2-[(pyridin-3-ylmethyl)amino]pyridin-3-yl]-methanone.** (5-Chloro-2,3-dihydro-1*H*-indol-1-yl)[2-[(pyridin-2-ylmethyl)amino]-pyridin-3-yl]methanone was prepared using the procedure described for [2-(benzyl-amino)pyridin-3-yl](5-chloro-2,3-dihydro-1*H*-indol-1-yl)methanone except benzylamine was replaced with 3-(aminomethyl)pyridine. MS: 365 (MH⁺).

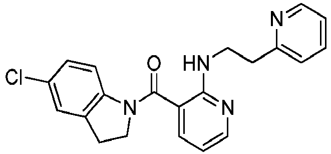

**(5-Chloro-2,3-dihydro-1*H*-indol-1-yl)[2-[2-[(pyridin-2-yl)ethyl]amino]pyridin-3-yl)]-methanone.** (5-Chloro-2,3-dihydro-1*H*-indol-1-yl)[2-[2-[(pyridin-2-yl)ethyl]amino]-pyridin-3-yl)methanone was prepared using the procedure described for [2-(benzyl-amino)pyridin-3-yl](5-chloro-2,3-dihydro-1*H*-indol-1-yl)methanone except benzylamine was replaced with 2-(2-aminoethyl)pyridine. MS: 379 (MH⁺).

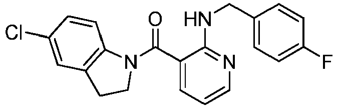

**(5-Chloro-2,3-dihydro-1*H*-indol-1-yl)[2-[(4-fluorobenzyl)amino]pyridin-3-yl]-methanone.** (5-Chloro-2,3-dihydro-1*H*-indol-1-yl)[2-[(4-fluorobenzyl)amino]pyridin-3-yl]methanone was prepared using the procedure described for [2-(benzylamino)-pyridin-3-yl](5-chloro-2,3-dihydro-1*H*-indol-1-yl)methanone except benzylamine was replaced with 4-fluorobenzylamine. MS: 382 (MH⁺).

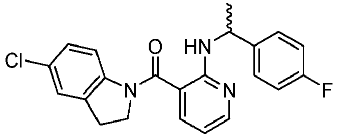

**(5-Chloro-2,3-dihydro-1*H*-indol-1-yl)[2-[(1-(4-fluorophenyl)ethyl)amino]pyridin-3-yl]methanone.** (5-Chloro-2,3-dihydro-1*H*-indol-1-yl)[2-[(1-(4-fluorophenyl)ethyl)-amino]pyridin-3-yl]methanone was prepared using the procedure described for [2-(benzylamino)pyridin-3-yl](5-chloro-2,3-dihydro-1*H*-indol-1-yl)methanone except benzylamine was replaced with α-methyl-4-fluorobenzylamine. MS: 396 (MH⁺).

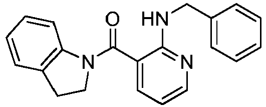

**[2-(Benzylamino)pyridin-3-yl](2,3-dihydro-1*H*-indol-1-yl)methanone.** [2-(Benzylamino)pyridin-3-yl](2,3-dihydro-1*H*-indol-1-yl)methanone was prepared using the procedure described for [2-(benzylamino)pyridin-3-yl](5-chloro-2,3-dihydro-1*H-*indol-1-yl)methanone except 5-chloroindoline was replaced with indoline. MS: 330 (MH⁺).

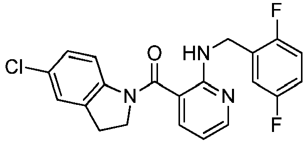

**(5-Chloro-2,3-dihydro-1H-indol-1-yl)[2-(2,5-difluorobenzylamino)pyridin-3-yl]-methanone.** (5-Chloro-2,3-dihydro-1H-indol-1-yl)[2-(2,5-difluorobenzylamino)pyridin-3-yl]-methanone was prepared using the procedure described for [2-(benzylamino)pyridin-3-yl](5-chloro-2,3-dihydro-1*H*-indol-1-yl)methanone except benzylamine was replaced with 2,5-difluorobenzylamine. MS: 400 (MH⁺).

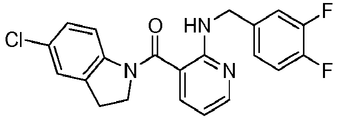

**(5-Chloro-2,3-dihydro-1H-indol-1-yl)[2-(3,4-difluorobenzylamino)pyridin-3-yl]-methanone.** (5-Chloro-2,3-dihydro-1H-indol-1-yl)[2-(3,4-difluorobenzylamino)pyridin-3-yl]-methanone was prepared using the procedure described for [2-(benzylamino)pyridin-3-yl](5-chloro-2,3-dihydro-1*H*-indol-1-yl)methanone except benzylamine was replaced with 3,4-difluorobenzylamine. MS: 400 (MH⁺).

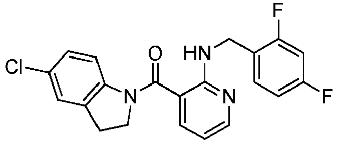

**(5-Chloro-2,3-dihydro-1H-indol-1-yl)[2-(2,4-difluorobenzylamino)pyridin-3-yl]-methanone.** (5-Chloro-2,3-dihydro-1H-indol-1-yl)[2-(2,4-difluorobenzylamino)pyridin-3-yl]-methanone was prepared using the procedure described for [2-(benzylamino)pyridin-3-yl](5-chloro-2,3-dihydro-1*H*-indol-1-yl)methanone except benzylamine was replaced with 2,4-difluorobenzylamine. MS: 400 (MH⁺).

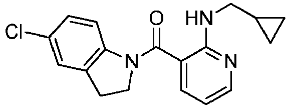

**(5-Chloro-2,3-dihydro-1H-indol-1-yl)[2-(cyclopropylmethylamino)pyridin-3-yl]-methanone.** (5-Chloro-2,3-dihydro-1H-indol-1-yl)[2-(cyclopropylmethylamino)pyridin-3-yl]methanone was prepared using the procedure described for [2-(benzylamino)pyridin-3-yl](5-chloro-2,3-dihydro-1*H*-indol-1-yl)methanone except benzylamine was replaced with cyclopropylmethylamine. MS: 328 (MH⁺).

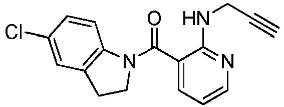

**(5-Chloro-2,3-dihydro-1H-indol-1-yl)[2-(2-propyn-1-ylamino)pyridin-3-yl]-methanone.** (5-Chloro-2,3-dihydro-1H-indol-1-yl)[2-(2-propyn-1-ylamino)pyridin-3-yl]methanone was prepared using the procedure described for [2-(benzylamino)pyridin-3-yl](5-chloro-2,3-dihydro-1*H*-indol-1-yl)methanone except benzylamine was replaced with propargylamine. MS: 312 (MH⁺).

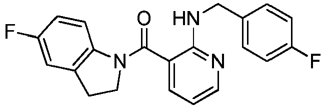

**(5-Fluoro-2,3-dihydro-1H-indol-1-yl)[2-(4-fluorobenzylamino)pyridin-3-yl]-methanone.** (5-Fluoro-2,3-dihydro-1H-indol-1-yl)[2-(4-fluorobenzylamino)pyridin-3-yl]methanone was prepared using the procedure described for [2-(benzylamino)pyridin-3-yl](5-chloro-2,3-dihydro-1*H*-indol-1-yl)methanone except 5-chloroindoline was replaced with 5-fluoroindoline and benzylamine was replaced with 4-fluorobenzylamine. MS: 366 (MH⁺).

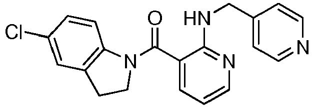

**(5-Chloro-2,3-dihydro-1H-indol-1-yl)[2-[(pyridin-4-ylamino)pyridin-3-yl]-methanone.** (5-Chloro-2,3-dihydro-1H-indol-1-yl)[2-[(pyridin-4-ylamino)pyridin-3-yl]methanone was prepared using the procedure described for [2-(benzylamino)pyridin-3-yl](5-chloro-2,3-dihydro-1*H*-indol-1-yl)methanone except benzylamine was replaced with 4-(aminomethyl)pyridine. MS: 365 (MH⁺).

### Example 2

### [3-(Benzylamino)pyridazin-4-yl](5-chloro-2,3-dihydro-1H-indol-1-yl)methanone

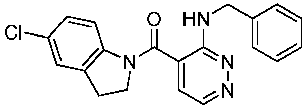

**3-(Benzylamino)-6-chloropyridazine-4-carboxylic acid.** To a stirred solution of 3,6-dichloropyridazine-4-carboxylic acid (579 mg, 3.00 mmol) in dimethylsulfoxide (3 mL) under argon was added benzylamine (0.65 mL, 6.0 mmol). The reaction mixture was then stirred at 100°C for 12 hours. After cooling to room temperature, the mixture was diluted with methanol and purified by reverse-phase HPLC to yield 3-(benzylamino)-6-chloropyridazine-4-carboxylic acid. MS: 264 (MH⁺).

**3-(Benzylamino)pyridazine-4-carboxylic acid.** To a stirred solution of 3-(benzylamino)-6-chloropyridazine-4-carboxylic acid (451 mg, 1.7 mmol) in methanol (30 mL) was added ammonium formate (270 mg, 3.40 mmol) and 10% Pd/C (100 mg). The reaction mixture was then stirred at 50°C for 3 hours. After cooling to room temperature, the mixture was filtered and the solvent was removed under vacuum. The residue was washed with water and filtered to yield 3-(benzylamino)pyridazine-4-carboxylic acid. MS: 230 (MH⁺).

### [3-(Benzylamino)pyridazin-4-yl](5-chloro-2,3-dihydro-1H-indol-1-yl)methanone.

To a stirred solution of 3-(benzylamino)pyridazine-4-carboxylic acid (157 mg, 0.68 mmol) in DMF (4.5 mL) was added O-benzotriazol-1-yl-N,N,N',N'-tetramethyluronium tetrafluoroborate (270 mg, 0.88 mmol), triethylamine (1.36 mmol, 0.2 mL) and 5-chloroindoline (205 mg, 1.36 mmol). The reaction mixture was then stirred at room temperature for 12 hours. It was diluted with water and extracted with methylene chloride. The solvent was removed under vacuum and the residue was purified by reverse-phase HPLC to yield [3-(benzylamino)pyridazin-4-yl](5-chloro-2,3-dihydro-1*H*-indol-1-yl)methanone. MS: 365 (MH⁺).

### Example 3

### (5-Chloro-2,3-dihydro-1H-indol-1-yl)[2-[(4-fluorobenzyl)amino]pyrazin-3-yl]methanone

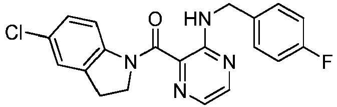

**Methyl 2-[(4-fluorophenylmethylene)amino]pyrazine-3-carboxylate.** A solution of methyl 2-aminopyrazine-3-carboxylate in EtOH was treated with an excess of 4-fluorobenzaldehyde and heated at reflux for 72 h. The solvent was removed in vacuo and the residue was carried on without purification.

**Methyl 2-[(4-fluorobenzyl)amino]pyrazine-3-carboxylate.** Methyl 2-[(4-fluorophenylmethylene)amino]pyrazine-3-carboxylate (1.263 g, 4.87 mmol) in 1,2-dichloroethane (10 mL) was treated with sodium triacetoxyborohydride (3.05 g, 14.4 mmol) and 0.3 mL of glacial HOAc. After stirring at rt overnight, an additional 3.12 g of triacetoxyborohydride, 20 mL of 1,2-dichloroethane and 0.5 mL glacial HOAc were added. After stirring for an additional 3 days, the reaction was quenched with cold water and a sat. aq. NaHCO₃ solution was added. The aqueous layer was washed with CH₂Cl₂ (2 x 50 mL) and the pooled organic layers were washed with water and a sat. aq. NaCl solution. After drying (MgSO₄), the mixture was filtered and conc. in vacuo. The crude product was purified by flash silica gel chromatography (2.5% MeOH/CH₂Cl₂) affording 340 mg of the product as a light yellow solid.

**2-[(4-Fluorobenzyl)amino]pyrazine-3-carboxylic acid.** A solution of methyl 2-[(4-fluorobenzyl)amino]pyrazine-3-carboxylate (340 mg, 1.30 mmol) in 11 mL of MeOH was treated with a IN aq. NaOH solution (4 mL). The solution was heated at 60 °C for 1 h and then allowed to cool to rt. Most of the MeOH was removed in vacuo and the residue was treated with 25 mL of cold water and 5 mL of a 1.2M aq. HCl solution. The ppt that formed was isolated by filtration and washed with water, affording 346 mg of the acid.

**(5-Chloro-2,3-dihydro-1H-indol-1-yl)[2-[(4-fluorobenzyl)amino]pyrazin-3-yl]-methanone.** A solution of 2-[(4-fluorobenzyl)amino]pyrazine-3-carboxylic acid (337 mg, 1.37 mmol) in DMF (9 mL) was treated with O-benzotriazol-1-yl-N,N,N',N'-tetramethyluronium tetrafluoroborate (547 mg, 1.70 mmol) and Et₃N (0.5 mL). Neat 5-chloroindoline (0.31 mL, 2.63 mmol) was added and the cloudy solution was stirred at rt overnight. The reaction was cooled in an ice-water bath and diluted with cold water. The resulting ppt was washed with a IN aq HCl solution (3 x 10 mL) and water (3 x 10 mL) affording 468 mg of the title compound as a solid. MS: 383 (MH⁺).

### Example 4

### N-(4-Ethoxyphenyl)-2-[(2-phenylethyl)amino]pyridine-3-carboxamide

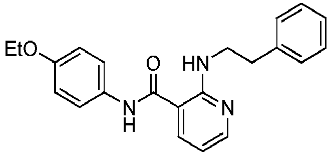

**2-Chloro-*N*-(4-ethoxyphenyl)pyridine-3-carboxamide.** To a stirred solution of 2-chloronicotinoyl chloride (528 mg, 3.00 mmol) in methylene chloride (10 mL) at 0°C under argon was added pyridine (0.27 mL, 3.35 mmol) followed by the dropwise addition of p-phenetidine (0.4 mL, 3.1 mmol). The reaction mixture was then stirred at room temperature for 2 hours. It was quenched with saturated aqueous sodium bicarbonate and extracted with methylene chloride. The organic layer was washed with brine, dried over magnesium sulfate and evaporated to yield 2-chloro-*N*-(4-ethoxyphenyl)pyridine-3-carboxamide which was used without further purification.

***N*-(4-Ethoxyphenyl)-2-[(2-phenylethyl)amino]pyridine-3-carboxamide.** 2-Chloro-***N*-(4-ethoxyphenyl)pyridine-3-carboxamide** was dissolved in DMSO (10 mL) and phenethylamine (0.34 mL, 2.7 mmol) was added. The reaction mixture was heated at 130°C and was stirred overnight. After cooling, the reaction was diluted with acetonitrile and purified by reverse-phase HPLC to yield of *N*-(4-ethoxyphenyl)-2-[(2-phenylethyl)-amino]pyridine-3-carboxamide. MS: 362 (MH⁺).

**2-Chloro-N-phenylpyridine-3-carboxamide.** 2-Chloro-*N*-phenylpyridine-3-carbox-amide was prepared using the procedure described for 2-chloro-*N*-(4-ethoxyphenyl)pyridine-3-carboxamide except p-phenetidine was replaced with aniline.

**2-Chloro-*N*-(4-chlorophenyl)pyridine-3-carboxamide.** 2-Chloro-*N*-(4-chlorophenyl)-pyridine-3-carboxamide was prepared using the procedure described for 2-chloro-*N*-(4-ethoxyphenyl)pyridine-3-carboxamide except p-phenetidine was replaced with 4-chloroaniline.

**2-Chloro-*N*-(4-hydroxyphenyl)pyridine-3-carboxamide.** 2-Chloro-*N*-(4-hydroxyphenyl)pyridine-3-carboxamide was prepared using the procedure described for 2-chloro-*N*-(4-ethoxyphenyl)pyridine-3-carboxamide except p-phenetidine was replaced with 4-hydroxyaniline.

**4-Chloro-*N*-(4-ethoxyphenyl)pyridine-3-carboxamide.** 4-Chloro-*N*-(4-ethoxyphenyl)-pyridine-3-carboxamide was prepared using the procedure described for 2-chloro-*N*-(4-ethoxyphenyl)pyridine-3-carboxamide except 2-chloronicotinoyl chloride was replaced with 4-chloronicotinoyl chloride.

***N*-(4-Ethoxyphenyl)-3-fluoropyridine-4-carboxamide.** *N*-(4-Ethoxyphenyl)-3-fluoro-pyridine-4-carboxamide was prepared using the procedure described for 2-chloro-*N*-(4-ethoxyphenyl)pyridine-3-carboxamide except 2-chloronicotinoyl chloride was replaced with 3-fluoropyridine-4-carbonyl chloride.

**3,6-Dichloro-*N*-(4-ethoxyphenyl)pyridazine-4-carboxamide.** 3,6-Dichloropyridazine-N-(4-ethoxyphenyl)-4-carboxamide was prepared using the procedure described for 2-chloro-*N*-(4-ethoxyphenyl)pyridine-3-carboxamide except 2-chloronicotinoyl chloride was replaced with 3,6-dichloropyridazine-4-carbonyl chloride.

**2-Chloro-*N*-(4-chlorophenyl)pyridine-3-carboxamide.** 2-Chloro-*N*-(4-chlorophenyl)-pyridine-3-carboxamide was prepared using the procedure described for 2-chloro-*N*-(4-ethoxyphenyl)pyridine-3-carboxamide except p-phenetidine was replaced with 4-chloroaniline.

**2-Chloro-*N*-(4-trifluoromethylphenyl)pyridine-3-carboxamide.** 2-Chloro-*N*-(4-tri-chloromethylphenyl)pyridine-3-carboxamide was prepared using the procedure described for 2-chloro-*N*-(4-ethoxyphenyl)pyridine-3-carboxamide except p-phenetidine was replaced with 4-trifluoromethylaniline.

**2-Chloro-*N*-(4-fluorophenyl)pyridine-3-carboxamide.** 2-Chloro-N-(4-fluorophenyl)-pyridine-3-carboxamide was prepared using the procedure described for 2-chloro-*N*-(4-ethoxyphenyl)pyridine-3-carboxamide except p-phenetidine was replaced with 4-fluoroaniline.

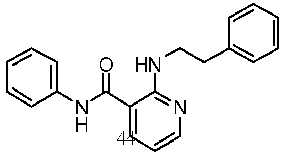

***N*-Phenyl-2-[(2-phenylethyl)amino]pyridine-3-carboxamide.** *N*-Phenyl-2-[(2-phenyl-ethyl)amino]pyridine-3-carboxamide was prepared using the procedure described for *N*-(4-ethoxyphenyl)-2-[(2-phenylethyl)amino]pyridine-3-carboxamide except 2-chloro-*N*-(4-ethoxyphenyl)pyridine-3-carboxamide was replaced with 2-chloro-*N*-phenylpyridine-3-carboxamide. MS: 318 (MH⁺).

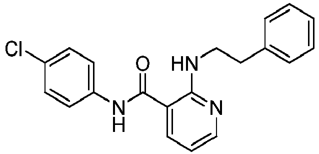

***N*-(4-Chlorophenyl)-2-[(2-phenylethyl)amino]pyridine-3-carboxamide.** *N*-(4-Chloro-phenyl)-2-[(2-phenylethyl)amino]pyridine-3-carboxamide was prepared using the procedure described for *N*-(4-ethoxyphenyl)-2-[(2-phenylethyl)amino]pyridine-3-carboxamide except 2-chloro-*N*-(4-ethoxyphenyl)pyridine-3-carboxamide was replaced with 2-chloro-*N*-(4-chlorophenyl)pyridine-3-carboxamide. MS: 352 (MH⁺).

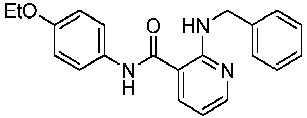

**2-(Benzylamino)-*N*-(4-ethoxyphenyl)pyridine-3-carboxamide.** 2-(Benzylamino)-*N*-(4-ethoxyphenyl)pyridine-3-carboxamide was prepared using the procedure described for *N*-(4-ethoxyphenyl)-2-[(2-phenylethyl)amino]pyridine-3-carboxamide except phenethyl-amine was replaced with benzylamine. MS: 348 (MH⁺).

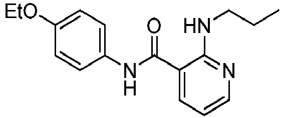

***N*-(4-Ethoxyphenyl)-2-(propylamino)pyridine-3-carboxamide.** *N*-(4-Ethoxyphenyl)-2-(propylamino)pyridine-3-carboxamide was prepared using the procedure described for *N*-(4-ethoxyphenyl)-2-[(2-phenylethyl)amino]pyridine-3-carboxamide except phenethyl-amine was replaced with propylamine. MS: 300 (MH⁺).

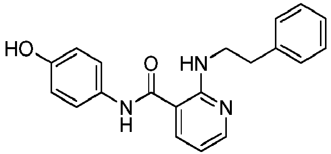

***N*-(4-Hydroxyphenyl)-2-[(2-phenylethyl)amino]pyridine-3-carboxamide.** *N*-(4-Hydroxyphenyl)-2-[(2-phenylethyl)amino]pyridine-3-carboxamide was prepared using the procedure described for *N*-(4-ethoxyphenyl)-2-[(2-phenylethyl)amino]pyridine-3-carboxamide except 2-chloro-*N*-(4-ethoxyphenyl)pyridine-3-carboxamide was replaced with 2-chloro-*N*-(4-hydroxyphenyl)pyridine-3-carboxamide. MS: 334 (MH⁺).

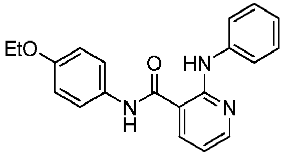

***N*-(4-Ethoxyphenyl)-2-(phenylamino)pyridine-3-carboxamide.** *N*-(4-Ethoxyphenyl)-2-(phenylamino)pyridine-3-carboxamide was prepared using the procedure described for *N*-(4-ethoxyphenyl)-2-[(2-phenylethyl)amino]pyridine-3-carboxamide except phenethyl-amine was replaced with aniline. MS: 334 (MH⁺).

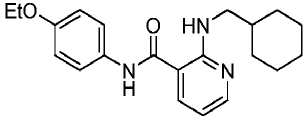

**2-[(Cyclohexylmethyl)amino]-*N*-(4-ethoxyphenyl)pyridine-3-carboxamide.** 2-[(Cyclohexylmethyl)amino]-*N*-(4-ethoxyphenyl)pyridine-3-carboxamide was prepared using the procedure described for *N*-(4-ethoxyphenyl)-2-[(2-phenylethyl)amino]pyridine-3-carboxamide except phenethylamine was replaced with cyclohexylmethylamine. MS: 354 (MH⁺).

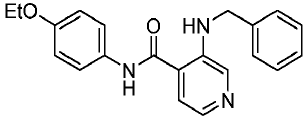

**3-(Benzylamino)-*N*-(4-ethoxyphenyl)pyridine-4-carboxamide.** 3-(Benzylamino)-*N*-(4-ethoxyphenyl)pyridine-4-carboxamide was prepared using the procedure described for *N*-(4-ethoxyphenyl)-2-[(2-phenylethyl)amino]pyridine-3-carboxamide except 2-chloro-*N*-(4-ethoxyphenyl)pyridine-3-carboxamide was replaced with *N*-(4-ethoxyphenyl)-3-fluoropyridine-4-carboxamide. MS: 348 (MH⁺).

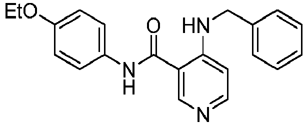

**4-(Benzylamino)-*N*-(4-ethoxyphenyl)pyridine-3-carboxamide.** 4-(Benzylamino)-*N*-(4-ethoxyphenyl)pyridine-3-carboxamide was prepared using the procedure described for *N*-(4-ethoxyphenyl)-2-[(2-phenylethyl)amino]pyridine-3-carboxamide except 2-chloro-*N*-(4-ethoxyphenyl)pyridine-3-carboxamide was replaced with 4-chloro-*N*-(4-ethoxy-phenyl)pyridine-3-carboxamide. MS: 348 (MH⁺).

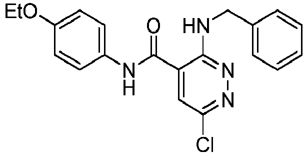

3**-(Benzylamino)-6-chloro-*N*-(4-ethoxyphenyl)pyridazine-4-carboxamide.** 3-(Benzyl-amino)-6-chloro-*N*-(4-ethoxyphenyl)pyridazine-4-carboxamide was prepared using the procedure described for *N*-(4-ethoxyphenyl)-2-[(2-phenylethyl)amino]pyridine-3-carboxamide except 2-chloro-*N*-(4-ethoxyphenyl)pyridine-3-carboxamide was replaced with *N*-(4-ethoxyphenyl)-3,6-dichloropyridazine-4-carboxamide. MS: 383 (MH⁺).

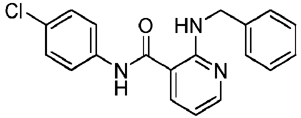

**2-(Benzylamino)**-*N*-(4-chlorophenyl)pyridine-3-carboxamide. 2-(Benzylamino)-*N-*(4-chlorophenyl)pyridine-3-carboxamide was prepared using the procedure described for *N*-(4-ethoxyphenyl)-2-[(2-phenylethyl)amino]pyridine-3-carboxamide except phenethyl-amine was replaced with benzylamine. MS: 338 (MH⁺).

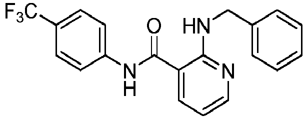

**2-(Benzylamino)-*N*-[4-(trifluoromethyl)phenyl]pyridine-3-carboxamide.** 2-(Benzyl-amino)-*N*-[4-(trifluoromethyl)phenyl]pyridine-3-carboxamide was prepared using the procedure described for *N*-(4-ethoxyphenyl)-2-[(2-phenylethyl)amino]pyridine-3-carboxamide except phenethylamine was replaced with benzylamine. MS: 372 (MH⁺).

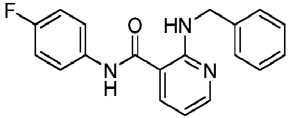

**2-(Benzylamino)-*N*-(4-fluorophenyl)pyridine-3-carboxamide.** 2-(**B**enzylamino)-*N-*(4-fluorophenyl)pyridine-3-carboxamide was prepared using the procedure described for *N*-(4-ethoxyphenyl)-2-[(2-phenylethyl)amino]pyridine-3-carboxamide except phenethyl-amine was replaced with benzylamine. MS: 322 (MH⁺).

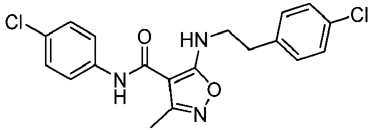

### N-(4-Chlorophenyl)-5-[[2-(4-chlorophenyl)ethyl]amino]-3-methyl-4-isoxazole-carboxamide.

***N*-(3-Methyl-5-isoxazolyl)-4-chlorobenzeneacetamide.** Neat (4-chlorophenyl)acetyl chloride (0.73 mL, 5.0 mmol) was added to a solution of 3-methyl-5-aminoisoxazole (490 mg, 5.00 mmol) in 12 mL of ether and 12 mL of a sat. aq. NaHCO₃ solution at 0°C. The ice bath was removed and the reaction was stirred at rt for 1h. The mixture was diluted with ether and the organic layer was separated, dried (MgSO₄), filtered and conc. to dryness. The amide was isolated in 93% yield as a white solid.

***N*-[2-(4-Chlorophenyl)ethyl]-3-methyl-5-isoxazoleamine.** A solution of *N*-(3-methyl-5-isoxazolyl)-4-chlorobenzeneacetamide (640 mg, 2.56 mmol) in 10 mL of THF was added to a suspension of LiAlH₄ (195 mg, 5.12 mmol) in 15 mL of THF at 0°C. The reaction was allowed to warm to rt and then stirred for 1h. The reaction was quenched at 0°C with a 10% aq. HCl solution. This mixture was extracted with EtOAc. The EtOAc layers were combined, dried over MgSO₄, filtered and conc. The residue was purified by chromatography to give 315 mg of the desired amine.

***N*-4-(Chlorophenyl)-5-[[2-(4-chlorophenyl)ethyl]amino]-3-methyl-4-isoxazole-carboxamide.** A solution of *N*-[2-(4-chlorophenyl)ethyl]-3-methyl-5-isoxazolamine (66 mg, 0.27 mmol) in 5 mL of toluene was treated with neat 4-chlorophenylisocyanate (47 mg, 0.28 mmol) and heated at reflux for 4h. The reaction was conc to dryness and the crude product was purified by RPHPLC. MS: 390 (MH⁺).

### Oocyte Electrophysiology

The modulation of compounds of the invention was determined in oocytes expressing human α7 nAChRs as described above. Preferred compounds exhibited at least 100% modulation of the nicotine EC₅ at 10 µM. Compounds 1-34 exhibited at least 100% modulation of the nicotine EC₅ at 10 µM. More preferred compounds exhibited at least 500% modulation of the nicotine EC₅ at 10 µM. Even more preferred compounds exhibited at least 1000% modulation of the nicotine EC₅ at 10 µM.

## Claims

1. A compound of Formula I: 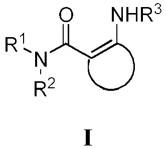 or a pharmaceutically acceptable salt thereof, wherein:
R¹ and R² taken together with the nitrogen atom to which they are attached form a bicyclic heteroaryl or partially unsaturated bicyclic heteroaryl group wherein said bicyclic heteroaryl group or partially unsaturated bicyclic heteroaryl group is chosen from the following: 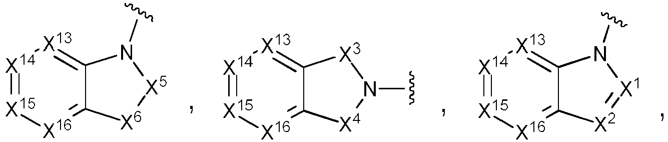 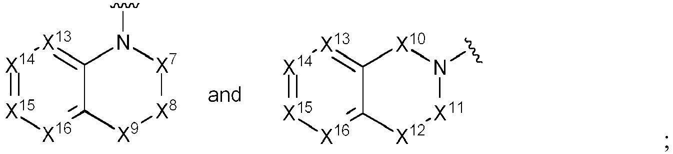
and 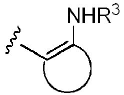 is a heteroaryl group selected from the group consisting of: 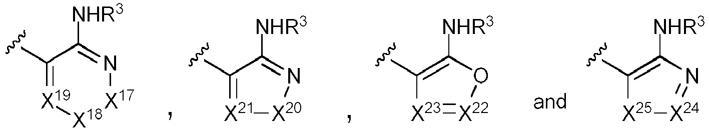
X¹ is N or CR⁴;
X² is N or CR⁵ except that X¹ and X² are not both N;
each of X³, X⁴, X⁵, X⁶, X⁷, X⁸, X⁹, X¹⁰, X¹¹ and X¹² is independently O, C=O, S(=O)ₘ, NR⁶ or CR⁷R⁸;
X¹³ is N or CR⁹;
X¹⁴ is N or CR¹⁰;
X¹⁵ is N or CR¹¹;
X¹⁶ is N or CR¹²;
X¹⁷ is N or CR¹³;
X¹⁸ is N or CR¹⁴;
X¹⁹ is N or CR¹⁵;
X²⁰ is NR⁶, S(O)ₘ or O;
X²¹ is N or CR¹⁶;
X²² is N or CR¹⁷;
X²³ is N or CR¹⁸;
X²⁴ is N or CR¹⁹;
X²⁵ is NR⁶, S(O)ₘ or O;
m is 0, 1 or 2;
R³ is selected from the group consisting of C₂₋₈ alkyl, C₂₋₈ alkenyl, C₂₋₈ alkynyl, and C₁₋₈ haloalkyl, each optionally substituted; or
R³ is selected from the group consisting of an arylalkyl and heteroarylalkyl group, each optionally substituted; or
R³ is selected from the group consisting of C₃₋₈ cycloalkyl, cycloalkenyl, carbon-attached heterocycloalkyl and carbon-attached heterocycloalkenyl, each optionally substituted; and
R⁶ is selected from the group consisting of hydrogen, C₁₋₈ alkyl, C₃₋₈ alkenyl, C₃₋₈ alkynyl, C₁₋₈ haloalkyl, aryl, heteroaryl and C₃₋₈ cycloalkyl; and
R⁴, R⁵, R⁷, R⁸, R⁹, R¹⁰, R¹¹, R¹², R¹⁴, R¹⁵, R¹⁶, R¹⁷, R¹⁸ and R¹⁹ are each independently selected from the group consisting of hydrogen, halogen, nitro, cyano, hydroxyl, amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkamino, C₁₋₈ haloalkamino, dialkylamino, alkenylamino, alkynylamino, arylamino, heteroarylamino, C₁₋₈ cycloalkamino, cycloalkenylamino, heterocycloalkylamino, heterocycloalkenylamino, C₁₋₈ alkthio, C₁₋₈ haloalkthio, alkenylthio, alkynylthio, arylthio, heteroarylthio, C₃₋₈ cycloalkthio, cycloalkenylthio, heterocycloalkylthio, heterocycloalkenylthio, -C(=O)R²⁰, -N(R²¹)C(=O)R²², -OC(=O)R²², -N(R²¹)S(=O)₂R²², - S(=O)₂R²⁰, and -S(=O)R²⁰, each optionally substituted; and
R¹³ is selected from the group consisting of hydrogen, halogen, nitro, cyano, hydroxyl, C₁₋₈ alkyl, C₂₋₈ alkenyl, C₂₋₈ alkynyl, C₁₋₈ haloalkyl, aryl, heteroaryl, C₁₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ haloalkamino, alkenylamino, alkynylamino, arylamino, heteroarylamino, C₃₋₈ cycloalkamino, cycloalkenylamino, heterocycloalkylamino, heterocycloalkenylamino, C₁₋₈ alkthio, C₁₋₈ haloalkthio, alkenylthio, alkynylthio, arylthio, heteroarylthio, C₃₋₈ cycloalkthio, cycloalkenylthio, heterocycloalkylthio, heterocycloalkenylthio, -C(=O)R²⁰, -N(R²¹)C(=O)R²², -OC(=O)R²², -N(R²¹)S(=O)₂R²², -S(=O)₂R²⁰, and -S(=O)R²⁰, each optionally substituted; and
R⁴ and R⁵, or R⁶ and R¹⁶, or R⁶ and R¹⁹, or R⁹ and R¹⁰, or R¹⁰ and R¹¹, or R¹¹ and R¹², or R¹³ and R¹⁴, or R¹⁴ and R¹⁵, or R¹⁷ and R¹⁸ taken together with the atoms to which they are attached form an unsubstituted or substituted fused 5 or 6-membered unsaturated or partially unsaturated ring optionally interrupted by one -O-, -NR⁶-, -S-, -SO- or -SO₂-; and
each R²⁰ is independently selected from the group consisting of hydroxyl, amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkylamino, C₁₋₈ haloalkylamino, dialkylamino, alkenylamino, alkynylamino, arylamino, heteroarylamino, C₃₋₈ cycloalkylamino, cycloalkenylamino, heterocycloalkylamino, and heterocycloalkenylamino, each optionally substituted; and
each R²¹ is independently selected from the group consisting of hydrogen, hydroxyl, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, and heterocycloalkenyloxy, each optionally substituted; and
each R²² is independently selected from the group consisting of amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkylamino, C₁₋₈ haloalkylamino, dialkylamino, alkenylamino, alkynylamino, arylamino, heteroarylamino, C₃₋₈ cycloalkylamino, cycloalkenylamino, heterocycloalkylamino, and heterocycloalkenylamino, each optionally substituted.

2. A compound according to claim 1, which is a compound according to Formula **II:** 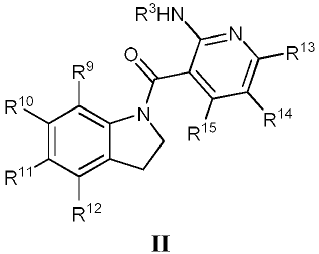 or a pharmaceutically acceptable salt thereof, wherein:
R³ is selected from the group consisting of C₂₋₈ alkyl, C₂₋₈ alkenyl, C₂₋₈ alkynyl, C₁₋₈ haloalkyl, each optionally substituted; or
R³ is selected from the group consisting of an arylalkyl and heteroarylalkyl group, each optionally substituted; or
R³ is selected from the group consisting of C₃₋₈ cycloalkyl, cycloalkenyl, carbon-attached heterocycloalkyl and carbon-attached heterocycloalkenyl, each optionally substituted; and
R⁶ is selected from the group consisting of hydrogen, C₁₋₈ alkyl, C₃₋₈ alkenyl, C₃₋₈ alkynyl, C₁₋₈ haloalkyl, aryl, heteroaryl and C₃₋₈ cycloalkyl; and
R⁹, R¹⁰, R¹¹, R¹², R¹⁴ and R¹⁵ are each independently selected from the group consisting of hydrogen, halogen, nitro, cyano, hydroxyl, amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkamino, C₁₋₈ haloalkamino, dialkylamino, alkenylamino, alkynylamino, arylamino, heteroarylamino, C₁₋₈ cycloalkamino, cycloalkenylamino, heterocycloalkylamino, heterocycloalkenylamino, C₁₋₈ alkthio, C₁₋₈ haloalkthio, alkenylthio, alkynylthio, arylthio, heteroarylthio, C₃₋₈ cycloalkthio, cycloalkenylthio, heterocycloalkylthio, heterocycloalkenylthio, -C(=O)R²⁰, -N(R²¹)C(=O)R²², -OC(=O)R²²,-N(R²¹)S(=O)₂R²², -S(=O)₂R²⁰, and -S(=O)R²⁰, each optionally substituted; and
R¹³ is selected from the group consisting of hydrogen, halogen, nitro, cyano, hydroxyl, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ haloalkamino, alkenylamino, alkynylamino, arylamino, heteroarylamino, C₃₋₈ cycloalkamino, cycloalkenylamino, heterocycloalkylamino, heterocycloalkenylamino, C₁₋₈ alkthio, C₁₋₈ haloalkthio, alkenylthio, alkynylthio, arylthio, heteroarylthio, C₃₋₈ cycloalkthio, cycloalkenylthio, heterocycloalkylthio, heterocycloalkenylthio, -C(=O)R²⁰, -N(R²¹)C(=O)R²², -OC(=O)R²², -N(R²¹)S(=O)₂R²², -S(=O)₂R²⁰, and -S(=O)R²⁰, each optionally substituted; and
R⁹ and R¹⁰, or R¹⁰ and R¹¹, or R¹¹ and R¹² , or R¹³ and R¹⁴, or R¹⁴ and R¹⁵ taken together with the atoms to which they are attached form an unsubstituted or substituted fused 5 or 6-membered unsaturated or partially unsaturated ring optionally interrupted by one -O-, -NR⁶-, -S-, -SO- or -SO₂-; and
each R²⁰ is independently selected from the group consisting of hydroxyl, amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₆ alkylamino, C₁₋₈ haloalkylamino, dialkylamino, alkenylamino, alkynylamino, arylamino, heteroarylamino, C₃₋₈ cycloalkylamino, cycloalkenylamino, heterocycloalkylamino, and heterocycloalkenylamino, each optionally substituted; and
each R²¹ is independently selected from the group consisting of hydrogen, hydroxyl, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, and heterocycloalkenyloxy, each optionally substituted; and
each R²² is independently selected from the group consisting of amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkylamino, C₁₋₈ haloalkylamino, dialkylamino, alkenylamino, alkynylamino, arylamino, heteroarylamino, C₃₋₈ cycloalkylamino, cycloalkenylamino, heterocycloalkylamino, and heterocycloalkenylamino, each optionally substituted.

3. A compound according to claim 1, which is a compound according to
Formula **III:** 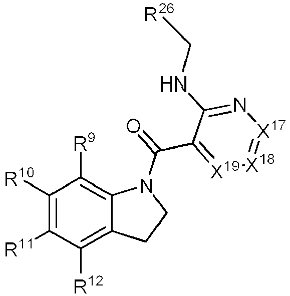 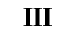 or a pharmaceutically acceptable salt thereof, wherein:
R⁶ is selected from the group consisting of hydrogen, C₁₋₈ alkyl, C₃₋₈ alkenyl, C₃₋₈ alkynyl, C₁₋₈ haloalkyl, aryl, heteroaryl and C₃₋₈ cycloalkyl; and
R⁹, R¹⁰, R¹¹, R¹², R¹⁴ and R¹⁵ are each independently selected from the group consisting of hydrogen, halogen, nitro, cyano, hydroxyl, amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkamino, C₁₋₈ haloalkamino, dialkylamino, alkenylamino, alkynylamino, arylamino, heteroarylamino, C₃₋₈ cycloalkamino, cycloalkenylamino, heterocycloalkylamino, heterocycloalkenylamino, C₁₋₈ alkthio, C₁₋₈ haloalkthio, alkenylthio, alkynylthio, arylthio, heteroarylthio, C₃₋₈ cycloalkthio, cycloalkenylthio, heterocycloalkylthio, heterocycloalkenylthio, -C(=O)R²⁰, -N(R²¹)C(=O)R²², -OC(=O)R²², - N(R²¹)S(=O)₂R²², -S(=O)₂R²⁰, and -S(=O)R²⁰, each optionally substituted; and
R¹³ is selected from the group consisting of hydrogen, halogen, nitro, cyano, hydroxyl, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ haloalkamino, alkenylamino, alkynylamino, arylamino, heteroarylamino, C₃₋₈ cycloalkamino, cycloalkenylamino, heterocycloalkylamino, heterocycloalkenylamino, C₁₋₈ alkthio, C₁₋₈ haloalkthio, alkenylthio, alkynylthio, arylthio, heteroarylthio, C₃₋₈ cycloalkthio, cycloalkenylthio, heterocycloalkylthio, heterocycloalkenylthio, -C(=O)R²⁰, -N(R²¹)C(=O)R²², -OC(=O)R²², -N(R²¹)S(=O)₂R²², -S(=O)₂R²⁰, and -S(=O)R²⁰, each optionally substituted; and
R⁹ and R¹⁰, or R¹⁰ and R¹¹, or R¹¹ and R¹² taken together with the atoms to which they are attached form an unsubstituted or substituted fused 5 or 6-membered unsaturated or partially unsaturated ring optionally interrupted by one -O-, -NR⁶-, -S-, -SO- or -SO₂-; and
each R²⁰ is independently selected from the group consisting of hydroxyl, amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkylamino, C₁₋₈ haloalkylamino, dialkylamino, alkenylamino, alkynylamino, arylamino, heteroarylamino, C₃₋₈ cycloalkylamino, cycloalkenylamino, heterocycloalkylamino, and heterocycloalkenylamino, each optionally substituted; and
each R²¹ is independently selected from the group consisting of hydrogen, hydroxyl, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, and heterocycloalkenyloxy, each optionally substituted; and
each R²² is independently selected from the group consisting of amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkylamino, C₁₋₈ haloalkylamino, dialkylamino, alkenylamino, alkynylamino, arylamino, heteroarylamino, C₃₋₈ cycloalkylamino, cycloalkenylamino, heterocycloalkylamino, and heterocycloalkenylamino, each optionally substituted; and
R²⁶ is an aryl or heteroaryl group selected from: 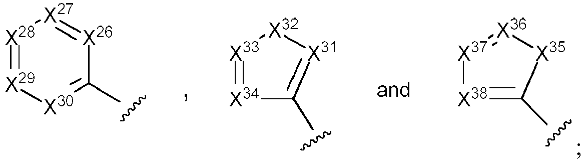
X¹⁷ is N or CR¹³;
X¹⁸ is N or CR¹⁴;
X¹⁹ is N or CR¹⁵;
X²⁶ is N or C-R²⁷;
X²⁷ is N or C-R²⁸;
X²⁸ is N or C-R²⁹;
X²⁹ is N or C-R³⁰;
X³⁰ is N or C-R³¹;
X³¹ is N or C-R³²;
X³² is NR⁶, O or S(O)ₘ;
X³³ is N or CR³³;
X³⁴ is N or C-R³⁴;
X³⁵ is NR⁶ or O;
X³⁶ is N or C-R³⁵;
X³⁷ is N or C-R³⁶;
X³⁸ is N or C-R³⁷;
R²⁷, R²⁸, R³⁰, R³¹, R³², R³³, R³⁴, R³⁵, R³⁶ and R³⁷ are independently selected from the group consisting of hydrogen, halogen, nitro, cyano, hydroxyl, amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkamino, C₁₋₈ haloalkamino, dialkylamino, alkenylamino, alkynylamino, arylamino, heteroarylamino, C₃₋₈ cycloalkamino, cycloalkenylamino, heterocycloalkylamino, heterocycloalkenylamino, C₁₋₈ alkthio, C₁₋₈ haloalkthio, alkenylthio, alkynylthio, arylthio, heteroarylthio, C₃₋₈ cycloalkthio, cycloalkenylthio, heterocycloalkylthio, heterocycloalkenylthio, -C(=O)R²⁰, -N(R²¹)C(=O)R²², -OC(=O)R²², -N(R²¹)S(=O)₂R²², -S(=O)₂R²⁰, and -S(=O)R²⁰, each optionally substituted; and
R²⁹ is selected from the group consisting of hydrogen, halogen, nitro, cyano, hydroxyl, amino, C₁₋₈ alkyl, C₂₋₈ alkenyl, C₂₋₈ alkynyl, C₁₋₈ haloalkyl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkamino, C₁₋₈ haloalkamino, dialkylamino, alkenylamino, alkynylamino, arylamino, heteroarylamino, C₃₋₈ cycloalkamino, cycloalkenylamino, heterocycloalkylamino, heterocycloalkenylamino, C₁₋₈ alkthio, C₁₋₈ haloalkthio, alkenylthio, alkynylthio, arylthio, heteroarylthio, C₃₋₈ cycloalkthio, cycloalkenylthio, heterocycloalkylthio, heterocycloalkenylthio, -C(=O)R²⁰, -N(R²¹)C(=O)R²², -OC(=O)R²², -N(R²¹)S(=O)₂R²²,-S(=O)₂R²⁰, and -S(=O)R²⁰, each optionally substituted; and
R²⁷ and R²⁸, or R²⁸ and R²⁹ or R²⁹ and R³⁰, or R³⁰ and R³¹, or R³² and R⁶, or R⁶ and R³³, or R³³ and R³⁴, or R⁶ and R³⁵, or R³⁵ and R³⁶, or R³⁶ and R³⁷ taken together with the atoms to which they are attached form an unsubstituted or substituted fused 5 or 6-membered unsaturated or partially unsaturated ring optionally interrupted by one -O-, -NR⁶-, -S-, -SO- or -SO₂-.

4. A compound according to claim 1, which is a compound according to
Formula IV: 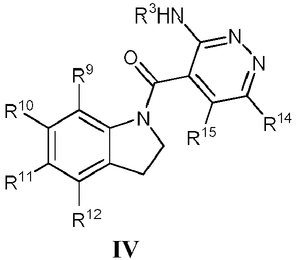 or a pharmaceutically acceptable salt thereof, wherein:
R³ is selected from the group consisting of C₂₋₈ alkyl, C₂₋₈ alkenyl, C₂₋₈ alkynyl, and C₁₋₈ haloalkyl, each optionally substituted; or
R³ is selected from the group consisting of an arylalkyl and heteroarylalkyl group, each optionally substituted; or
R³ is selected from the group consisting of C₃₋₈ cycloalkyl, cycloalkenyl, carbon-attached heterocycloalkyl and carbon-attached heterocycloalkenyl, each optionally substituted; and
R⁶ is selected from the group consisting of hydrogen, C₁₋₈ alkyl, C₃₋₈ alkenyl, C₃₋₈ alkynyl, C₁₋₈ haloalkyl, aryl, heteroaryl and C₃₋₈ cycloalkyl; and
R⁹, R¹⁰, R¹¹, R¹², R¹⁴ and R¹⁵ are each independently selected from the group consisting of hydrogen, halogen, nitro, cyano, hydroxyl, amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkamino, C₁₋₈ haloalkamino, dialkylamino, alkenylamino, alkynylamino, arylamino, heteroarylamino, C₃₋₈ cycloalkamino, cycloalkenylamino, heterocycloalkylamino, heterocycloalkenylamino, C₁₋₈ alkthio, C₁₋₈ haloalkthio, alkenylthio, alkynylthio, arylthio, heteroarylthio, C₃₋₈ cycloalkthio, cycloalkenylthio, heterocycloalkylthio, heterocycloalkenylthio, -C(=O)R²⁰, -N(R²¹)C(=O)R²², -OC(=O)R²²,-N(R²¹)S(=O)₂R²², -S(=O)₂R²⁰, and -S(=O)R²⁰, each optionally substituted; or
R⁹ and R¹⁰, or R¹⁰ and R¹¹, or R¹¹ and R¹², or R¹⁴ and R¹⁵ taken together with the atoms to which they are attached form an unsubstituted or substituted fused 5 or 6-membered unsaturated or partially unsaturated ring optionally interrupted by one -O-, -NR⁶-, -S-, -SO- or -SO₂-; and
each R²⁰ is independently selected from the group consisting of hydroxyl, amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₆ alkylamino, C₁₋₈ haloalkylamino, dialkylamino, alkenylamino, alkynylamino, arylamino, heteroarylamino, C₃₋₈ cycloalkylamino, cycloalkenylamino, heterocycloalkylamino, and heterocycloalkenylamino, each optionally substituted; and
each R²¹ is independently selected from the group consisting of hydrogen, hydroxyl, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, and heterocycloalkenyloxy, each optionally substituted; and
each R²² is independently selected from the group consisting of amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkylamino, C₁₋₈ haloalkylamino, dialkylamino, alkenylamino, alkynylamino, arylamino, heteroarylamino, C₃₋₈ cycloalkylamino, cycloalkenylamino, heterocycloalkylamino, and heterocycloalkenylamino, each optionally substituted.

5. A compound according to claim 1, which is a compound according to
Formula V: 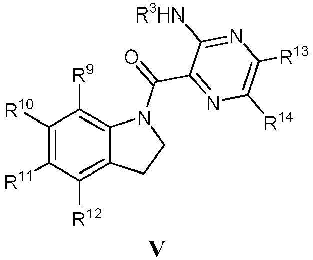 or a pharmaceutically acceptable salt thereof, wherein:
R³ is selected from the group consisting of C₂₋₈ alkyl, C₂₋₈ alkenyl, C₂₋₈ alkynyl, and C₁₋₈ haloalkyl, each optionally substituted; or
R³ is selected from the group consisting of arylalkyl and heteroarylalkyl group, each optionally substituted; or
R³ is selected from the group consisting of C₃₋₈ cycloalkyl, cycloalkenyl, carbon-attached heterocycloalkyl and carbon-attached heterocycloalkenyl, each optionally substituted; and
R⁶ is selected from the group consisting of hydrogen, C₁₋₈ alkyl, C₃₋₈ alkenyl, C₃₋₈ alkynyl, C₁₋₈ haloalkyl, aryl, heteroaryl and C₃₋₈ cycloalkyl; and
R⁹, R¹⁰, R¹¹, R¹² and R¹⁴ are each independently selected from the group consisting of hydrogen, halogen, nitro, cyano, hydroxyl, amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkamino, C₁₋₈ haloalkamino, dialkylamino, alkenylamino, alkynylamino, arylamino, heteroarylamino, C₃₋₈ cycloalkamino, cycloalkenylamino, heterocycloalkylamino, heterocycloalkenylamino, C₁₋₈ alkthio, C₁₋₈ haloalkthio, alkenylthio, alkynylthio, arylthio, heteroarylthio, C₃₋₈ cycloalkthio, cycloalkenylthio, heterocycloalkylthio, heterocycloalkenylthio, -C(=O)R²⁰, -N(R²¹)C(=O)R²², -OC(=O)R²²,-N(R²¹)S(=O)₂R²², -S(=O)₂R²⁰, and -S(=O)R²⁰, each optionally substituted; and
R¹³ is selected from the group consisting of hydrogen, halogen, nitro, cyano, hydroxyl, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ haloalkamino, alkenylamino, alkynylamino, arylamino, heteroarylamino, C₃₋₈ cycloalkamino, cycloalkenylamino, heterocycloalkylamino, heterocycloalkenylamino, C₁₋₈ alkthio, C₁₋₈ haloalkthio, alkenylthio, alkynylthio, arylthio, heteroarylthio, C₃₋₈ cycloalkthio, cycloalkenylthio, heterocycloalkylthio, heterocycloalkenylthio, -C(=O)R²⁰, -N(R²¹)C(=O)R²², -OC(=O)R²², -N(R²¹)S(=O)₂R²², -S(=O)₂R²⁰, and -S(=O)R²⁰, each optionally substituted; and
R⁹ and R¹⁰, or R¹⁰ and R¹¹, or R¹¹ and R¹², or R¹³ and R¹⁴ taken together with the atoms to which they are attached form an unsubstituted or substituted fused 5 or 6-membered unsaturated or partially unsaturated ring optionally interrupted by one -O-, -NR⁶-, -S-, -SO- or -SO₂-; and
each R²⁰ is independently selected from the group consisting of hydroxyl, amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₆ alkylamino, C₁₋₈ haloalkylamino, dialkylamino, alkenylamino, alkynylamino, arylamino, heteroarylamino, C₃₋₈ cycloalkylamino, cycloalkenylamino, heterocycloalkylamino, and heterocycloalkenylamino, each optionally substituted; and
each R²¹ is independently selected from the group consisting of hydrogen, hydroxyl, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, and heterocycloalkenyloxy, each optionally substituted; and
each R²² is independently selected from the group consisting of amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkylamino, C₁₋₈ haloalkylamino, dialkylamino, alkenylamino, alkynylamino, arylamino, heteroarylamino, C₃₋₈ cycloalkylamino, cycloalkenylamino, heterocycloalkylamino, and heterocycloalkenylamino, each optionally substituted.

6. A compound according to claim 1, which is a compound according to
Formula **VI:** 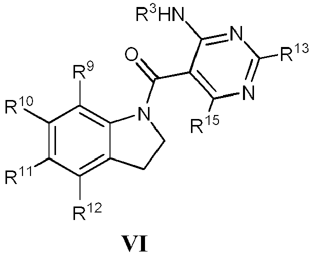 or a pharmaceutically acceptable salt thereof, wherein:
R³ is selected from the group consisting of C₂₋₈ alkyl, C₂₋₈ alkenyl, C₂₋₈ alkynyl, and C₁₋₈ haloalkyl, each optionally substituted; or
R³ is selected from the group consisting of an arylalkyl and heteroarylalkyl group, each optionally substituted; or
R³ is selected from the group consisting of C₃₋₈ cycloalkyl, cycloalkenyl, carbon-attached heterocycloalkyl and carbon-attached heterocycloalkenyl, each optionally substituted; and
R⁶ is selected from the group consisting of hydrogen, C₁₋₈ alkyl, C₃₋₈ alkenyl, C₃₋₈ alkynyl, C₁₋₈ haloalkyl, aryl, heteroaryl and C₃₋₈ cycloalkyl; and
R⁹, R¹⁰, R¹¹, R¹² and R¹⁵ are each independently selected from the group consisting of hydrogen, halogen, nitro, cyano, hydroxyl, amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkamino, C₁₋₈ haloalkamino, dialkylamino, alkenylamino, alkynylamino, arylamino, heteroarylamino, C₃₋₈ cycloalkamino, cycloalkenylamino, heterocycloalkylamino, heterocycloalkenylamino, C₁₋₈ alkthio, C₁₋₈ haloalkthio, alkenylthio, alkynylthio, arylthio, heteroarylthio, C₃₋₈ cycloalkthio, cycloalkenylthio, heterocycloalkylthio, heterocycloalkenylthio, -C(=O)R²⁰, -N(R²¹)C(=O)R²², -OC(=O)R²²,-N(R²¹)S(=O)₂R²², -S(=O)₂R²⁰, and -S(=O)R²⁰, each optionally substituted; and
R¹³ is selected from the group consisting of hydrogen, halogen, nitro, cyano, hydroxyl, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ haloalkamino, alkenylamino, alkynylamino, arylamino, heteroarylamino, C₃₋₈ cycloalkamino, cycloalkenylamino, heterocycloalkylamino, heterocycloalkenylamino, C₁₋₈ alkthio, C₁₋₈ haloalkthio, alkenylthio, alkynylthio, arylthio, heteroarylthio, C₃₋₈ cycloalkthio, cycloalkenylthio, heterocycloalkylthio, heterocycloalkenylthio, -C(=O)R²⁰, -N(R²¹)C(=O)R²², -OC(=O)R²², -N(R²¹)S(=O)₂R²², -S(=O)₂R²⁰, and -S(=O)R²⁰, each optionally substituted; and
R⁹ and R¹⁰, or R¹⁰ and R¹¹, or R¹¹ and R¹² taken together with the atoms to which they are attached form an unsubstituted or substituted fused 5 or 6-membered unsaturated or partially unsaturated ring optionally interrupted by one -O-, -NR⁶-, -S-, -SO- or -SO₂-; and
each R²⁰ is independently selected from the group consisting of hydroxyl, amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₆ alkylamino, C₁₋₈ haloalkylamino, dialkylamino, alkenylamino, alkynylamino, arylamino, heteroarylamino, C₃₋₈ cycloalkylamino, cycloalkenylamino, heterocycloalkylamino, and heterocycloalkenylamino, each optionally substituted; and
each R²¹ is independently selected from the group consisting of hydrogen, hydroxyl, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, and heterocycloalkenyloxy, each optionally substituted; and
each R²² is independently selected from the group consisting of amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkylamino, C₁₋₈ haloalkylamino, dialkylamino, alkenylamino, alkynylamino, arylamino, heteroarylamino, C₃₋₈ cycloalkylamino, cycloalkenylamino, heterocycloalkylamino, and heterocycloalkenylamino, each optionally substituted.

7. The compound of claim 3 wherein
R⁹, R¹⁰, R¹¹ and R¹² are each independently selected from the group consisting of hydrogen, halogen, C₁₋₈ alkyl, C₁₋₈ haloalkyl, C₃₋₈ cycloalkyl, C₁₋₈ alkoxy, C₁₋₈ haloalkoxy, and C₃₋₈ cycloalkoxy, each optionally substituted; and
R¹³, R¹⁴ and R¹⁵ are each independently selected from the group consisting of hydrogen, halogen, C₁₋₈ alkyl, C₁₋₈ haloalkyl, C₃₋₈ cycloalkyl, and C₁₋₈ haloalkoxy, each optionally substituted; and
R²⁶ is an aryl group selected from: 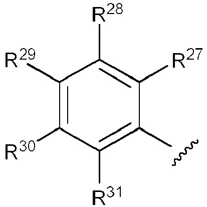
X¹⁷ is N or CR¹³;
X¹⁸ is N or CR¹⁴;
X¹⁹ is N or CR¹⁵;
R²⁷, R²⁸, R²⁹, R³⁰ and R³¹ are independently selected from the group consisting of hydrogen, halogen, nitro, cyano, C₁₋₈ alkyl, C₁₋₈ haloalkyl, C₃₋₈ cycloalkyl, C₁₋₈ alkoxy, C₁₋₈ haloalkoxy, alkenyloxy, alkynyloxy, and C₃₋₈ cycloalkoxy, and pharmaceutically acceptable salts thereof.

8. The compound of claim 7 wherein: R⁹ and R¹² are hydrogen; and pharmaceutically acceptable salts thereof.

9. The compound of claim 8 wherein:
X¹⁷ is CR¹³;
X¹⁸ is CR¹⁴;
X¹⁹ is CR¹⁵;
and pharmaceutically acceptable salts thereof; or wherein:
X¹⁷ is CR¹³;
X¹⁸ is CR¹⁴;
X¹⁹ is N;
and pharmaceutically acceptable salts thereof; or wherein:
X¹⁷ is N;
X¹⁸ is CR¹⁴;
X¹⁹ is CR¹⁵;
and pharmaceutically acceptable salts thereof; or wherein:
X¹⁷ is CR¹³;
X¹⁸ is N;
X¹⁹ is CR¹⁵;
and pharmaceutically acceptable salts thereof.

10. A compound according to claim 1, which is
selected from the group consisting of:
[2-(benzylamino)pyridin-3-yl](5-chloro-2,3-dihydro-1H-indol-1-yl)methanone;
(5-chloro-2,3-dihydro-1*H-*indol-1-yl)[2-(phenylamino)pyridin-3-yl]methanone;
(5-chloro-2,3-dihydro-1*H-*indol-1-yl)[2-[(pyridin-2-ylmethyl)amino]pyridin-3-yl]methanone;
(5-chloro-2,3-dihydro-1*H-*indol-1-yl)[2-[(2-phenylethyl)amino]pyridin-3-yl]methanone;
(5-chloro-2,3-dihydro-1*H-*indol-1-yl)[2-[(pyridin-3-ylmethyl)amino]pyridin-3-yl]methanone;
(5-chloro-2,3-dihydro-1*H-*indol-1-yl)[2-[[2-(pyridin-2-yl)ethyl]amino]-pyridin-3-yl]methanone;
(5-chloro-2,3-dihydro-1*H-*indol-1-yl)[2-[(4-fluorobenzyl)amino]pyridin-3-yl]methanone;
(5-chloro-2,3-dihydro-1*H-*indol-1-yl)[2-[(1-(4-fluorophenyl)ethyl)amino]-pyridin-3-yl]methanone;
[2-(benzylamino)pyridin-3-yl](2,3-dihydro-1*H-*indol-1-yl)methanone;
[3-(benzylamino)pyridazin-4-yl](5-chloro-2,3-dihydro-1H-indol-1-yl)methanone;
(5-chloro-2,3-dihydro-1H-indol-1-yl)[2-[(4-fluorobenzyl)amino]pyrazin-3-yl]methanone;
(5-chloro-2,3-dihydro-1H-indol-1-yl)[2-(2,5-difluorobenzylamino)pyridin-3-yl-]methanone;
(5-chloro-2,3-dihydro-1H-indol-1-yl)[2-(3,4-difluorobenzylamino)pyridin-3-yl]-methanone;
(5-chloro-2,3-dihydro-1H-indol-1-yl)[2-(2,4-difluorobenzylamino)pyridin-3-yl]-methanone;
(5-chloro-2,3-dihydro-1H-indol-1-yl)[2-(cyclopropylmethylamino)pyridin-3-yl]-methanone;
(5-chloro-2,3-dihydro-1H-indol-1-yl)[2-(2-propyn-1-ylamino)pyridin-3-yl]-methanone;
(5-fluoro-2,3-dihydro-1H-indol-1-yl)[2-(4-fluorobenzylamino)pyridin-3-yl]-methanone;
(5-chloro-2,3-dihydro-1H-indol-1-yl)[2-[(pyridin-4-ylamino)pyridin-3-yl]-methanone, and pharmaceutically acceptable salts and solvates thereof.

11. A pharmaceutical composition comprising a compound according to any one of Claims 1-10, or a pharmaceutically acceptable salt thereof, and a pharmaceutically acceptable carrier or diluent.

12. A pharmaceutical composition according to claim 11,
comprising a pharmaceutically acceptable excipient and a compound selected from:
[2-(benzylamino)pyridin-3-yl](5-chloro-2,3-dihydro-1H-indol-1-yl)methanone (compound 1);
(5-chloro-2,3-dihydro-1*H-*indol-1-yl)[2-(phenylamino)pyridin-3-yl]methanone (compound 2);
(5-chloro-2,3-dihydro-1*H-*indol-1-yl)[2-[(pyridin-2-ylmethyl)amino]pyridin-3-yl]-methanone (compound 3);
(5-chloro-2,3-dihydro-1*H-*indol-1-yl)[2-[(2-phenylethyl)amino]pyridin-3-yl]methanone (compound 4);
(5-chloro-2,3-dihydro-1*H-*indol-1-yl)[2-[(pyridin-3-ylmethyl)amino]pyridin-3-yl]-methanone (compound 5);
(5-chloro-2,3-dihydro-1*H-*indol-1-yl)[2-[[2-(pyridin-2-yl)ethyl]amino]-pyridin-3-yl]-methanone (compound 6);
(5-chloro-2,3-dihydro-1*H-*indol-1-yl)[2-[(4-fluorobenzyl)amino]pyridin-3-yl]methanone (compound 7);
(5-chloro-2,3-dihydro-1*H-*indol-1-yl)[2-[(1-(4-fluorophenyl)ethyl)-amino]pyridin-3-yl]-methanone (compound 8);
[2-(benzylamino)pyridin-3-yl](2,3-dihydro-1*H-*indol-1-yl)methanone (compound 9);
[3-(benzylamino)pyridazin-4-yl](5-chloro-2,3-dihydro-1H-indol-1-yl)methanone (compound 10);
(5-chloro-2,3-dihydro-1H-indol-1-yl)[2-[(4-fluorobenzyl)amino]pyrazin-3-yl]methanone (compound 11);
*N*-(4-ethoxyphenyl)-2-[(2-phenylethyl)amino]pyridine-3-carboxamide (compound 12);
*N*-phenyl-2-[(2-phenylethyl)amino]pyridine-3-carboxamide (compound 13);
*N*-(4-chlorophenyl)-2-[(2-phenylethyl)amino]pyridine-3-carboxamide (compound 14);
2-(benzylamino)-*N*-(4-ethoxyphenyl)pyridine-3-carboxamide (compound 15);
*N*-(4-ethoxyphenyl)-2-(propylamino)pyridine-3-carboxamide (compound 16);
*N*-(4-hydroxyphenyl)-2-[(2-phenylethyl)amino]pyridine-3-carboxamide (compound 17);
*N*-(4-ethoxyphenyl)-2-(phenylamino)pyridine-3-carboxamide (compound 18);
2-[(cyclohexylmethyl)amino]-*N*-(4-ethoxyphenyl)pyridine-3-carboxamide (compound 19);
3-(benzylamino)-*N*-(4-ethoxyphenyl)pyridine-4-carboxamide (compound 20);
4-(benzylamino)-*N*-(4-ethoxyphenyl)pyridine-3-carboxamide (compound 21);
3-(benzylamino)-6-chloro-*N*-(4-ethoxyphenyl)pyridazine-4-carboxamide (compound 22);
2-(benzylamino)-*N*-[4-(trifluoromethyl)phenyl]pyridine-3-carboxamide (compound 24);
2-(benzylamino)-*N*-(4-fluorophenyl)pyridine-3-carboxamide (compound 25);
*N*-(4-chlorophenyl)-5-[2-[(4-chlorophenyl)ethyl]amino]-3-methyl-4-isoxazolecarbox-amide (compound 26);
(5-chloro-2,3-dihydro-1H-indol-1-yl)[2-(2,5-difluorobenzylamino)pyridin-3-yl]-methanone (compound 27);
(5-chloro-2,3-dihydro-1H-indol-1-yl)[2-(3,4-difluorobenzylamino)pyridin-3-yl]-methanone (compound 28);
(5-chloro-2,3-dihydro-1H-indol-1-yl)[2-(2,4-difluorobenzylamino)pyridin-3-yl]-methanone (compound 29);
(5-chloro-2,3-dihydro-1H-indol-1-yl)[2-(cyclopropylmethylamino)pyridin-3-yl]-methanone (compound 30);
(5-chloro-2,3-dihydro-1H-indol-1-yl)[2-(2-propyn-1-ylamino)pyridin-3-yl]methanone (compound 31);
(5-fluoro-2,3-dihydro-1H-indol-1-yl)[2-(4-fluorobenzylamino)pyridin-3-yl]methanone (compound 32); and
(5-chloro-2,3-dihydro-1H-indol-1-yl)[2-[(pyridin-4-ylamino)pyridin-3-yl]methanone (compound 33), and pharmaceutically acceptable salts thereof.

13. A pharmaceutical composition according to claim 11,
comprising a pharmaceutically acceptable diluent or carrier and a compound of Formula **VII:** 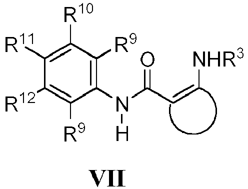 or a pharmaceutically acceptable salt thereof, wherein: and 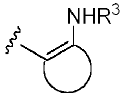 is a heteroaryl group selected from the group consisting of: 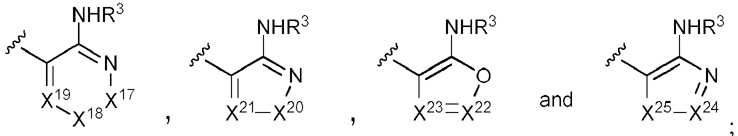
X¹⁷ is N or CR¹³;
X¹⁸ is N or CR¹⁴;
X¹⁹ is N or CR¹⁵;
X²⁰ is NR⁶, S(O)ₘ or O;
X²¹ is N or CR¹⁶;
X²² is N or CR¹⁷;
X²³ is N or CR¹⁸;
X²⁴ is N or CR¹⁹;
X²⁵ is NR⁶, S(O)ₘ or O;
m is 0, 1 or 2;
R³ is selected from the group consisting of C₂₋₈ alkyl, C₂₋₈ alkenyl, C₂₋₈ alkynyl, and C₁₋₈ haloalkyl, each optionally substituted; or
R³ is selected from the group consisting of an arylalkyl and heteroarylalkyl group, each optionally substituted; or
R³ is selected from the group consisting of C₃₋₈ cycloalkyl, cycloalkenyl, carbon-attached heterocycloalkyl and carbon-attached heterocycloalkenyl, each optionally substituted; and
R⁶ is selected from the group consisting of hydrogen, C₁₋₈ alkyl, C₃₋₈ alkenyl, C₃₋₈ alkynyl, C₁₋₈ haloalkyl, aryl, heteroaryl and C₃₋₈ cycloalkyl; and
R⁹, R¹⁰, R¹¹, R¹², R¹⁴, R¹⁵, R¹⁶, R¹⁷, R¹⁸ and R¹⁹ are each independently selected from the group consisting of hydrogen, halogen, nitro, cyano, hydroxyl, amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkamino, C₁₋₈ haloalkamino, dialkylamino, alkenylamino, alkynylamino, arylamino, heteroarylamino, C₃₋₈ cycloalkamino, cycloalkenylamino, heterocycloalkylamino, heterocycloalkenylamino, C₁₋₈ alkthio, C₁₋₈ haloalkthio, alkenylthio, alkynylthio, arylthio, heteroarylthio, C₃₋₈ cycloalkthio, cycloalkenylthio, heterocycloalkylthio, heterocycloalkenylthio, -C(=O)R²⁰, -N(R²¹)C(=O)R²², -OC(=O)R²², -N(R²¹)S(=O)₂R²², -S(=O)₂R²⁰, and -S(=O)R²⁰, each optionally substituted; and
R¹³ is selected from the group consisting of hydrogen, halogen, nitro, cyano, hydroxyl, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ haloalkamino, alkenylamino, alkynylamino, arylamino, heteroarylamino, C₃₋₈ cycloalkamino, cycloalkenylamino, heterocycloalkylamino, heterocycloalkenylamino, C₁₋₈ alkthio, C₁₋₈ haloalkthio, alkenylthio, alkynylthio, arylthio, heteroarylthio, C₃₋₈ cycloalkthio, cycloalkenylthio, heterocycloalkylthio, heterocycloalkenylthio, -C(=O)R²⁰, -N(R²¹)C(=O)R²², -OC(=O)R²², -N(R²¹)S(=O)₂R²², -S(=O)₂R²⁰, and -S(=O)R²⁰, each optionally substituted; and
R⁶ and R¹⁶, or R⁶ and R¹⁹, or R⁹ and R¹⁰, or R¹⁰ and R¹¹, or R¹¹ and R¹² or R¹³ and R¹⁴, or R¹⁴ and R¹⁵, or R¹⁷ and R¹⁸ taken together with the atoms to which they are attached form an unsubstituted or substituted fused 5 or 6-membered unsaturated or partially unsaturated ring optionally interrupted by one -O-, -NR⁶-, -S-, -SO- or -SO₂-; and
each R²⁰ is independently selected from the group consisting of hydroxyl, amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkylamino, C₁₋₈ haloalkylamino, dialkylamino, alkenylamino, alkynylamino, arylamino, heteroarylamino, C₃₋₈ cycloalkylamino, cycloalkenylamino, heterocycloalkylamino, and heterocycloalkenylamino, each optionally substituted; and
each R²¹ is independently selected from the group consisting of hydrogen, hydroxyl, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, and heterocycloalkenyloxy, each optionally substituted; and
each R²² is independently selected from the group consisting of amino, C₁₋₈ alkyl, C₂₋₈ alkenyl, C₂₋₈ alkynyl, C₁₋₈ haloalkyl, aryl, heteroaryl, C₃₋₈ cycloalkyl, cycloalkenyl, heterocycloalkyl, heterocycloalkenyl, C₁₋₈ alkoxy, C₁₋₈ haloalkoxy, alkenyloxy, alkynyloxy, aryloxy, heteroaryloxy, C₃₋₈ cycloalkoxy, cycloalkenyloxy, heterocycloalkyloxy, heterocycloalkenyloxy, C₁₋₈ alkylamino, C₁₋₈ haloalkylamino, dialkylamino, alkenylamino, alkynylamino, arylamino, heteroarylamino, C₃₋₈ cycloalkylamino, cycloalkenylamino, heterocycloalkylamino, and heterocycloalkenylamino, each optionally substituted.

14. A compound according to any one of Claims 1-10 or a pharmaceutically acceptable salt thereof, or a composition according to any one of Claims 11 - 13, for use as a medicament.

15. A compound according to any one of Claims 1-10, or a pharmaceutically acceptable salt thereof, or a composition according to any one of Claims 11 - 13, for use in the treatment of a disorder amenable to modulation of α7 nAChR.

16. A compound according to any one of Claims 1-10 or a pharmaceutically acceptable salt thereof, or a composition according to any one of Claims 11 - 13, for use in the treatment of a disorder selected from neurodegenerative diseases, senile dementias, schizophrenia, Alzheimer's disease, learning, cognition and attention deficits, memory loss, Lewy Body dementia, attentiondeficit disorder, attention deficit hyperactivity disorder, anxiety, mania, manic depression, Parkinson's disease, Huntington's disease, depression, amyotrophic lateral sclerosis, brain inflammation, cognitive deficit due to traumatic brain injury, Tourette's syndrome, autism spectrum disorder, a cognitive disorder related to learning or memory comprising, mild cognitive impairment, pain, inflammation, immune system disorders, septic shock, ulcerative colitis, Crohn's disease or irritable bowel syndrome.

## Patentansprüche

1. Verbindung der Formel I: 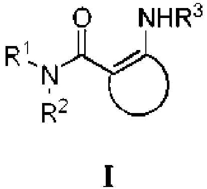 oder pharmazeutisch unbedenkliches Salz davon, mit den folgenden Bedeutungen:
R¹ und R² gemeinsam mit dem Stickstoffatom, an das sie gebunden sind, bilden eine bicyclische Heteroarylgruppe oder eine teilweise ungesättigte bicyclische Heteroarylgruppe, wobei die bicyclische Heteroarylgruppe oder teilweise ungesättigte bicyclische Heteroarylgruppe aus den Folgenden ausgewählt ist: 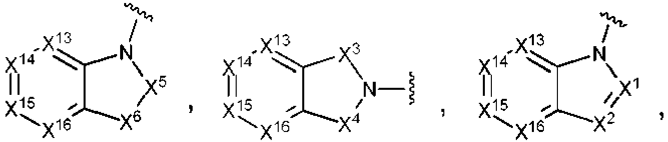 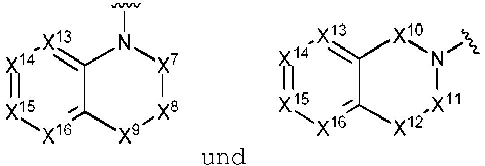
und 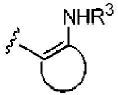 bedeutet eine Heteroarylgruppe, ausgewählt aus der Gruppe bestehend aus: 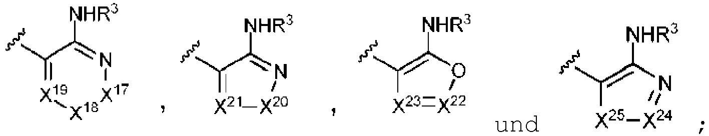
X¹ bedeutet N oder CR⁴;
X² bedeutet N oder CR⁵, nur dass X¹ und X² nicht beide N bedeuten;
jedes von X³, X⁴, X⁵, X⁶, X⁷, X⁸, X⁹, X¹⁰, X¹¹ und X¹² bedeutet unabhängig voneinander O, C=O, S(=O)ₘ, NR⁶ oder CR⁷R⁸;
X¹³ bedeutet N oder CR⁹;
X¹⁴ bedeutet N oder CR¹⁰;
X¹⁵ bedeutet N oder CR¹¹;
X¹⁶ bedeutet N oder CR¹²;
X¹⁷ bedeutet N oder CR¹³;
X¹⁸ bedeutet N oder CR¹⁴;
X¹⁹ bedeutet N oder CR¹⁵;
X²⁰ bedeutet NR⁶, S(O)ₘ oder O;
X²¹ bedeutet N oder CR¹⁶;
X²² bedeutet N oder CR¹⁷;
X²³ bedeutet N oder CR¹⁸;
X²⁴ bedeutet N oder CR¹⁹;
X²⁵ bedeutet NR⁶, S(O)ₘ oder O;
m bedeutet 0, 1 oder 2;
R³ ist aus der Gruppe bestehend aus C₂₋₈-Alkyl, C₂₋₈-Alkenyl, C₂₋₈-Alkinyl und C₁₋₈-Halogenalkyl, jeweils gegebenenfalls substituiert, ausgewählt; oder
R³ ist aus der Gruppe bestehend aus einer Arylalkyl- und Heteroarylalkylgruppe, jeweils gegebenenfalls substituiert, ausgewählt; oder
R³ ist aus der Gruppe bestehend aus C₃₋₈-Cycloalkyl, Cycloalkenyl, über Kohlenstoff gebundenem Heterocycloalkyl und über Kohlenstoff gebundenem Heterocycloalkenyl, jeweils gegebenenfalls substituiert, ausgewählt; und
R⁶ ist aus der Gruppe bestehend aus Wasserstoff, C₁₋₈-Alkyl, C₃₋₈-Alkenyl, C₃₋₈-Alkinyl, C₁₋₈-Halogenalkyl, Aryl, Heteroaryl und C₃₋₈-Cycloalkyl ausgewählt; und
R⁴, R⁵, R⁷, R⁸, R⁹, R¹⁰, R¹¹, R¹², R¹⁴, R¹⁵, R¹⁶, R¹⁷, R¹⁸ und R¹⁹ sind jeweils unabhängig voneinander aus der Gruppe bestehend aus Wasserstoff, Halogen, Nitro, Cyano, Hydroxyl, Amino, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Alkamino, C₁₋₈-Halogenalkamino, Dialkylamino, Alkenylamino, Alkinylamino, Arylamino, Heteroarylamino, C₁₋₈-Cycloalkamino, Cycloalkenylamino, Heterocycloalkylamino, Heterocycloalkenylamino, C₁₋₈-Alkthio, C₁₋₈-Halogenalkthio, Alkenylthio, Alkinylthio, Arylthio, Heteroarylthio, C₃₋₈-Cycloalkthio, Cycloalkenylthio, Heterocycloalkylthio, Heterocycloalkenylthio, -C(=O)R²⁰, -N(R²¹)C(=O)R²², -OC(=O)R²², -N(R²¹)S(=O)₂R²², -S(=O)₂R²⁰ und -S(=O)R²⁰, jeweils gegebenenfalls substituiert, ausgewählt; und
R¹³ ist aus der Gruppe bestehend aus Wasserstoff, Halogen, Nitro, Cyano, Hydroxyl, C₁₋₈-Alkyl, C₂₋₈-Alkenyl, C₂₋₈-Alkinyl, C₁₋₈-Halogenalkyl Aryl, Heteroaryl, C₁₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Halogenalkamino, Alkenylamino, Alkinylamino, Arylamino, Heteroarylamino, C₃₋₈-Cycloalkamino, Cycloalkenylamino, Heterocycloalkylamino, Heterocycloalkenylamino, C₁₋₈-Alkthio, C₁₋₈-Halogenalkthio, Alkenylthio, Alkinylthio, Arylthio, Heteroarylthio, C₃₋₈-Cycloalkthio, Cycloalkenylthio, Heterocycloalkylthio, Heterocycloalkenylthio, -C(=O)R²⁰, -N(R²¹)C(=O)R²², -OC(=O)R²², -N(R²¹)S(=O)₂R²², -S(=O)₂R²⁰ und -S(=O)R²⁰, jeweils gegebenenfalls substituiert, ausgewählt; und
R⁴ und R⁵, oder R⁶ und R¹⁶, oder R⁶ und R¹⁹, oder R⁹ und R¹⁰, oder R¹⁰ und R¹¹, oder R¹¹ und R¹², oder R¹³ und R¹⁴, oder R¹⁴ und R¹⁵, oder R¹⁷ und R¹⁸ gemeinsam mit den Atomen, an die sie gebunden sind, bilden einen unsubstituierten oder substituierten, anellierten 5- oder 6-gliedrigen ungesättigten oder teilweise ungesättigten Ring, der gegebenenfalls durch eine der Gruppen -O-, -NR⁶-, -S-, -SO- oder -SO₂- unterbrochen ist; und
jedes R²⁰ ist unabhängig aus der Gruppe bestehend aus Hydroxyl, Amino, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Alkylamino, C₁₋₈-Halogenalkylamino, Dialkylamino, Alkenylamino, Alkinylamino, Arylamino, Heteroarylamino, C₃₋₈-Cycloalkylamino, Cycloalkenylamino, Heterocycloalkylamino und Heterocycloalkenylamino, jeweils gegebenenfalls substituiert, ausgewählt; und
jedes R²¹ ist unabhängig aus der Gruppe bestehend aus Wasserstoff, Hydroxyl, C₁₋₈-Alkyl, C₂₋₈-Alkeriyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy und Heterocycloalkenyloxy, jeweils gegebenenfalls substituiert, ausgewählt; und
jedes R²² ist unabhängig aus der Gruppe bestehend aus Amino, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Alkylamino, C₁₋₈-Halogenalkylamino, Dialkylamino, Alkenylamino, Alkinylamino, Arylamino, Heteroarylamino, C₃₋₈-Cycloalkylamino, Cycloalkenylamino, Heterocycloalkylamino und Heterocycloalkenylamino, jeweils gegebenenfalls substituiert, ausgewählt.

2. Verbindung nach Anspruch 1, bei der es sich um eine Verbindung gemäß Formel II handelt: 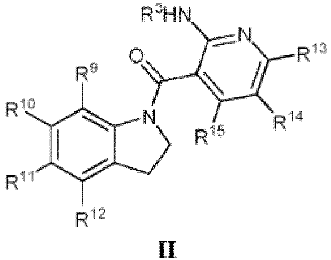 oder pharmazeutisch unbedenkliches Salz davon, mit den folgenden Bedeutungen:
R³ ist aus der Gruppe bestehend aus C₂₋₈-Alkyl, C₂₋₈-Alkenyl, C₂₋₈-Alkinyl, C₁₋₈-Halogenalkyl, jeweils gegebenenfalls substituiert, ausgewählt; oder
R³ ist aus der Gruppe bestehend aus einer Arylalkyl- und Heteroarylalkylgruppe, jeweils gegebenenfalls substituiert, ausgewählt; oder
R³ ist aus der Gruppe bestehend aus C₃₋₈-Cycloalkyl, Cycloalkenyl, über Kohlenstoff gebundenem Heterocycloalkyl und über Kohlenstoff gebundenem Heterocycloalkenyl, jeweils gegebenenfalls substituiert, ausgewählt; und
R⁶ ist aus der Gruppe bestehend aus Wasserstoff, C₁₋₈-Alkyl, C₃₋₈-Alkenyl, C₃₋₈-Alkinyl, C₁₋₈-Halogenalkyl, Aryl, Heteroaryl und C₃₋₈-Cycloalkyl ausgewählt; und
R⁹, R¹⁰, R¹¹, R¹², R¹⁴ und R¹⁵ sind jeweils unabhängig voneinander aus der Gruppe bestehend aus Wasserstoff, Halogen, Nitro, Cyano, Hydroxyl, Amino, C₁₋₈-Alkyl, C₂₋₈-Alkenyl, C₂₋₈-Alkinyl, C₁₋₈-Halogenalkyl Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Alkamino, C₁₋₈-Halogenalkamino, Dialkylamino, Alkenylamino, Alkinylamino, Arylamino, Heteroarylamino, C₁₋₈-Cycloalkamino, Cycloalkenylamino, Heterocycloalkylamino, Heterocycloalkenylamino, C₁₋₈-Alkthio, C₁₋₈-Halogenalkthio, Alkenylthio, Alkinylthio, Arylthio, Heteroarylthio, C₃₋₈-Cycloalkthio, Cycloalkenylthio, Heterocycloalkylthio, Heterocycloalkenylthio, -C(=O)R²⁰, -N(R²¹)C(=O)R²², -OC(=O)R²², -N(R²¹)S(=O)₂R²², -S(=O)₂R²⁰ und -S(=O)R²⁰, jeweils gegebenenfalls substituiert, ausgewählt; und
R¹³ ist aus der Gruppe bestehend aus Wasserstoff, Halogen, Nitro, Cyano, Hydroxyl, C₁₋₈-Alkyl, C₂₋₈-Alkenyl, C₂₋₈-Alkinyl, C₁₋₈-Halogenalkyl Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Halogenalkamino, Alkenylamino, Alkinylamino, Arylamino, Heteroarylamino, C₃₋₈-Cycloalkamino, Cycloalkenylamino, Heterocycloalkylamino, Heterocycloalkenylamino, C₁₋₈-Alkthio, C₁₋₈-Halogenalkthio, Alkenylthio, Alkinylthio, Arylthio, Heteroarylthio, C₃₋₈-Cycloalkthio, Cycloalkenylthio, Heterocycloalkylthio, Heterocycloalkenylthio, -C(=O)R²⁰, -N(R²¹)C(=O)R²², -OC(=O)R²², -N(R²¹)S(=O)₂R²², -S(=O)₂R²⁰ und -S(=O)R²⁰, jeweils gegebenenfalls substituiert, ausgewählt; und
R⁹ und R¹⁰, oder R¹⁰ und R¹¹, oder R¹¹ und R¹², oder R¹³ und R¹⁴, oder R¹⁴ und R¹⁵ gemeinsam mit den Atomen, an die sie gebunden sind, bilden einen unsubstituierten oder substituierten, anellierten 5- oder 6-gliedrigen ungesättigten oder teilweise ungesättigten Ring, der gegebenenfalls durch eine Gruppe -0-, -NR⁶-, -S-, -SO-oder -SO₂- unterbrochen ist; und
jedes R²⁰ ist unabhängig aus der Gruppe bestehend aus Hydroxyl, Amino, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₆-Alkylamino, C₁₋₈-Halogenalkylamino, Dialkylamino, Alkenylamino, Alkinylamino, Arylamino, Heteroarylamino, C₃₋₈-Cycloalkylamino, Cycloalkenylamino, Heterocycloalkylamino und Heterocycloalkenylamino, jeweils gegebenenfalls substituiert, ausgewählt; und
jedes R²¹ ist unabhängig aus der Gruppe bestehend aus Wasserstoff, Hydroxyl, C₁₋₈-Alkyl, C₂₋₈-Alkenyl, C₂₋₈-Alkinyl, C₁₋₈-Haogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy und Heterocycloalkenyloxy, jeweils gegebenenfalls substituiert, ausgewählt; und
jedes R²² ist unabhängig aus der Gruppe bestehend aus Amino, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Alkylamino, C₁₋₈-Halogenalkylamino, Dialkylamino, Alkenylamino, Alkinylamino, Arylamino, Heteroarylamino, C₃₋₈-Cycloalkylamino, Cycloalkenylamino, Heterocycloalkylamino und Heterocycloalkenylamino, jeweils gegebenenfalls substituiert, ausgewählt.

3. Verbindung nach Anspruch 1, bei der es sich um eine Verbindung gemäß Formel **III** handelt: 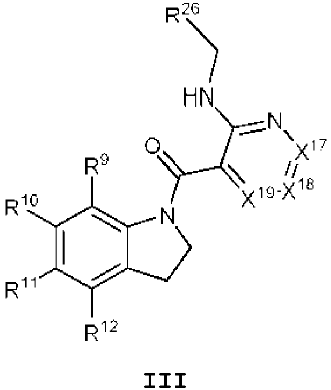 oder pharmazeutisch unbedenkliches Salz davon, mit den folgenden Bedeutungen:
R⁶ ist aus der Gruppe bestehend aus Wasserstoff, C₁₋₈-Alkyl, C₃₋₈-Alkenyl, C₃₋₈-Alkinyl, C₁₋₈-Halogenalkyl, Aryl, Heteroaryl und C₃₋₈-Cycloalkyl ausgewählt; und
R⁹, R¹⁰, R¹¹, R¹², R¹⁴ und R¹⁵ sind jeweils unabhängig voneinander aus der Gruppe bestehend aus Wasserstoff, Halogen, Nitro, Cyano, Hydroxyl, Amino, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Alkamino, C₁₋₈-Halogenalkamino, Dialkylamino, Alkenylamino, Alkinylamino, Arylamino, Heteroarylamino, C₃₋₈-Cycloalkamino, Cycloalkenylamino, Heterocycloalkylamino, Heterocycloalkenylamino, C₁₋₈-Alkthio, C₁₋₈-Halogenalkthio, Alkenylthio, Alkinylthio, Arylthio, Heteroarylthio, C₃₋₈-Cycloalkthio, Cycloalkenylthio, Heterocycloalkylthio, Heterocycloalkenylthio, -C(=O)R²⁰, -N(R²¹)C(=O)R²², -OC(=O)R²², -N(R²¹)S(=O)₂R²², -S(=O)₂R²⁰ und -S(=O)R²⁰, jeweils gegebenenfalls substituiert, ausgewählt; und
R¹³ ist aus der Gruppe bestehend aus Wasserstoff, Halogen, Nitro, Cyano, Hydroxyl, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Halogenalkamino, Alkenylamino, Alkinylamino, Arylamino, Heteroarylamino, C₃₋₈-Cycloalkamino, Cycloalkenylamino, Heterocycloalkylamino, Heterocycloalkenylamino, C₁₋₈-Alkthio, C₁₋₈-Halogenalkthio, Alkenylthio, Alkinylthio, Arylthio, Heteroarylthio, C₃₋₈-Cycloalkthio, Cycloalkenylthio, Heterocycloalkylthio, Heterocycloalkenylthio, -C(=O)R²⁰, -N(R²¹)C(=O)R²², -OC(=O)R²², -N(R²¹)S(=O)₂R²², -S(=O)₂R²⁰ und -S(=O)R²⁰, jeweils gegebenenfalls substituiert, ausgewählt; und
R⁹ und R¹⁰, oder R¹⁰ und R¹¹, oder R¹¹ und R¹² gemeinsam mit den Atomen, an die sie gebunden sind, bilden einen unsubstituierten oder substituierten, anellierten 5- oder 6-gliedrigen ungesättigten oder teilweise ungesättigten Ring, der gegebenenfalls durch eine Gruppe -O-, -NR⁶-, -S-, -SO- oder -SO₂-unterbrochen ist; und
jedes R²⁰ ist unabhängig aus der Gruppe bestehend aus Hydroxyl, Amino, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Alkylamino, C₁₋₈-Halogenalkylamino, Dialkylamino, Alkenylamino, Alkinylamino, Arylamino, Heteroarylamino, C₃₋₈-Cycloalkylamino, Cycloalkenylamino, Heterocycloalkylamino und Heterocycloalkenylamino, jeweils gegebenenfalls substituiert, ausgewählt; und
jedes R²¹ ist unabhängig aus der Gruppe bestehend aus Wasserstoff, Hydroxyl, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, und Heterocycloalkenyloxy, jeweils gegebenenfalls substituiert, ausgewählt; und
jedes R²² ist unabhängig aus der Gruppe bestehend aus Amino, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Alkylamino, C₁₋₈-Halogenalkylamino, Dialkylamino, Alkenylamino, Alkinylamino, Arylamino, Heteroarylamino, C₃₋₈-Cycloalkylamino, Cycloalkenylamino, Heterocycloalkylamino, und Heterocycloalkenylamino, jeweils gegebenenfalls substituiert, ausgewählt; und
R²⁶ ist eine Aryl- oder Heteroarylgruppe, ausgewählt aus: 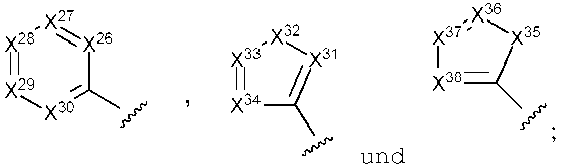
X¹⁷ bedeutet N oder CR¹³;
X¹⁸ bedeutet N oder CR¹⁴;
X¹⁹ bedeutet N oder CR¹⁵;
X²⁶ bedeutet N oder C-R²⁷;
X²⁷ bedeutet N oder C-R²⁸;
X²⁸ bedeutet N oder C-R²⁹;
X²⁹ bedeutet N oder C-R³⁰;
X³⁰ bedeutet N oder C-R³¹;
X³¹ bedeutet N oder C-R³²;
X³² bedeutet NR⁶, O oder S(O)ₘ;
X³³ bedeutet N oder C-R³³;
X³⁴ bedeutet N oder C-R³⁴;
X³⁵ bedeutet NR⁶ oder O;
X³⁶ bedeutet N oder C-R³⁵;
X³⁷ bedeutet N oder C-R³⁶;
X³⁸ bedeutet N oder C-R³⁷;
R²⁷, R²⁸, R³⁰, R³¹, R³², R³³, R³⁴, R³⁵, R³⁶ und R³⁷ sind unabhängig voneinander aus der Gruppe bestehend aus Wasserstoff, Halogen, Nitro, Cyano, Hydroxyl, Amino, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Alkamino, C₁₋₈-Halogenalkamino, Dialkylamino, Alkenylamino, Alkinylamino, Arylamino, Heteroarylamino, C₃₋₈-Cycloalkamino, Cycloalkenylamino, Heterocycloalkylamino, Heterocycloalkenylamino, C₁₋₈-Alkthio, C₁₋₈-Halogenalkthio, Alkenylthio, Alkinylthio, Arylthio, Heteroarylthio, C₃₋₈-Cycloalkthio, Cycloalkenylthio, Heterocycloalkylthio, Heterocycloalkenylthio, -C(=O)R²⁰, -N(R²¹)C(=O)R²², -OC(=O)R²², -N(R²¹)S(=O)₂R²², -S(=O)₂R²⁰ und -S(=O)R²⁰, jeweils gegebenenfalls substituiert, ausgewählt; und
R²⁹ ist aus der Gruppe bestehend aus Wasserstoff, Halogen, Nitro, Cyano, Hydroxyl, Amino, C₁₋₈-Alkyl, C₂₋₈-Alkenyl, C₂₋₈-Alkinyl, C₁₋₈-Halogenalkyl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Alkamino, C₁₋₈-Halogenalkamino, Dialkylamino, Alkenylamino, Alkinylamino, Arylamino, Heteroarylamino, C₃₋₈-Cycloalkamino, Cycloalkenylamino, Heterocycloalkylamino, Heterocycloalkenylamino, C₁₋₈-Alkthio, C₁₋₈-Halogenalkthio, Alkenylthio, Alkinylthio, Arylthio, Heteroarylthio, C₃₋₈-Cycloalkthio, Cycloalkenylthio, Heterocycloalkylthio, Heterocycloalkenylthio, -C(=O)R²⁰, -N(R²¹)C(=O)R²², -OC(=O)R²², -N(R²¹)S(=O)₂R²², -S(=O)₂R²⁰ und -S(=O)R²⁰, jeweils gegebenenfalls substituiert, ausgewählt; und
R²⁷ und R²⁸, oder R²⁸ und R²⁹, oder R²⁹ und R³⁰, oder R³⁰ und R³¹, oder R³² und R⁶, oder R⁶ und R³³, oder R³³ und R³⁴, oder R⁶ und R³⁵, oder R³⁵ und R³⁶, oder R³⁶ und R³⁷ gemeinsam mit den Atomen, an die sie gebunden sind, bilden einen unsubstituierten oder substituierten, anellierten 5- oder 6-gliedrigen ungesättigten oder teilweise ungesättigten Ring, der gegebenenfalls durch eine Gruppe -O-, -NR⁶-, -S-, -SO- oder -SO₂-unterbrochen ist.

4. Verbindung nach Anspruch 1, bei der es sich um eine Verbindung gemäß Formel IV handelt: 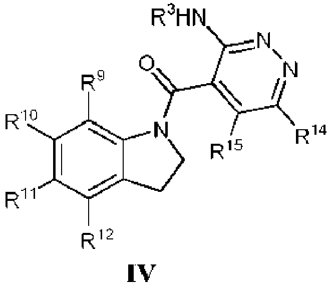 oder pharmazeutisch unbedenkliches Salz davon, mit den folgenden Bedeutungen:
R³ ist aus der Gruppe bestehend aus C₂₋₈-Alkyl, C₂₋₈-Alkenyl, C₂₋₈-Alkinyl und C₁₋₈-Halogenalkyl, jeweils gegebenenfalls substituiert, ausgewählt; oder
R³ ist aus der Gruppe bestehend aus einer Arylalkyl- und Heteroarylalkylgruppe, jeweils gegebenenfalls substituiert, ausgewählt; oder
R³ ist aus der Gruppe bestehend aus C₃₋₈-Cycloalkyl, Cycloalkenyl, über Kohlenstoff gebundenem Heterocycloalkyl und über Kohlenstoff gebundenem Heterocycloalkenyl, jeweils gegebenenfalls substituiert, ausgewählt; und
R⁶ ist aus der Gruppe bestehend aus Wasserstoff, C₁₋₈-Alkyl, C₃₋₈-Alkenyl, C₃₋₈-Alkinyl, C₁₋₈-Halogenalkyl, Aryl, Heteroaryl und C₃₋₈-Cycloalkyl ausgewählt; und
R⁹, R¹⁰, R¹¹, R¹², R¹⁴ und R¹⁵ sind jeweils unabhängig voneinander aus der Gruppe bestehend aus Wasserstoff, Halogen, Nitro, Cyano, Hydroxyl, Amino, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Alkamino, C₁₋₈-Halogenalkamino, Dialkylamino, Alkenylamino, Alkinylamino, Arylamino, Heteroarylamino, C₃₋₈-Cycloalkamino, Cycloalkenylamino, Heterocycloalkylamino, Heterocycloalkenylamino, C₁₋₈-Alkthio, C₁₋₈-Halogenalkthio, Alkenylthio, Alkinylthio, Arylthio, Heteroarylthio, C₃₋₈-Cycloalkthio, Cycloalkenylthio, Heterocycloalkylthio, Heterocycloalkenylthio, -C(=O)R²⁰, -N(R²¹)C(=O)R²², -OC(=O)R²², -N(R²¹)S(=O)₂R²², -S(=O)₂R²⁰ und -S(=O)R²⁰, jeweils gegebenenfalls substituiert, ausgewählt; oder
R⁹ und R¹⁰, oder R¹⁰ und R¹¹, oder R¹¹ und R¹², oder R¹⁴ und R¹⁵ gemeinsam mit den Atomen, an die sie gebunden sind, bilden einen unsubstituierten oder substituierten, anellierten 5- oder 6-gliedrigen ungesättigten oder teilweise ungesättigten Ring, der gegebenenfalls durch eine Gruppe -O-, -NR⁶-, -S-, -SO-oder -SO₂- unterbrochen ist; und
jedes R²⁰ ist unabhängig aus der Gruppe bestehend aus Hydroxyl, Amino, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₆-Alkylamino, C₁₋₈-Halogenalkylamino, Dialkylamino, Alkenylamino, Alkinylamino, Arylamino, Heteroarylamino, C₃₋₈-Cycloalkylamino, Cycloalkenylamino, Heterocycloalkylamino und Heterocycloalkenylamino, jeweils gegebenenfalls substituiert, ausgewählt; und
jedes R²¹ ist unabhängig aus der Gruppe bestehend aus Wasserstoff, Hydroxyl, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy und Heterocycloalkenyloxy, jeweils gegebenenfalls substituiert, ausgewählt; und
jedes R²² ist unabhängig aus der Gruppe bestehend aus Amino, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Alkylamino, C₁₋₈-Halogenalkylamino, Dialkylamino, Alkenylamino, Alkinylamino, Arylamino, Heteroarylamino, C₃₋₈-Cycloalkylamino, Cycloalkenylamino, Heterocycloalkylamino und Heterocycloalkenylamino, jeweils gegebenenfalls substituiert, ausgewählt.

5. Verbindung nach Anspruch 1, bei der es sich um eine Verbindung gemäß Formel **V** handelt: 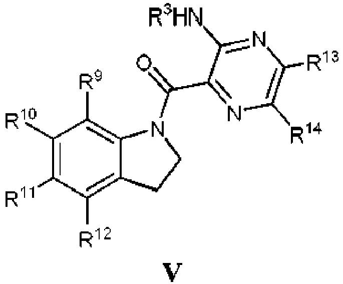 oder pharmazeutisch unbedenkliches Salz davon, mit den folgenden Bedeutungen:
R³ ist aus der Gruppe bestehend aus C₂₋₈-Alkyl, C₂₋₈-Alkenyl, C₂₋₈-Alkinyl und C₁₋₈-Halogenalkyl, jeweils gegebenenfalls substituiert, ausgewählt; oder
R³ ist aus der Gruppe bestehend aus einer Arylalkyl- und Heteroarylalkylgruppe, jeweils gegebenenfalls substituiert, ausgewählt; oder
R³ ist aus der Gruppe bestehend aus C₃₋₈-Cycloalkyl, Cycloalkenyl, über Kohlenstoff gebundenem Heterocycloalkyl und über Kohlenstoff gebundenem Heterocycloalkenyl, jeweils gegebenenfalls substituiert, ausgewählt; und
R⁶ ist aus der Gruppe bestehend aus Wasserstoff, C₁₋₈-Alkyl, C₃₋₈-Alkenyl, C₃₋₈-Alkinyl, C₁₋₈-Halogenalkyl, Aryl, Heteroaryl und C₃₋₈-Cycloalkyl ausgewählt; und
R⁹, R¹⁰, R¹¹, R¹² und R¹⁴ sind jeweils unabhängig voneinander aus der Gruppe bestehend aus Wasserstoff, Halogen, Nitro, Cyano, Hydroxyl, Amino, C₁₋₆-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Alkamino, C₁₋₈-Halogenalkamino, Dialkylamino, Alkenylamino, Alkinylamino, Arylamino, Heteroarylamino, C₃₋₈-Cycloalkamino, Cycloalkenylamino, Heterocycloalkylamino, Heterocycloalkenylamino C₁₋₈-Alkthio, C₁₋₈-Halogenalkthio, Alkenylthio, Alkinylthio, Arylthio, Heteroarylthio, C₃₋₈-Cycloalkthio, Cycloalkenylthio, Heterocycloalkylthio, Heterocycloalkenylthio, -C(=O)R²⁰, -N(R²¹)C(=O)R²², -OC(=O)R²², -N(R²¹)S(=O)₂R²², -S(=O)₂R²⁰ und -S(=O)R²⁰, jeweils gegebenenfalls substituiert, ausgewählt; und
R¹³ ist aus der Gruppe bestehend aus Wasserstoff, Halogen, Nitro, Cyano, Hydroxyl, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Halogenalkamino, Alkenylamino, Alkinylamino, Arylamino, Heteroarylamino, C₃₋₈-Cycloalkamino, Cycloalkenylamino, Heterocycloalkylamino, Heterocycloalkenylamino, C₁₋₈-Alkthio, C₁₋₈-Halogenalkthio, Alkenylthio, Alkinylthio, Arylthio, Heteroarylthio, C₃₋₈-Cycloalkthio, Cycloalkenylthio, Heterocycloalkylthio, Heterocycloalkenylthio, -C(=O)R²⁰, -N(R²¹)C(=O)R²², -OC(=O)R²², -N(R²¹)S(=O)₂R²², -S(=O)₂R²⁰ und -S(=O)R²⁰, jeweils gegebenenfalls substituiert, ausgewählt; und
R⁹ und R¹⁰, oder R¹⁰ und R¹¹, oder R¹¹ und R¹², oder R¹³ und R¹⁴ gemeinsam mit den Atomen, an die sie gebunden sind, bilden einen unsubstituierten oder substituierten, anellierten 5- oder 6-gliedrigen ungesättigten oder teilweise ungesättigten Ring, der gegebenenfalls durch eine Gruppe -0-, -NR⁶-, -S-, -SO- oder -SO₂- unterbrochen ist; und
jedes R²⁰ ist unabhängig aus der Gruppe bestehend aus Hydroxyl, Amino, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₆-Alkylamino, C₁₋₈-Halogenalkylamino, Dialkylamino, Alkenylamino, Alkinylamino, Arylamino, Heteroarylamino, C₃₋₈-Cycloalkylamino, Cycloalkenylamino, Heterocycloalkylamino und Heterocycloalkenylamino, jeweils gegebenenfalls substituiert, ausgewählt; und
jedes R²¹ ist unabhängig aus der Gruppe bestehend aus Wasserstoff, Hydroxyl, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy und Heterocycloalkenyloxy, jeweils gegebenenfalls substituiert, ausgewählt; und
jedes R²² ist unabhängig aus der Gruppe bestehend aus Amino, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Alkylamino, C₁₋₈-Halogenalkylamino, Dialkylamino, Alkenylamino, Alkinylamino, Arylamino, Heteroarylamino, C₃₋₈-Cycloalkylamino, Cycloalkenylamino, Heterocycloalkylamino und Heterocycloalkenylamino, jeweils gegebenenfalls substituiert, ausgewählt.

6. Verbindung nach Anspruch 1, bei der es sich um eine Verbindung gemäß Formel **VI** handelt: 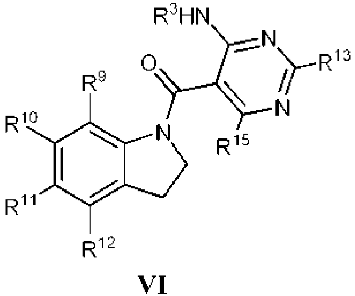 oder pharmazeutisch unbedenkliches Salz davon, mit den folgenden Bedeutungen:
R³ ist aus der Gruppe bestehend aus C₂₋₈-Alkyl, C₂₋₈-Alkenyl, C₂₋₈-Alkinyl und C₁₋₈-Halogenalkyl, jeweils gegebenenfalls substituiert, ausgewählt; oder
R³ ist aus der Gruppe bestehend aus einer Arylalkyl- und Heteroarylalkylgruppe, jeweils gegebenenfalls substituiert, ausgewählt; oder
R³ ist aus der Gruppe bestehend aus C₃₋₈-Cycloalkyl, Cycloalkenyl, über Kohlenstoff gebundenem Heterocycloalkyl und über Kohlenstoff gebundenem Heterocycloalkenyl, jeweils gegebenenfalls substituiert, ausgewählt; und
R⁶ ist aus der Gruppe bestehend aus Wasserstoff, C₁₋₈-Alkyl, C₃₋₈-Alkenyl, C₃₋₈-Alkinyl, C₁₋₈-Halogenalkyl, Aryl, Heteroaryl und C₃₋₈-Cycloalkyl ausgewählt; und
R⁹, R¹⁰, R¹¹, R¹² und R¹⁵ sind jeweils unabhängig voneinander aus der Gruppe bestehend aus Wasserstoff, Halogen, Nitro, Cyano, Hydroxyl, Amino, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Alkamino, C₁₋₈-Halogenalkamino, Dialkylamino, Alkenylamino, Alkinylamino, Arylamino, Heteroarylamino, C₃₋₈-Cycloalkamino, Cycloalkenylamino, Heterocycloalkylamino, Heterocycloalkenylamino, C₁₋₈-Alkthio, C₁₋₈-Halogenalkthio, Alkenylthio, Alkinylthio, Arylthio, Heteroarylthio, C₃₋₈-Cycloalkthio, Cycloalkenylthio, Heterocycloalkylthio, Heterocycloalkenylthio, -C(=O)R²⁰, -N(R²¹)C(=O)R²², -OC(=O)R²², -N(R²¹)S(=O)₂R²², -S(=O)₂R²⁰ und -S(=O)R²⁰, jeweils gegebenenfalls substituiert, ausgewählt; und
R¹³ ist aus der Gruppe bestehend aus Wasserstoff, Halogen, Nitro, Cyano, Hydroxyl, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Halogenalkamino, Alkenylamino, Alkinylamino, Arylamino, Heteroarylamino, C₃₋₈-Cycloalkamino, Cycloalkenylamino, Heterocycloalkylamino, Heterocycloalkenylamino, C₁₋₈-Alkthio, C₁₋₈-Halogenalkthio, Alkenylthio, Alkinylthio, Arylthio, Heteroarylthio, C₃₋₈-Cycloalkthio, Cycloalkenylthio, Heterocycloalkylthio, Heterocycloalkenylthio, -C(=O)R²⁰, -N(R²¹)C(=O)R²², -OC(=O)R²², -N(R²¹)S(=O)₂R²², -S(=O)₂R²⁰ und -S(=O)R²⁰, jeweils gegebenenfalls substituiert, ausgewählt; und
R⁹ und R¹⁰ , oder R¹⁰ und R¹¹, oder R¹¹ und R¹² gemeinsam mit den Atomen, an die sie gebunden sind, bilden einen unsubstituierten oder substituierten, anellierten 5- oder 6-gliedrigen ungesättigten oder teilweise ungesättigten Ring, der gegebenenfalls durch eine Gruppe -0-, -NR⁶-, -S-, -SO- oder -SO₂-unterbrochen ist; und
jedes R²⁰ ist unabhängig aus der Gruppe bestehend aus Hydroxyl, Amino, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₆-Alkylamino, C₁₋₈-Halogenalkylamino, Dialkylamino, Alkenylamino, Alkinylamino, Arylamino, Heteroarylamino, C₃₋₈-Cycloalkylamino, Cycloalkenylamino, Heterocycloalkylamino und Heterocycloalkenylamino, jeweils gegebenenfalls substituiert, ausgewählt; und
jedes R²¹ ist unabhängig aus der Gruppe bestehend aus Wasserstoff, Hydroxyl, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy und Heterocycloalkenyloxy, jeweils gegebenenfalls substituiert, ausgewählt; und
jedes R²² ist unabhängig aus der Gruppe bestehend aus Amino, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Alkylamino, C₁₋₈-Halogenalkylamino, Dialkylamino, Alkenylamino, Alkinylamino, Arylamino, Heteroarylamino, C₃₋₈-Cycloalkylamino, Cycloalkenylamino, Heterocycloalkylamino und Heterocycloalkenylamino, jeweils gegebenenfalls substituiert, ausgewählt.

7. Verbindung nach Anspruch 3, mit den folgenden Bedeutungen:
R⁹, R¹⁰, R¹¹ und R¹² sind jeweils unabhängig voneinander aus der Gruppe bestehend aus Wasserstoff, Halogen, C₁₋₈-Alkyl, C₁₋₈-Halogenalkyl, C₃₋₈-Cycloalkyl, C₁₋₈-Alkoxy, C₁₋₈-Halogenalkoxy und C₃₋₈-Cycloalkoxy, jeweils gegebenenfalls substituiert, ausgewählt; und
R¹³, R¹⁴ und R¹⁵ sind jeweils unabhängig voneinander aus der Gruppe bestehend aus Wasserstoff, Halogen, C₁₋₈-Alkyl, C₁₋₈-Halogenalkyl, C₃₋₈-Cycloalkyl und C₁₋₈-Halogenalkoxy, jeweils gegebenenfalls substituiert, ausgewählt; und
R²⁶ ist eine Arylgruppe, ausgewählt aus: 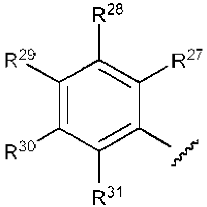
X¹⁷ bedeutet N oder CR¹³;
X¹⁸ bedeutet N oder CR¹⁴;
X¹⁹ bedeutet N oder CR¹⁵;
R²⁷, R²⁸, R²⁹, R³⁰ und R³¹ sind unabhängig voneinander aus der Gruppe bestehend aus Wasserstoff, Halogen, Nitro, Cyano, C₁₋₈-Alkyl, C₁₋₈-Halogenalkyl, C₃₋₈-Cycloalkyl, C₁₋₈-Alkoxy, C₁₋₈-Halogenalkoxy, Alkenyloxy, Alkinyloxy und C₃₋₈-Cycloalkoxy ausgewählt, und pharmazeutisch unbedenkliche Salze davon.

8. Verbindung nach Anspruch 7, mit den folgenden Bedeutungen: R⁹ und R¹² bedeuten Wasserstoff; und pharmazeutisch unbedenkliche Salze davon.

9. Verbindung nach Anspruch 8, mit den folgenden Bedeutungen:
X¹⁷ bedeutet CR¹³;
X¹⁸ bedeutet CR¹⁴;
X¹⁹ bedeutet CR¹⁵;
und pharmazeutisch unbedenkliche Salze davon; oder mit den folgenden Bedeutungen:
X¹⁷ bedeutet CR¹³;
X¹⁸ bedeutet CR¹⁴;
X¹⁹ bedeutet N;
und pharmazeutisch unbedenkliche Salze davon; oder mit den folgenden Bedeutungen:
X¹⁷ bedeutet N;
X¹⁸ bedeutet CR¹⁴;
X¹⁹ bedeutet CR¹⁵;
und pharmazeutisch unbedenkliche Salze davon; oder mit den folgenden Bedeutungen:
X¹⁷ bedeutet CR¹³;
X¹⁸ bedeutet N;
X¹⁹ bedeutet CR¹⁵;
und pharmazeutisch unbedenkliche Salze davon.

10. Verbindung nach Anspruch 1, die aus der Gruppe bestehend aus den Folgenden ausgewählt ist:
[2-(Benzylamino)pyridin-3-yl](5-chlor-2,3-dihydro-1H-indol-1-yl)methanon;
(5-Chlor-2,3-dihydro-1*H*-indol-1-yl)[2-(phenylamino)pyridin-3-yl]methanon;
(5-Chlor-2,3-dihydro-1*H*-indol-1-yl)[2-[(pyridin-2-ylmethyl)amino]pyridin-3-yl]methanon;
(5-Chlor-2,3-dihydro-1*H*-indol-1-yl)[2-[(2-phenylethyl)amino]pyridin-3-yl]methanon;
(5-Chlor-2,3-dihydro-1*H*-indol-1-yl)[2-[(pyridin-3-ylmethyl)amino]pyridin-3-yl]methanon;
(5-Chlor-2,3-dihydro-1*H*-indol-1-yl)[2-[[2-(pyridin-2-yl)ethyl]amino]pyridin-3-yl]methanon;
(5-Chlor-2,3-dihydro-1*H*-indol-1-yl)[2-[(4-fluorbenzyl)amino]pyridin-3-yl]methanon;
(5-Chlor-2,3-dihydro-1*H*-indol-1-yl)[2-[(1-(4-fluorphenyl)ethyl)amino]pyridin-3-yl]methanon;
[2-(Benzylamino)pyridin-3-yl](2,3-dihydro-1*H*-indol-1-yl)methanon;
[3-(Benzylamino)pyridazin-4-yl](5-chlor-2,3-dihydro-1*H-*indol-1-yl)methanon;
(5-Chlor-2,3-dihydro-1H-indol-1-yl)[2-[(4-fluorbenzyl)amino]pyrazin-3-yl]methanon;
(5-Chlor-2,3-dihydro-1H-indol-1-yl)[2-(2,5-difluorbenzylamino)pyridin-3-yl-]methanon;
(5-Chlor-2,3-dihydro-1H-indol-1-yl)[2-(3,4-difluorbenzylamino)pyridin-3-yl]methanon;
(5-Chlor-2,3-dihydro-1H-indol-1-yl)[2-(2,4-difluorbenzylamino)pyridin-3-yl]methanon;
(5-Chlor-2,3-dihydro-1H-indol-1-yl)[2-(cyclopropylmethylamino)pyridin-3-yl]methanon;
(5-Chlor-2,3-dihydro-1H-indol-1-yl)[2-(2-propin-1-ylamino)pyridin-3-yl]methanon;
(5-Fluor-2,3-dihydro-1H-indol-1-yl)[2-(4-fluorbenzylamino)pyridin-3-yl]methanon;
(5-Chlor-2,3-dihydro-1*H*-indol-1-yl)[2-[(pyridin-4-ylamino)pyridin-3-yl]methanon, und pharmazeutisch unbedenklichen Salzen und Solvaten davon.

11. Pharmazeutische Zusammensetzung, umfassend eine Verbindung nach einem der Ansprüche 1-10 oder ein pharmazeutisch unbedenkliches Salz davon sowie einen pharmazeutisch unbedenklichen Träger oder ein pharmazeutisch unbedenkliches Verdünnungsmittel.

12. Pharmazeutische Zusammensetzung nach Anspruch 11, umfassend einen pharmazeutisch unbedenklichen Grundstoff und eine Verbindung, ausgewählt aus:
[2-(Benzylamino)pyridin-3-yl](5-chlor-2,3-dihydro-1H-indol-1-yl)methanon (Verbindung 1);
(5-Chlor-2,3-dihydro-1*H*-indol-1-yl)[2-(phenylamino)pyridin-3-yl]methanon (Verbindung 2);
(5-Chlor-2,3-dihydro-1*H*-indol-1-yl)[2-[(pyridin-2-ylmethyl)amino]pyridin-3-yl]methanon (Verbindung 3);
(5-Chlor-2,3-dihydro-1*H*-indol-1-yl)[2-[(2-phenylethyl)amino]pyridin-3-yl]methanon (Verbindung 4);
(5-Chlor-2,3-dihydro-1*H*-indol-1-yl)[2-[(pyridin-3-ylmethyl)amino]pyridin-3-yl]methanon (Verbindung 5);
(5-Chlor-2,3-dihydro-1*H*-indol-1-yl)[2-[[2-(pyridin-2-yl)ethyl]amino]pyridin-3-yl]methanon (Verbindung 6);
(5-Chlor-2,3-dihydro-1*H*-indol-1-yl)[2-[(4-fluorbenzyl)amino]pyridin-3-yl]methanon (Verbindung 7);
(5-Chlor-2,3-dihydro-1*H*-indol-1-yl)[2-[(1-(4-fluorphenyl)ethyl)amino]pyridin-3-yl]methanon (Verbindung 8);
[2-(Benzylamino)pyridin-3-yl](2,3-dihydro-1*H*-indol-1-yl)methanon (Verbindung 9);
[3-(Benzylamino)pyridazin-4-yl](5-chlor-2,3-dihydro-1*H-*indol-1-yl)methanon (Verbindung 10);
(5-Chlor-2,3-dihydro-1H-indol-1-yl)[2-[(4-fluorbenzyl)amino]pyrazin-3-yl]methanon (Verbindung 11) ;
*N*-(4-Ethoxyphenyl)-2-[(2-phenylethyl)amino]pyridin-3-carboxamid (Verbindung 12);
*N*-Phenyl-2-[(2-phenylethyl)amino]pyridin-3-carboxamid (Verbindung 13);
*N*-(4-Chlorphenyl)-2-[(2-phenylethyl)amino]pyridin-3-carboxamid (Verbindung 14);
2-(Benzylamino)-*N*-(4-ethoxyphenyl)pyridin-3-carboxamid (Verbindung 15);
N-(4-Ethoxyphenyl)-2-(propylamino)pyridin-3-carboxamid (Verbindung 16);
*N*-(4-Hydroxyphenyl)-2-[(2-phenylethyl)amino]pyridin-3-carboxamid (Verbindung 17);
*N*-(4-Ethoxyphenyl)-2-(phenylamino)pyridin-3-carboxamid (Verbindung 18);
2-[(Cyclohexylmethyl)amino]-*N*-(4-ethoxyphenyl)pyridin-3-carboxamid (Verbindung 19);
3-(Benzylamino)-*N*-(4-ethoxyphenyl)pyridin-4-carboxamid (Verbindung 20);
4-(Benzylamino)-*N*-(4-ethoxyphenyl)pyridin-3-carboxamid (Verbindung 21);
3-(Benzylamino)-6-chlor-*N*-(4-ethoxyphenyl)pyridazin-4-carboxamid (Verbindung 22);
2-(Benzylamino)-*N*-[4-(trifluormethyl)phenyl]pyridin-3-carboxamid (Verbindung 24);
2-(Benzylamino)-*N*-(4-fluorphenyl)pyridin-3-carboxamid (Verbindung 25);
*N*-(4-Chlorphenyl)-5-[2-[(4-chlorphenyl)ethyl]amino]-3-methyl-4-isoxazolcarboxamid (Verbindung 26);
(5-Chlor-2,3-dihydro-1H-indol-1-yl)[2-(2,5-difluorbenzylamino)pyridin-3-yl]methanon (Verbindung 27);
(5-Chlor-2,3-dihydro-1H-indol-1-yl)[2-(3,4-difluorbenzylamino)pyridin-3-yl]methanon (Verbindung 28) ;
(5-Chlor-2,3-dihydro-1*H*-indol-1-yl)[2-(2,4-difluorbenzylamino)pyridin-3-yl]methanon (Verbindung 29) ;
(5-Chlor-2,3-dihydro-1*H*-indol-1-yl)[2-(cyclopropylmethylamino)pyridin-3-yl]methanon (Verbindung 30);
(5-Chlor-2,3-dihydro-1H-indol-1-yl)[2-(2-propin-1-ylamino)pyridin-3-yl]methanon (Verbindung 31);
(5-Fluor-2,3-dihydro-1*H*-indol-1-yl)[2-(4-fluorbenzylamino)pyridin-3-yl]methanon (Verbindung 32); und
(5-Chlor-2,3-dihydro-1H-indol-1-yl)[2-[(pyridin-4-ylamino)pyridin-3-yl]methanon (Verbindung 33), und pharmazeutisch unbedenklichen Salzen davon.

13. Pharmazeutische Zusammensetzung nach Anspruch 11, umfassend ein pharmazeutisch unbedenkliches Verdünnungsmittel oder einen pharmazeutisch unbedenklichen Träger und eine Verbindung der Formel **VII:** 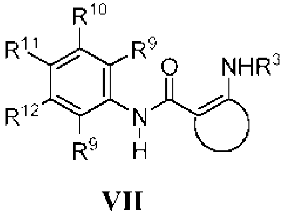 oder ein pharmazeutisch unbedenkliches Salz davon, mit den folgenden Bedeutungen: und 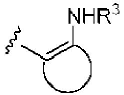 bedeutet eine Heteroarylgruppe, ausgewählt aus der Gruppe bestehend aus: 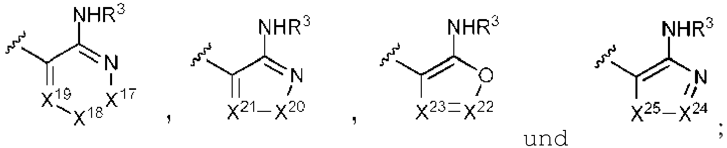
X^{1s} bedeutet N oder CR¹³;
X¹⁸ bedeutet N oder CR¹⁴;
X¹⁹ bedeutet N oder CR¹⁵;
X²⁰ bedeutet NR⁶, S(O)ₘ oder 0;
X²¹ bedeutet N oder CR¹⁶;
X²² bedeutet N oder CR¹⁷;
X²³ bedeutet N oder CR¹⁸;
X²⁴ bedeutet N oder CR¹⁹;
X²⁵ bedeutet NR⁶, S(O)ₘ oder 0;
m bedeutet 0, 1 oder 2;
R³ ist aus der Gruppe bestehend aus C₂₋₈-Alkyl, C₂₋₈-Alkenyl, C₂₋₈-Alkinyl und C₁₋₈-Halogenalkyl, jeweils gegebenenfalls substituiert, ausgewählt; oder
R³ ist aus der Gruppe bestehend aus einer Arylalkyl- und Heteroarylalkylgruppe, jeweils gegebenenfalls substituiert, ausgewählt; oder
R³ ist aus der Gruppe bestehend aus C₃₋₈-Cycloalkyl, Cycloalkenyl, über Kohlenstoff gebundenem Heterocycloalkyl und über Kohlenstoff gebundenem Heterocycloalkenyl, jeweils gegebenenfalls substituiert, ausgewählt; und
R⁶ ist aus der Gruppe bestehend aus Wasserstoff, C₁₋₈-Alkyl, C₃₋₉-Alkenyl, C₃₋₈-Alkinyl, C₁₋₈-Halogenalkyl, Aryl, Heteroaryl und C₃₋₈-Cycloalkyl ausgewählt; und
R⁹, R¹⁰, R¹¹, R¹², R¹⁴, R¹⁵, R¹⁶, R¹⁷, R¹⁸ und R¹⁹ sind jeweils unabhängig voneinander aus der Gruppe bestehend aus Wasserstoff, Halogen, Nitro, Cyano, Hydroxyl, Amino, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Alkamino, C₁₋₈-Halogenalkamino, Dialkylamino, Alkenylamino, Alkinylamino, Arylamino, Heteroarylamino, C₃₋₈-Cycloalkamino, Cycloalkenylamino, Heterocycloalkylamino, Heterocycloalkenylamino, C₁₋₈-Alkthio, C₁₋₈-Halogenalkthio, Alkenylthio, Alkinylthio, Arylthio, Heteroarylthio, C₃₋₈-Cycloalkthio, Cycloalkenylthio, Heterocycloalkylthio, Heterocycloalkenylthio, -C(=O)R²⁰, -N(R²¹)C(=O)R²², -OC(=O)R²², -N(R²¹)S(=O)₂R²², -S(=O)₂R²⁰ und -S(=O)R²⁰, jeweils gegebenenfalls substituiert, ausgewählt; und
R¹³ ist aus der Gruppe bestehend aus Wasserstoff, Halogen, Nitro, Cyano, Hydroxyl, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Halogenalkamino, Alkenylamino, Alkinylamino, Arylamino, Heteroarylamino, C₃₋₈-Cycloalkamino, Cycloalkenylamino, Heterocycloalkylamino, Heterocycloalkenylamino, C₁₋₈-Alkthio, C₁₋₈-Halogenalkthio, Alkenylthio, Alkinylthio, Arylthio, Heteroarylthio, C₃₋₈-Cycloalkthio, Cycloalkenylthio, Heterocycloalkylthio, Heterocycloalkenylthio, -C(=O)R²⁰, -N(R²¹)C(=O)R²², -OC(=O)R²², -N(R²¹) S(=O)₂R²², -S(=O)₂R²⁰ und -S(=O)R²⁰, jeweils gegebenenfalls substituiert, ausgewählt; und
R⁶ und R¹⁶, oder R⁶ und R¹⁹, oder R⁹ und R¹⁰, oder R¹⁰ und R¹¹, oder R¹¹ und R¹² oder R¹³ und R¹⁴, oder R¹⁴ und R¹⁵, oder R¹⁷ und R¹⁸ gemeinsam mit den Atomen, an die sie gebunden sind, bilden einen unsubstituierten oder substituierten, anellierten 5- oder 6-gliedrigen ungesättigten oder teilweise ungesättigten Ring, der gegebenenfalls durch eine Gruppe -0-, -NR⁶-, -S-, -SO- oder -SO₂- unterbrochen ist; und
jedes R²⁰ ist unabhängig aus der Gruppe bestehend aus Hydroxyl, Amino, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Alkylamino, C₁₋₈-Halogenalkylamino, Dialkylamino, Alkenylamino, Alkinylamino, Arylamino, Heteroarylamino, C₃₋₈-Cycloalkylamino, Cycloalkenylamino, Heterocycloalkylamino und Heterocycloalkenylamino, jeweils gegebenenfalls substituiert, ausgewählt; und
jedes R²¹ ist unabhängig aus der Gruppe bestehend aus Wasserstoff, Hydroxyl, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy und Heterocycloalkenyloxy, jeweils gegebenenfalls substituiert, ausgewählt; und
jedes R²² ist unabhängig aus der Gruppe bestehend aus Amino, C₁₋₈-Alkyl, C₂₋₈-Alkenyl, C₂₋₈-Alkinyl, C₁₋₈-Halogenalkyl, Aryl, Heteroaryl, C₃₋₈-Cycloalkyl, Cycloalkenyl, Heterocycloalkyl, Heterocycloalkenyl, C₁₋₈-Alkoxy, C₁₋₈-Halogenalkoxy, Alkenyloxy, Alkinyloxy, Aryloxy, Heteroaryloxy, C₃₋₈-Cycloalkoxy, Cycloalkenyloxy, Heterocycloalkyloxy, Heterocycloalkenyloxy, C₁₋₈-Alkylamino, C₁₋₈-Halogenalkylamino, Dialkylamino, Alkenylamino, Alkinylamino, Arylamino, Heteroarylamino, C₃₋₈-Cycloalkylamino, Cycloalkenylamino, Heterocycloalkylamino und Heterocycloalkenylamino, jeweils gegebenenfalls substituiert, ausgewählt.

14. Verbindung nach einem der Ansprüche 1 - 10 oder pharmazeutisch unbedenkliches Salz davon, oder Zusammensetzung nach einem der Ansprüche 11 - 13, zur Verwendung als Arzneimittel.

15. Verbindung nach einem der Ansprüche 1-10 oder pharmazeutisch unbedenkliches Salz davon, oder Zusammensetzung nach einem der Ansprüche 11 - 13, zur Verwendung in der Behandlung einer Krankheit, die gegenüber Modulation von α7-nAChR zugänglich ist.

16. Verbindung nach einem der Ansprüche 1 - 10 oder pharmazeutisch unbedenkliches Salz davon, oder Zusammensetzung nach einem der Ansprüche 11 - 13, zur Verwendung in der Behandlung einer Krankheit, ausgewählt aus neurodegenerativen Erkrankungen, senilen Demenzen, Schizophrenie, Morbus Alzheimer, Lernschwäche, Denkschwäche und Aufmerksamkeitsschwäche, Gedächtnisverlust, Lewy-Körper-Demenz, Aufmerksamkeitsmangel-Störung, Aufmerksamkeitsmangel-Hyperaktivitätsstörung, Angst, Manie, manischer Depression, Morbus Parkinson, Morbus Huntington, Depression, amyotropher Lateralsklerose, Entzündung des Gehirns, kognitiver Störung aufgrund von traumatischer Hirnverletzung, Tourette-Syndrom, Autismusspektrum-Störung, einer kognitiven Störung, die mit dem Lernvermögen oder dem Gedächtnis in Zusammenhang steht, umfassend milde kognitive Behinderung, Schmerzen, Entzündung, Immunsystemstörungen, Sepsis-Schock, ulzerierender Kolitis, Morbus Crohn oder Reizdarmsyndrom.

## Revendications

1. Composé de formule I : 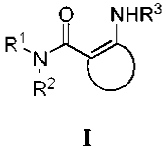 ou sel pharmaceutiquement acceptable de celui-ci, dans lequel :
R¹ et R² conjointement avec l'atome d'azote auquel ils sont liés forment un groupe hétéroaryle bicyclique ou un groupe hétéroaryle bicyclique partiellement insaturé où ledit groupe hétéroaryle bicyclique ou groupe hétéroaryle bicyclique partiellement insaturé est choisi parmi ce qui suit : 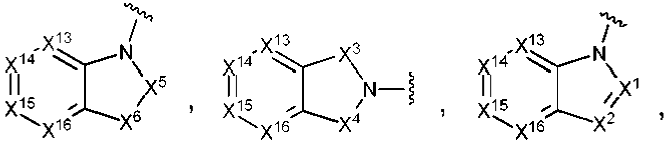 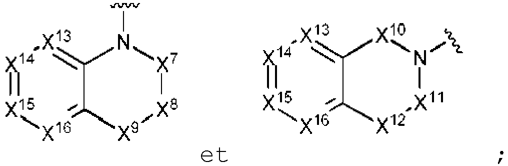 et 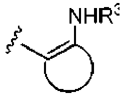 est un groupe hétéroaryle choisi dans le groupe constitué de : 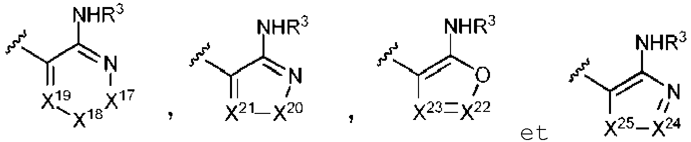
X¹ est N ou CR⁴ ;
X² est N ou CR⁵ à l'exception du fait que X¹ et X² ne sont pas tous deux N ;
chacun de X³, X⁴, X⁵, X⁶, X⁷, X⁸, X⁹, X¹⁰, X¹¹ et X¹² est indépendamment O, C=O, S(=O)ₘ, NR⁶ ou CR⁷R⁸ ;
X¹³ est N ou CR⁹ ;
X¹⁴ est N ou CR¹⁰ ;
X¹⁵ est N ou CR¹¹ ;
X¹⁶ est N ou CR¹² ;
X¹⁷ est N ou CR¹³ ;
X¹⁸ est N ou CR¹⁴ ;
X¹⁹ est N ou CR¹⁵ ;
X²⁰ est NR⁶, S(O)ₘ ou O ;
X²¹ est N ou CR¹⁶ ;
X²² est N ou CR¹⁷ ;
X²³ est N ou CR¹⁸ ;
X²⁴ est N ou CR¹⁹ ;
X²⁵ est NR⁶, S(O)ₘ ou O ;
m est 0, 1 ou 2 ;
R³ est choisi dans le groupe constitué d'alkyle en C₂₋₈, alcényle en C₂₋₈, alcynyle en C₂₋₈, et halogénoalkyle en C₁₋₈, chacun facultativement substitué ; ou
R³ est choisi dans le groupe constitué d'un groupe arylalkyle et hétéroarylalkyle, chacun facultativement substitué ; ou
R³ est choisi dans le groupe constitué de cycloalkyle en C₃₋₈, cycloalcényle, hétérocycloalkyle lié à un carbone et hétérocycloalcényle lié à un carbone, chacun facultativement substitué ; et
R⁶ est choisi dans le groupe constitué d'hydrogène, alkyle en C₁₋₈, alcényle en C₃₋₈, alcynyle en C₃₋₈, halogénoalkyle en C₁₋₈, aryle, hétéroaryle et cycloalkyle en C₃₋₈ ; et
R⁴, R⁵, R⁷, R⁸, R⁹, R¹⁰, R¹¹, R¹², R¹⁴, R¹⁵, R¹⁶, R¹⁷, R¹⁸ et R¹⁹ sont chacun indépendamment choisis dans le groupe constitué d'hydrogène, halogène, nitro, cyano, hydroxyle, amino, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₈)amino, halogéno (alkyle en C₁₋₈)amino, dialkylamino, alcénylamino, alcynylamino, arylamino, hétéroarylamino, (cycloalkyle en C₁₋₈)amino, cycloalcénylamino, hétérocycloalkylamino, hétérocycloalcénylamino, (alkyle en C₁₋₈)thio, halogéno (alkyle en C₁₋₈)thio, alcénylthio, alcynylthio, arylthio, hétéroarylthio, (cycloalkyle en C₃₋₈)thio, cycloalcénylthio, hétérocycloalkylthio, hétérocycloalcénylthio, -C(=O)R²⁰, -N(R²¹)C(=O)R²², -OC(= O)R²², -N(R²¹)S(=O)₂R²², -S(=O)₂R²⁰, et -S(=O)R²⁰, chacun facultativement substitué ; et
R¹³ est choisi dans le groupe constitué d'hydrogène, halogène, nitro, cyano, hydroxyle, alkyle en C₁₋₈, alcényle en C₂₋₈, alcynyle en C₂₋₈, halogénoalkyle en C₁₋₈, aryle, hétéroaryle, cycloalkyle en C₁₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, halogéno(alkyle en C₁₋₈)amino, alcénylamino, alcynylamino, arylamino, hétéroarylamino, (cycloalkyle en C₃₋₈)amino, cycloalcénylamino, hétérocycloalkylamino, hétérocycloalcénylamino, (alkyle en C₁₋₈)thio, halogéno (alkyle en C₁₋₈)thio, alcénylthio, alcynylthio, arylthio, hétéroarylthio, (cycloalkyle en C₃₋₈)thio, cycloalcénylthio, hétérocycloalkylthio, hétérocycloalcénylthio, -C(=O)R²⁰, -N(R²¹)C(=O)R²², -OC(= O)R²², -N(R²¹)S(=O)₂R²², -S(=O)₂R²⁰, et -S(=O)R²⁰, chacun facultativement substitué ; et
R⁴ et R⁵, ou R⁶ et R¹⁶, ou R⁶ et R¹⁹, ou R⁹ et R¹⁰, ou R¹⁰ et R¹¹, ou R¹¹ et R¹², ou R¹³ et R¹⁴, ou R¹⁴ et R¹⁵, ou R¹⁷ et R¹⁸ conjointement avec les atomes auxquels ils sont liés forment un cycle insaturé ou partiellement insaturé de 5 ou 6 chaînons condensé non substitué ou substitué facultativement interrompu par un -0-, -NR⁶-, -S-, -SO- ou -SO₂- ; et
chaque R²⁰ est indépendamment choisi dans le groupe constitué d'hydroxyle, amino, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₈)amino, halogéno (alkyle en C₁₋₈)amino, dialkylamino, alcénylamino, alcynylamino, arylamino, hétéroarylamino, (cycloalkyle en C₃₋₈)amino, cycloalcénylamino, hétérocycloalkylamino, et hétérocycloalcénylamino, chacun facultativement substitué ; et
chaque R²¹ est indépendamment choisi dans le groupe constitué d'hydrogène, hydroxyle, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, et hétérocycloalcényloxy, chacun facultativement substitué ; et
chaque R²² est indépendamment choisi dans le groupe constitué d' amino, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₈)amino, halogéno (alkyle en C₁₋₈)amino, dialkylamino, alcénylamino, alcynylamino, arylamino, hétéroarylamino, (cycloalkyle en C₃₋₈)amino, cycloalcénylamino, hétérocycloalkylamino, et hétérocycloalcénylamino, chacun facultativement substitué.

2. Composé selon la revendication 1, qui est un composé selon la formule II : 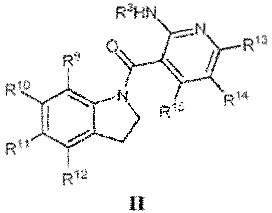 ou sel pharmaceutiquement acceptable de celui-ci, dans lequel :
R³ est choisi dans le groupe constitué d'alkyle en C₂₋₈, alcényle en C₂₋₈, alcynyle en C₂₋₈, halogénoalkyle en C₁₋₈, chacun facultativement substitué ; ou
R³ est choisi dans le groupe constitué d'un groupe arylalkyle et hétéroarylalkyle, chacun facultativement substitué ; ou
R³ est choisi dans le groupe constitué de cycloalkyle en C₃₋₈, cycloalcényle, hétérocycloalkyle lié à un carbone et hétérocycloalcényle lié à un carbone, chacun facultativement substitué ; et
R⁶ est choisi dans le groupe constitué d'hydrogène, alkyle en C₁₋₈, alcényle en C₃₋₈, alcynyle en C₃₋₈, halogénoalkyle en C₁₋₈, aryle, hétéroaryle et cycloalkyle en C₃₋₈ ; et
R⁹, R¹⁰, R¹¹, R¹², R¹⁴ et R¹⁵ sont chacun indépendamment choisis dans le groupe constitué d'hydrogène, halogène, nitro, cyano, hydroxyle, amino, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₈)amino, halogéno (alkyle en C₁₋₈)amino, dialkylamino, alcénylamino, alcynylamino, arylamino, hétéroarylamino, (cycloalkyle en C₁₋₈)amino, cycloalcénylamino, hétérocycloalkylamino, hétérocycloalcénylamino, (alkyle en C₁₋₈)thio, halogéno (alkyle en C₁₋₈)thio, alcénylthio, alcynylthio, arylthio, hétéroarylthio, (cycloalkyle en C₃₋₈)thio, cycloalcénylthio, hétérocycloalkylthio, hétérocycloalcénylthio, -C(=O)R²⁰, -N(R²¹)C(=O)R²², -OC(= O)R²², -N(R²¹)S(=O)₂R²², -S(=O)₂R²⁰, et -S(=O)R²⁰, chacun facultativement substitué ; et
R¹³ est choisi dans le groupe constitué d'hydrogène, halogène, nitro, cyano, hydroxyle, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, halogéno(alkyle en C₁₋₈)amino, alcénylamino, alcynylamino, arylamino, hétéroarylamino, (cycloalkyle en C₃₋₈)amino, cycloalcénylamino, hétérocycloalkylamino, hétérocycloalcénylamino, (alkyle en C₁₋₈)thio, halogéno (alkyle en C₁₋₈)thio, alcénylthio, alcynylthio, arylthio, hétéroarylthio, (cycloalkyle en C₃₋₈)thio, cycloalcénylthio, hétérocycloalkylthio, hétérocycloalcénylthio, -C(=O)R²⁰, -N(R²¹)C(=O)R²², -OC(= O)R²², -N(R²¹)S(=O)₂R²², -S(=O)₂R²⁰, et -S(=O)R²⁰, chacun facultativement substitué ; et
R⁹ et R¹⁰, ou R¹⁰ et R¹¹, ou R¹¹ et R¹², ou R¹³ et R¹⁴, ou R¹⁴ et R¹⁵ conjointement avec les atomes auxquels ils sont liés forment un cycle insaturé ou partiellement insaturé de 5 ou 6 chaînons condensé non substitué ou substitué facultativement interrompu par un -O-, -NR⁶-, -S- , -SO- ou -SO₂- ; et
chaque R²⁰ est indépendamment choisi dans le groupe constitué d'hydroxyle, amino, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₆)amino, halogéno (alkyle en C₁₋₈)amino, dialkylamino, alcénylamino, alcynylamino, arylamino, hétéroarylamino, (cycloalkyle en C₃₋₈)amino, cycloalcénylamino, hétérocycloalkylamino, et hétérocycloalcénylamino, chacun facultativement substitué ; et
chaque R²¹ est indépendamment choisi dans le groupe constitué d'hydrogène, hydroxyle, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, et
hétérocycloalcényloxy, chacun facultativement substitué ; et
chaque R²² est indépendamment choisi dans le groupe constitué d' amino, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₈)amino, halogéno (alkyle en C₁₋₈)amino, dialkylamino, alcénylamino, alcynylamino, arylamino, hétéroarylamino, (cycloalkyle en C₃₋₈)amino, cycloalcénylamino, hétérocycloalkylamino, et hétérocycloalcénylamino, chacun facultativement substitué.

3. Composé selon la revendication 1, qui est un composé selon la formule III : 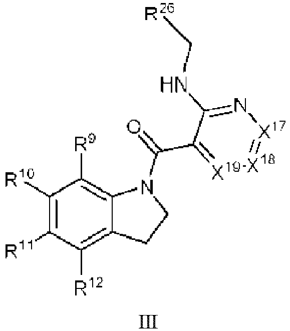 ou sel pharmaceutiquement acceptable de celui-ci, dans lequel :
R⁶ est choisi dans le groupe constitué d'hydrogène, alkyle en C₁₋₈, alcényle en C₃₋₈, alcynyle en C₃₋₈, halogénoalkyle en C₁₋₈, aryle, hétéroaryle et cycloalkyle en C₃₋₈ ; et
R⁹, R¹⁰, R¹¹, R¹², R¹⁴ et R¹⁵ sont chacun indépendamment choisis dans le groupe constitué d'hydrogène, halogène, nitro, cyano, hydroxyle, amino, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₈)amino, halogéno (alkyle en C₁₋₈)amino, dialkylamino, alcénylamino, alcynylamino, arylamino, hétéroarylamino, (cycloalkyle en C₃₋₈)amino, cycloalcénylamino, hétérocycloalkylamino, hétérocycloalcénylamino, (alkyle en C₁₋₈)thio, halogéno (alkyle en C₁₋₈)thio, alcénylthio, alcynylthio, arylthio, hétéroarylthio, (cycloalkyle en C₃₋₈)thio, cycloalcénylthio, hétérocycloalkylthio, hétérocycloalcénylthio, -C(=O)R²⁰, -N(R²¹)C(=O)R²², -OC(= O)R²², -N(R²¹)S(=O)₂R²², -S(=O)₂R²⁰, et -S(=O)R²⁰, chacun facultativement substitué ; et
R¹³ est choisi dans le groupe constitué d'hydrogène, halogène, nitro, cyano, hydroxyle, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, halogéno(alkyle en C₁₋₈)amino, alcénylamino, alcynylamino, arylamino, hétéroarylamino, (cycloalkyle en C₃₋₈)amino, cycloalcénylamino, hétérocycloalkylamino, hétérocycloalcénylamino, (alkyle en C₁₋₈)thio, halogéno (alkyle en C₁₋₈)thio, alcénylthio, alcynylthio, arylthio, hétéroarylthio, (cycloalkyle en C₃₋₈)thio, cycloalcénylthio, hétérocycloalkylthio, hétérocycloalcénylthio, -C(=O)R²⁰, -N(R²¹)C(=O)R²², -OC(= O)R²², -N(R²¹)S(=O)₂R²², -S(=O)₂R²⁰, et -S(=O)R²⁰, chacun facultativement substitué ; et
R⁹ et R¹⁰ , ou R¹⁰ et R¹¹, ou R¹¹ et R¹² conjointement avec les atomes auxquels ils sont liés forment un cycle insaturé ou partiellement insaturé de 5 ou 6 chaînons condensé non substitué ou substitué facultativement interrompu par un -0-, -NR⁶-, -S-, -SO- ou -SO₂- ; et
chaque R²⁰ est indépendamment choisi dans le groupe constitué d'hydroxyle, amino, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₈)amino, halogéno (alkyle en C₁₋₈)amino, dialkylamino, alcénylamino, alcynylamino, arylamino, hétéroarylamino, (cycloalkyle en C₃₋₈)amino, cycloalcénylamino, hétérocycloalkylamino, et hétérocycloalcénylamino,
chacun facultativement substitué ; et
chaque R²¹ est indépendamment choisi dans le groupe constitué d'hydrogène, hydroxyle, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, et hétérocycloalcényloxy, chacun facultativement substitué ; et
chaque R²² est indépendamment choisi dans le groupe constitué d' amino, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₈)amino, halogéno (alkyle en C₁₋₈)amino, dialkylamino, alcénylamino, alcynylamino, arylamino, hétéroarylamino, (cycloalkyle en C₃₋₈)amino, cycloalcénylamino, hétérocycloalkylamino, et hétérocycloalcénylamino, chacun facultativement substitué ; et
R²⁶ est un groupe aryle ou hétéroaryle choisi parmi : 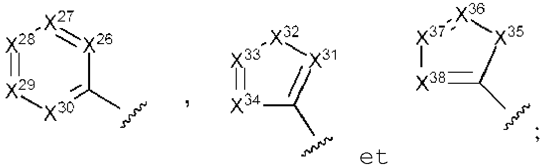
X¹⁷ est N ou CR¹³ ;
X¹⁸ est N ou CR¹⁴ ;
X¹⁹ est N ou CR¹⁵ ;
X²⁶ est N ou C-R²⁷ ;
X²⁷ est N ou C-R²⁸ ;
X²⁸ est N ou C-R²⁹ ;
X²⁹ est N ou C-R³⁰ ;
X³⁰ est N ou C-R³¹ ;
X³¹ est N ou C-R³² ;
X³² est NR⁶, O ou S(O)ₘ ;
X³³ est N ou C-R³³ ;
X³⁴ est N ou C-R³⁴ ;
X³⁵ est NR⁶ ou O ;
X³⁶ est N ou C-R³⁵ ;
X³⁷ est N ou C-R³⁶ ;
X³⁸ est N ou C-R³⁷ ;
R²⁷, R²⁸, R³⁰, R³¹, R³², R³³, R³⁴, R³⁵, R³⁶ et R³⁷ sont indépendamment choisis dans le groupe constitué d'hydrogène, halogène, nitro, cyano, hydroxyle, amino, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₈)amino, halogéno (alkyle en C₁₋₈)amino, dialkylamino, alcénylamino, alcynylamino, arylamino, hétéroarylamino, (cycloalkyle en C₃₋₈)amino, cycloalcénylamino, hétérocycloalkylamino, hétérocycloalcénylamino, (alkyle en C₁₋₈)thio, halogéno(alkyle en C₁₋₈)thio, alcénylthio, alcynylthio, arylthio, hétéroarylthio, (cycloalkyle en C₃₋₈)thio, cycloalcénylthio, hétérocycloalkylthio, hétérocycloalcénylthio, -C(=O)R²⁰, -N(R²¹)C(=O)R²², -OC(= O)R²², -N(R²¹)S(=O)₂R²², -S(=O)₂R²⁰, et -S(=O)R²⁰, chacun facultativement substitué ; et
R²⁹ est choisi dans le groupe constitué d'hydrogène, halogène, nitro, cyano, hydroxyle, amino, alkyle en C₁₋₈, alcényle en C₂₋₈, alcynyle en C₂₋₈, halogénoalkyle en C₁₋₈, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₈)amino, halogéno (alkyle en C₁₋₈)amino, dialkylamino, alcénylamino, alcynylamino, arylamino, hétéroarylamino, (cycloalkyle en C₃₋₈)amino, cycloalcénylamino, hétérocycloalkylamino, hétérocycloalcénylamino, (alkyle en C₁₋₈)thio, halogéno (alkyle en C₁₋₈)thio, alcénylthio, alcynylthio, arylthio, hétéroarylthio, (cycloalkyle en C₃₋₈)thio, cycloalcénylthio, hétérocycloalkylthio, hétérocycloalcénylthio, -C(=O)R²⁰, -N(R²¹)C(=O)R²², -OC(= O)R²², -N(R²¹)S(=O)₂R²², -S(=O)₂R²⁰, et -S(=O)R²⁰, chacun facultativement substitué ; et
R²⁷ et R²⁸, ou R²⁸ et R²⁹, ou R²⁹ et R³⁰, ou R³⁰ et R³¹, ou R³² et R⁶, ou R⁶ et R³³, ou R³³ et R³⁴, ou R⁶ et R³⁵, ou R³⁵ et R³⁶, ou R³⁶ et R³⁷ conjointement avec les atomes auxquels ils sont liés forment un cycle insaturé ou partiellement insaturé de 5 ou 6 chaînons condensé non substitué ou substitué facultativement interrompu par -O-, -NR⁶-, -S-, -SO- ou -SO₂-.

4. Composé selon la revendication 1, qui est un composé selon la formule IV : 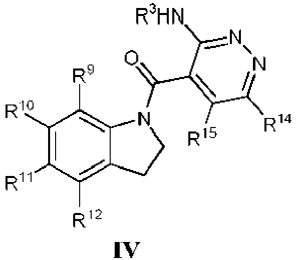 ou sel pharmaceutiquement acceptable de celui-ci, dans lequel :
R³ est choisi dans le groupe constitué d'alkyle en C₂₋₈, alcényle en C₂₋₈, alcynyle en C₂₋₈, et halogénoalkyle en C₁₋₈, chacun facultativement substitué ; ou
R³ est choisi dans le groupe constitué d'un groupe arylalkyle et hétéroarylalkyle, chacun facultativement substitué ; ou
R³ est choisi dans le groupe constitué de cycloalkyle en C₃₋₈, cycloalcényle, hétérocycloalkyle lié à un carbone et hétérocycloalcényle lié à un carbone, chacun facultativement substitué ; et
R⁶ est choisi dans le groupe constitué d'hydrogène, alkyle en C₁₋₈, alcényle en C₃₋₈, alcynyle en C₃₋₈, halogénoalkyle en C₁₋₈, aryle, hétéroaryle et cycloalkyle en C₃₋₈ ; et
R⁹, R¹⁰, R¹¹, R¹², R¹⁴ et R¹⁵ sont chacun indépendamment choisis dans le groupe constitué d'hydrogène, halogène, nitro, cyano, hydroxyle, amino, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₈)amino, halogéno (alkyle en C₁₋₈)amino, dialkylamino, alcénylamino, alcynylamino, arylamino, hétéroarylamino, (cycloalkyle en C₃₋₈)amino, cycloalcénylamino, hétérocycloalkylamino, hétérocycloalcénylamino, (alkyle en C₁₋₈)thio, halogéno (alkyle en C₁₋₈)thio, alcénylthio, alcynylthio, arylthio, hétéroarylthio, (cycloalkyle en C₃₋₈)thio, cycloalcénylthio, hétérocycloalkylthio, hétérocycloalcénylthio, -C(=O)R²⁰, -N(R²¹)C(=O)R²², -OC(= O)R²², -N(R²¹)S(=O)₂R²², -S(=O)₂R²⁰, et -S(=O)R²⁰, chacun facultativement substitué ; ou
R⁹ et R¹⁰, ou R¹⁰ et R¹¹, ou R¹¹ et R¹², ou R¹⁴ et R¹⁵ conjointement avec les atomes auxquels ils sont liés forment un cycle insaturé ou partiellement insaturé de 5 ou 6 chaînons condensé non substitué ou substitué facultativement interrompu par -O-, -NR⁶-, -S-, -SO- ou -SO₂- ; et
chaque R²⁰ est indépendamment choisi dans le groupe constitué d'hydroxyle, amino, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₆)amino, halogéno (alkyle en C₁₋₈)amino, dialkylamino, alcénylamino, alcynylamino, arylamino, hétéroarylamino, (cycloalkyle en C₃₋₈)amino, cycloalcénylamino, hétérocycloalkylamino, et hétérocycloalcénylamino, chacun facultativement substitué ; et
chaque R²¹ est indépendamment choisi dans le groupe constitué d'hydrogène, hydroxyle, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, et hétérocycloalcényloxy, chacun facultativement substitué ; et
chaque R²² est indépendamment choisi dans le groupe constitué d' amino, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₈)amino, halogéno (alkyle en C₁₋₈)amino, dialkylamino, alcénylamino, alcynylamino, arylamino, hétéroarylamino, (cycloalkyle en C₃₋₈)amino, cycloalcénylamino, hétérocycloalkylamino, et hétérocycloalcénylamino, chacun facultativement substitué.

5. Composé selon la revendication 1, qui est un composé selon la formule V : 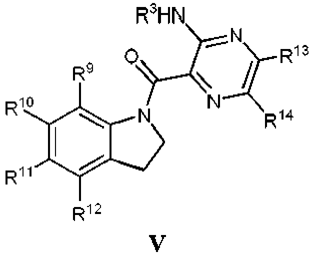 ou sel pharmaceutiquement acceptable de celui-ci, dans lequel :
R³ est choisi dans le groupe constitué d'alkyle en C₂₋₈, alcényle en C₂₋₈, alcynyle en C₂₋₈, et halogénoalkyle en C₁₋₈, chacun facultativement substitué ; ou
R³ est choisi dans le groupe constitué d'un groupe arylalkyle et hétéroarylalkyle, chacun facultativement substitué ; ou
R³ est choisi dans le groupe constitué de cycloalkyle en C₃₋₈, cycloalcényle, hétérocycloalkyle lié à un carbone et hétérocycloalcényle lié à un carbone, chacun facultativement substitué ; et
R⁶ est choisi dans le groupe constitué d'hydrogène, alkyle en C₁₋₈, alcényle en C₃₋₈, alcynyle en C₃₋₈, halogénoalkyle en C₁₋₈, aryle, hétéroaryle et cycloalkyle en C₃₋₈ ; et
R⁹, R¹⁰, R¹¹, R¹² et R¹⁴ sont chacun indépendamment choisis dans le groupe constitué d'hydrogène, halogène, nitro, cyano, hydroxyle, amino, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₈)amino, halogéno (alkyle en C₁₋₈)amino, dialkylamino, alcénylamino, alcynylamino, arylamino, hétéroarylamino, (cycloalkyle en C₃₋₈)amino, cycloalcénylamino, hétérocycloalkylamino, hétérocycloalcénylamino, (alkyle en C₁₋₈)thio, halogéno (alkyle en C₁₋₈)thio, alcénylthio, alcynylthio, arylthio, hétéroarylthio, (cycloalkyle en C₃₋₈)thio, cycloalcénylthio, hétérocycloalkylthio, hétérocycloalcénylthio, -C(=O)R²⁰, -N(R²¹)C(=O)R²², -OC(= O)R²², -N(R²¹)S(=O)₂R²², -S(=O)₂R²⁰, et -S(=O)R²⁰, chacun facultativement substitué ; et
R¹³ est choisi dans le groupe constitué d'hydrogène, halogène, nitro, cyano, hydroxyle, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, halogéno(alkyle en C₁₋₈)amino, alcénylamino, alcynylamino, arylamino, hétéroarylamino, (cycloalkyle en C₃₋₈)amino, cycloalcénylamino, hétérocycloalkylamino, hétérocycloalcénylamino, (alkyle en C₁₋₈)thio, halogéno (alkyle en C₁₋₈)thio, alcénylthio, alcynylthio, arylthio, hétéroarylthio, (cycloalkyle en C₃₋₈)thio, cycloalcénylthio, hétérocycloalkylthio, hétérocycloalcénylthio, -C(=O)R²⁰, -N(R²¹)C(=O)R²², -OC(= O)R²², -N(R²¹)S(=O)₂R²², -S(=O)₂R²⁰, et -S(=O)R²⁰, chacun facultativement substitué ; et
R⁹ et R¹⁰ , ou R¹⁰ et R¹¹, ou R¹¹ et R¹², ou R¹³ et R¹⁴ conjointement avec les atomes auxquels ils sont liés forment un cycle insaturé ou partiellement insaturé de 5 ou 6 chaînons condensé non substitué ou substitué facultativement interrompu par un -O-, -NR⁶-, -S-, -SO- ou -SO₂- ; et
chaque R²⁰ est indépendamment choisi dans le groupe constitué d'hydroxyle, amino, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₆)amino, halogéno (alkyle en C₁₋₈)amino, dialkylamino, alcénylamino, alcynylamino, arylamino, hétéroarylamino, (cycloalkyle en C₃-₈)amino, cycloalcénylamino, hétérocycloalkylamino, et hétérocycloalcénylamino, chacun facultativement substitué ; et
chaque R²¹ est indépendamment choisi dans le groupe constitué d'hydrogène, hydroxyle, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, et hétérocycloalcényloxy, chacun facultativement substitué ; et
chaque R²² est indépendamment choisi dans le groupe constitué d' amino, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₈)amino, halogéno (alkyle en C₁₋₈)amino, dialkylamino, alcénylamino, alcynylamino, arylamino, hétéroarylamino, (cycloalkyle en C₃₋₈)amino, cycloalcénylamino, hétérocycloalkylamino, et hétérocycloalcénylamino, chacun facultativement substitué.

6. Composé selon la revendication 1, qui est un composé selon la formule VI : 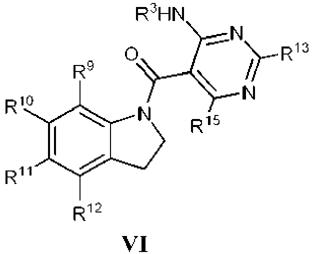 ou un sel pharmaceutiquement acceptable de celui-ci, dans lequel :
R³ est choisi dans le groupe constitué d'alkyle en C₂₋₈, alcényle en C₂₋₈, alcynyle en C₂₋₈, et halogénoalkyle en C₁₋₈, chacun facultativement substitué ; ou
R³ est choisi dans le groupe constitué d'un groupe arylalkyle et hétéroarylalkyle, chacun facultativement substitué ; ou
R³ est choisi dans le groupe constitué de cycloalkyle en C₃₋₈, cycloalcényle, hétérocycloalkyle lié à un carbone et hétérocycloalcényle lié à un carbone, chacun facultativement substitué ; et
R⁶ est choisi dans le groupe constitué d'hydrogène, alkyle en C₁₋₈, alcényle en C₃₋₈, alcynyle en C₃₋₈, halogénoalkyle en C₁₋₈, aryle, hétéroaryle et cycloalkyle en C₃₋₈ ; et
R⁹, R¹⁰, R¹¹, R¹² et R¹⁵ sont chacun indépendamment choisis dans le groupe constitué d'hydrogène, halogène, nitro, cyano, hydroxyle, amino, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₈)amino, halogéno (alkyle en C₁₋₈)amino, dialkylamino, alcénylamino, alcynylamino, arylamino, hétéroarylamino, (cycloalkyle en C₃₋₈)amino, cycloalcénylamino, hétérocycloalkylamino, hétérocycloalcénylamino, (alkyle en C₁₋₈)thio, halogéno (alkyle en C₁₋₈)thio, alcénylthio, alcynylthio, arylthio, hétéroarylthio, (cycloalkyle en C₃₋₈)thio, cycloalcénylthio, hétérocycloalkylthio, hétérocycloalcénylthio, -C(=O)R²⁰, -N(R²¹)C(=O)R²², -OC(= O)R²², -N(R²¹)S(=O)₂R²², -S(=O)₂R²⁰, et -S(=O)R²⁰, chacun facultativement substitué ; et
R¹³ est choisi dans le groupe constitué d'hydrogène, halogène, nitro, cyano, hydroxyle, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, halogéno(alkyle en C₁₋₈)amino, alcénylamino, alcynylamino, arylamino, hétéroarylamino, (cycloalkyle en C₃₋₈)amino, cycloalcénylamino, hétérocycloalkylamino, hétérocycloalcénylamino, (alkyle en C₁₋₈)thio, halogéno (alkyle en C₁₋₈)thio, alcénylthio, alcynylthio, arylthio, hétéroarylthio, (cycloalkyle en C₃₋₈)thio, cycloalcénylthio, hétérocycloalkylthio, hétérocycloalcénylthio, -C(=O)R²⁰, -N(R²¹)C(=O)R²², -OC(= O)R²², -N(R²¹)S(=O)₂R²², -S(=O)₂R²⁰, et -S(=O)R²⁰, chacun facultativement substitué ; et
R⁹ et R¹⁰, ou R¹⁰ et R¹¹, ou R¹¹ et R¹² conjointement avec les atomes auxquels ils sont liés forment un cycle insaturé ou partiellement insaturé de 5 ou 6 chaînons condensé non substitué ou substitué facultativement interrompu par un -O-, -NR⁶-, -S-, -SO- ou -SO₂- ; et
chaque R²⁰ est indépendamment choisi dans le groupe constitué d'hydroxyle, amino, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₆)amino, halogéno (alkyle en C₁₋₈)amino, dialkylamino, alcénylamino, alcynylamino, arylamino, hétéroarylamino, (cycloalkyle en C₃₋₈)amino, cycloalcénylamino, hétérocycloalkylamino, et hétérocycloalcénylamino, chacun facultativement substitué ; et
chaque R²¹ est indépendamment choisi dans le groupe constitué d'hydrogène, hydroxyle, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, et hétérocycloalcényloxy, chacun facultativement substitué ; et
chaque R²² est indépendamment choisi dans le groupe constitué d' amino, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₈)amino, halogéno(alkyle en C₁₋₈)amino, dialkylamino, alcénylamino, alcynylamino, arylamino, hétéroarylamino, (cycloalkyle en C₃₋₈)amino, cycloalcénylamino, hétérocycloalkylamino, et hétérocycloalcénylamino, chacun facultativement substitué.

7. Composé de la revendication 3 dans lequel
R⁹, R¹⁰, R¹¹ et R¹² sont chacun indépendamment choisis dans le groupe constitué d'hydrogène, halogène, alkyle en C₁₋₈, halogénoalkyle en C₁₋₈, cycloalkyle en C₃₋₈, alcoxy en C₁₋₈, halogénoalcoxy en C₁₋₈, et cycloalcoxy en C₃₋₈, chacun facultativement substitué ; et
R¹³, R¹⁴ et R¹⁵ sont chacun indépendamment choisis dans le groupe constitué d'hydrogène, halogène, alkyle en C₁₋₈, halogénoalkyle en C₁₋₈, cycloalkyle en C₃₋₈, et halogénoalcoxy en C₁₋₈, chacun facultativement substitué ; et
R²⁶ est un groupe aryle choisi parmi : 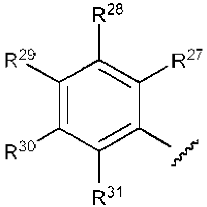
X¹⁷ est N ou CR¹³ ;
X¹⁸ est N ou CR¹⁴ ;
X¹⁹ est N ou CR¹⁵ ;
R²⁷, R²⁸, R²⁹, R³⁰ et R³¹ sont indépendamment choisis dans le groupe constitué d'hydrogène, halogène, nitro, cyano, alkyle en C₁₋₈, halogénoalkyle en C₁₋₈, cycloalkyle en C₃₋₈, alcoxy en C₁₋₈, halogénoalcoxy en C₁₋₈, alcényloxy, alcynyloxy, et cycloalcoxy en C₃₋₈, et sels pharmaceutiquement acceptables de celui-ci.

8. Composé de la revendication 7 dans lequel : R⁹ et R¹² sont hydrogène ; et sels pharmaceutiquement acceptables de celui-ci.

9. Composé de la revendication 8 dans lequel :
X¹⁷ est CR¹³ ;
X¹⁸ est CR¹⁴ ;
X¹⁹ est CR¹⁵ ;
et sels pharmaceutiquement acceptables de celui-ci ; ou dans lequel :
X¹⁷ est CR¹³ ;
X¹⁸ est CR¹⁴ ;
X¹⁹ est N ;
et sels pharmaceutiquement acceptables de celui-ci ; ou dans lequel :
X¹⁷ est N ;
X¹⁸ est CR¹⁴ ;
X¹⁹ est CR¹⁵ ;
et sels pharmaceutiquement acceptables de celui-ci ; ou dans lequel :
X¹⁷ est CR¹³ ;
X¹⁸ est N ;
X¹⁹ est CR¹⁵ ;
et sels pharmaceutiquement acceptables de celui-ci.

10. Composé selon la revendication 1, qui est choisi dans le groupe constitué de :
[2-(benzylamino)pyridin-3-yl](5-chloro-2,3-dihydro-1H-indol-1-yl)méthanone ;
(5-chloro-2,3-dihydro-1*H*-indol-1-yl)[2-(phénylamino)pyridin-3-yl]méthanone ;
(5-chloro-2,3-dihydro-1*H*-indol-1-yl)[2-[(pyridin-2-ylméthyl)amino]pyridin-3-yl]méthanone ;
(5-chloro-2,3-dihydro-1*H*-indol-1-yl)[2-[(2-phényléthyl)amino]pyridin-3-yl]méthanone ;
(5-chloro-2,3-dihydro-1*H*-indol-1-yl)[2-[(pyridin-3-ylméthyl)amino]pyridin-3-yl]méthanone ;
(5-chloro-2,3-dihydro-1*H*-indol-1-yl)[2-[[2-(pyridin-2-yl)éthyl]amino]-pyridin-3-yl]méthanone ;
(5-chloro-2,3-dihydro-1*H*-indol-1-yl)[2-[(4-fluorobenzyl)amino]pyridin-3-yl]méthanone ;
(5-chloro-2,3-dihydro-1*H*-indol-1-yl)[2-[(1-(4-fluorophényl)éthyl)amino]-pyridin-3-yl]méthanone ;
[2-(benzylamino)pyridin-3-yl](2,3-dihydro-1*H*-indol-1-yl)méthanone ;
[3-(benzylamino)pyridazin-4-yl](5-chloro-2,3-dihydro-1H-indol-1-yl)méthanone ;
(5-chloro-2,3-dihydro-1H-indol-1-yl)[2-[(4-fluorobenzyl)amino]pyrazin-3-yl]méthanone ;
(5-chloro-2,3-dihydro-1H-indol-1-yl)[2-(2,5-difluorobenzylamino)pyridin-3-yl-]méthanone ;
(5-chloro-2,3-dihydro-1H-indol-1-yl)[2-(3,4-difluorobenzylamino)pyridin-3-yl]-méthanone ;
(5-chloro-2,3-dihydro-1H-indol-1-yl)[2-(2,4-difluorobenzylamino)pyridin-3-yl]-méthanone ;
(5-chloro-2,3-dihydro-1H-indol-1-yl)[2-(cyclopropylméthylamino)pyridin-3-yl]-méthanone ;
(5-chloro-2,3-dihydro-1H-indol-1-yl)[2-(2-propyn-1-ylamino)pyridin-3-yl]-méthanone ;
(5-fluoro-2,3-dihydro-1H-indol-1-yl)[2-(4-fluorobenzylamino)pyridin-3-yl]-méthanone ;
(5-chloro-2,3-dihydro-1H-indol-1-yl)[2-[(pyridin-4-ylamino)pyridin-3-yl]-méthanone, et sels pharmaceutiquement acceptables et solvates de celui-ci.

11. Composition pharmaceutique comprenant un composé selon l'une quelconque des revendications 1 à 10, ou un sel pharmaceutiquement acceptable de celui-ci, et un véhicule ou diluant pharmaceutiquement acceptable.

12. Composition pharmaceutique selon la revendication 11, comprenant un excipient pharmaceutiquement acceptable et un composé choisi parmi :
[2-(benzylamino)pyridin-3-yl](5-chloro-2,3-dihydro-1H-indol-1-yl)méthanone (composé 1) ;
(5-chloro-2,3-dihydro-1*H*-indol-1-yl)[2-(phénylamino)pyridin-3-yl]méthanone (composé 2) ;
(5-chloro-2,3-dihydro-1*H*-indol-1-yl)[2-[(pyridin-2-ylméthyl)amino]pyridin-3-yl]-méthanone (composé 3) ;
(5-chloro-2,3-dihydro-1*H*-indol-1-yl)[2-[(2-phényléthyl)amino]pyridin-3-yl]méthanone (composé 4) ;
(5-chloro-2,3-dihydro-1*H*-indol-1-yl)[2-[(pyridin-3-ylméthyl)amino]pyridin-3-yl]-méthanone (composé 5) ;
(5-chloro-2,3-dihydro-1*H*-indol-1-yl)[2-[[2-(pyridin-2-yl)éthyl]amino]-pyridin-3-yl]-méthanone (composé 6) ;
(5-chloro-2,3-dihydro-1*H*-indol-1-yl)[2-[(4-fluorobenzyl)amino]pyridin-3-yl]méthanone (composé 7) ;
(5-chloro-2,3-dihydro-1*H*-indol-1-yl)[2-[(1-(4-fluorophényl)éthyl)-amino]pyridin-3-yl]-méthanone (composé 8) ;
[2-(benzylamino)pyridin-3-yl](2,3-dihydro-1*H*-indol-1-yl)méthanone (composé 9) ;
[3-(benzylamino)pyridazin-4-yl](5-chloro-2,3-dihydro-1H-indol-1-yl)méthanone (composé 10) ;
(5-chloro-2,3-dihydro-1H-indol-1-yl)[2-[(4-fluorobenzyl)amino]pyrazin-3-yl]méthanone (composé 11) ;
*N*-(4-éthoxyphényl)-2-[(2-phényléthyl)amino]pyridine-3-carboxamide (composé 12) ;
*N*-phényl-2-[(2-phényléthyl)amino]pyridine-3-carboxamide (composé 13) ;
*N*-(4-chlorophényl)-2-[(2-phényléthyl)amino]pyridine-3-carboxamide (composé 14) ;
2-(benzylamino)-*N*-(4-éthoxyphényl)pyridine-3-carboxamide (composé 15) ;
*N*-(4-éthoxyphényl)-2-(propylamino)pyridine-3-carboxamide (composé 16) ;
*N*-(4-hydroxyphényl)-2-[(2-phényléthyl)amino]pyridine-3-carboxamide (composé 17) ;
*N*-(4-éthoxyphényl)-2-(phénylamino)pyridine-3-carboxamide (composé 18) ;
2-[(cyclohexylméthyl)amino]-*N*-(4-éthoxyphényl)pyridine-3-carboxamide (composé 19) ;
3-(benzylamino)-*N*-(4-éthoxyphényl)pyridine-4-carboxamide (composé 20) ;
4-(benzylamino)-*N*-(4-éthoxyphényl)pyridine-3-carboxamide (composé 21) ;
3-(benzylamino)-6-chloro-*N*-(4-éthoxyphényl)pyridazine-4-carboxamide (composé 22) ;
2-(benzylamino)-*N*-[4-(trifluorométhyl)phényl]pyridine-3-carboxamide (composé 24) ;
2-(benzylamino)-*N*-(4-fluorophényl)pyridine-3-carboxamide (composé 25) ;
*N*-(4-chlorophényl)-5-[2-[(4-chlorophényl)éthyl]amino]-3-méthyl-4-isoxazolecarboxamide (composé 26) ;
(5-chloro-2,3-dihydro-1H-indol-1-yl)[2-(2,5-difluorobenzylamino)pyridin-3-yl]-méthanone (composé 27) ;
(5-chloro-2,3-dihydro-1H-indol-1-yl)[2-(3,4-difluorobenzylamino)pyridin-3-yl]-méthanone (composé 28) ;
(5-chloro-2,3-dihydro-1H-indol-1-yl)[2-(2,4-difluorobenzylamino)pyridin-3-yl]-méthanone (composé 29) ;
(5-chloro-2,3-dihydro-1H-indol-1-yl)[2-(cyclopropylméthylamino)pyridin-3-yl]-méthanone (composé 30) ;
(5-chloro-2,3-dihydro-1H-indol-1-yl)[2-(2-propyn-1-ylamino)pyridin-3-yl]méthanone (composé 31) ;
(5-fluoro-2,3-dihydro-1H-indol-1-yl)[2-(4-fluorobenzylamino)pyridin-3-yl]méthanone (composé 32) ; et
(5-chloro-2,3-dihydro-1H-indol-1-yl)[2-[(pyridin-4-ylamino)pyridin-3-yl]méthanone (composé 33), et sels pharmaceutiquement acceptables de celui-ci.

13. Composition pharmaceutique selon la revendication 11,
comprenant un diluant ou véhicule pharmaceutiquement acceptable et un composé de formule VII : 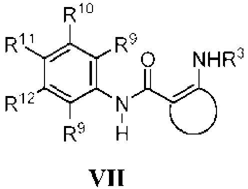 ou un sel pharmaceutiquement acceptable de celui-ci, dans laquelle : et 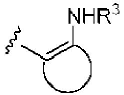 est un groupe hétéroaryle choisi dans le groupe constitué de : 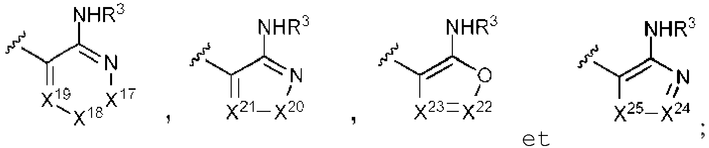
X¹⁷ est N ou CR¹³ ;
X¹⁸ est N ou CR¹⁴ ;
X¹⁹ est N ou CR¹⁵ ;
X²⁰ est NR⁶, S(O)ₘ ou O ;
X²¹ est N ou CR¹⁶ ;
X²² est N ou CR¹⁷ ;
X²³ est N ou CR¹⁸ ;
X²⁴ est N ou CR¹⁹ ;
X²⁵ est NR⁶, S(O)ₘ ou O ;
m est 0, 1 ou 2 ;
R³ est choisi dans le groupe constitué d'alkyle en C₂₋₈, alcényle en C₂₋₈, alcynyle en C₂₋₈, et halogénoalkyle en C₁₋₈, chacun facultativement substitué ; ou
R³ est choisi dans le groupe constitué d'un groupe arylalkyle et hétéroarylalkyle, chacun facultativement substitué ; ou
R³ est choisi dans le groupe constitué de cycloalkyle en C₃₋₈, cycloalcényle, hétérocycloalkyle lié à un carbone et hétérocycloalcényle lié à un carbone, chacun facultativement substitué ; et
R⁶ est choisi dans le groupe constitué d'hydrogène, alkyle en C₁₋₈, alcényle en C₃₋₈, alcynyle en C₃₋₈, halogénoalkyle en C₁₋₈, aryle, hétéroaryle et cycloalkyle en C₃₋₈ ; et
R⁹, R¹⁰, R¹¹, R¹², R¹⁴, R¹⁵, R¹⁶, R¹⁷, R¹⁸ et R¹⁹ sont chacun indépendamment choisis dans le groupe constitué d'hydrogène, halogène, nitro, cyano, hydroxyle, amino, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₈)amino, halogéno (alkyle en C₁₋₈)amino, dialkylamino, alcénylamino, alcynylamino, arylamino, hétéroarylamino, (cycloalkyle en C₃₋₈)amino,cycloalcénylamino, hétérocycloalkylamino, hétérocycloalcénylamino, (alkyle en C₁₋₈)thio, halogéno (alkyle en C₁₋₈)thio, alcénylthio, alcynylthio, arylthio, hétéroarylthio, (cycloalkyle en C₃₋₈)thio, cycloalcénylthio, hétérocycloalkylthio, hétérocycloalcénylthio, -C(=O)R²⁰, -N(R²¹)C(=O)R²², -OC(= O)R²², -N(R²¹)S(=O)₂R²², -S(=O)₂R²⁰, et -S(=O)R²⁰, chacun facultativement substitué ; et
R¹³ est choisi dans le groupe constitué d'hydrogène, halogène, nitro, cyano, hydroxyle, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, halogéno(alkyle en C₁₋₈)amino, alcénylamino, alcynylamino, arylamino, hétéroarylamino, (cycloalkyle en C₃₋₈)amino, cycloalcénylamino, hétérocycloalkylamino,
hétérocycloalcénylamino, (alkyle en C₁₋₈)thio, halogéno(alkyle en C₁₋₈)thio, alcénylthio, alcynylthio, arylthio, hétéroarylthio, (cycloalkyle en C₃₋₈)thio, cycloalcénylthio, hétérocycloalkylthio, hétérocycloalcénylthio, -C(=O)R²⁰, -N(R²¹)C(=O)R²², -OC(= O)R²², -N(R²¹)S(=O)₂R²², -S(=O)₂R²⁰, et -S(=O)R²⁰, chacun facultativement substitué ; et
R⁶ et R¹⁶, ou R⁶ et R¹⁹, ou R⁹ et R¹⁰, ou R¹⁰ et R¹¹, ou R¹¹ et R¹² ou R¹³ et R¹⁴, ou R¹⁴ et R¹⁵, ou R¹⁷ et R¹⁸ conjointement avec les atomes auxquels ils sont liés forment un cycle insaturé ou partiellement insaturé de 5 ou 6 chaînons condensé non substitué ou substitué facultativement interrompu par un -O-, -NR⁶-, -S-, -SO- ou -SO₂- ; et
chaque R²⁰ est indépendamment choisi dans le groupe constitué d'hydroxyle, amino, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₈)amino, halogéno (alkyle en C₁₋₈)amino, dialkylamino, alcénylamino, alcynylamino, arylamino, hétéroarylamino, (cycloalkyle en C₃₋₈)amino, cycloalcénylamino, hétérocycloalkylamino, et hétérocycloalcénylamino, chacun facultativement substitué ; et
chaque R²¹ est indépendamment choisi dans le groupe constitué d'hydrogène, hydroxyle, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, et hétérocycloalcényloxy, chacun facultativement substitué ; et
chaque R²² est indépendamment choisi dans le groupe constitué d' amino, alkyle en C₁₋₈, alcényle en C₂₋₈, alcynyle en C₂₋₈, halogénoalkyle en C₁₋₈, aryle, hétéroaryle, cycloalkyle en C₃₋₈, cycloalcényle, hétérocycloalkyle, hétérocycloalcényle, alcoxy en C₁₋₈, halogénoalcoxy en C₁₋₈, alcényloxy, alcynyloxy, aryloxy, hétéroaryloxy, cycloalcoxy en C₃₋₈, cycloalcényloxy, hétérocycloalkyloxy, hétérocycloalcényloxy, (alkyle en C₁₋₈)amino, halogéno (alkyle en C₁₋₈)amino, dialkylamino, alcénylamino, alcynylamino, arylamino, hétéroarylamino, (cycloalkyle en C₃₋₈)amino, cycloalcénylamino, hétérocycloalkylamino, et hétérocycloalcénylamino, chacun facultativement substitué.

14. Composé selon l'une quelconque des revendications 1 à 10 ou sel pharmaceutiquement acceptable de celui-ci, ou composition selon l'une quelconque des revendications 11 à 13, pour utilisation en tant que médicament.

15. Composé selon l'une quelconque des revendications 1 à 10, ou sel pharmaceutiquement acceptable de celui-ci, ou composition selon l'une quelconque des revendications 11 à 13, pour utilisation dans le traitement d'un trouble permettant la modulation de α7 nAChR.

16. Composé selon l'une quelconque des revendications 1 à 10 ou sel pharmaceutiquement acceptable de celui-ci, ou composition selon l'une quelconque des revendications 11 à 13, pour utilisation dans le traitement d'un trouble choisi parmi des maladies neurodégénératives, des démences séniles, la schizophrénie, la maladie d'Alzheimer, des déficits de l'apprentissage, la cognition et l'attention, une perte de mémoire, la démence à corps de Lewy, un trouble déficitaire de l'attention, un trouble déficitaire de l'attention avec hyperactivité, l'anxiété, une manie, une dépression maniaque, la maladie de Parkinson, la maladie de Huntington, la dépression, la sclérose latérale amyotrophique, une inflammation cérébrale, un déficit cognitif dû à une lésion cérébrale traumatique, le syndrome de Tourette, un trouble du spectre de l'autisme, un trouble cognitif lié à l'apprentissage ou la mémoire comprenant un trouble cognitif léger, la douleur, l'inflammation, des troubles du système immunitaire, un choc septique, la recto-colite hémorragique, la maladie de Crohn ou le syndrome du côlon irritable.
